# EUROPEAN PATENT APPLICATION

(11) **EP 4 509 608 A1**
(43) Date of publication of application: **19.02.2025**
(21) Application number: 23763533.9
(22) Date of filing: 02.03.2023
(51) Int. Cl.: C12N 15/79, C12Q 1/6825, C12P 21/00

(54) **CAPPED RNA AND METHOD FOR PRODUCING SAME, APPARATUS FOR PRODUCING PROTEIN, AND METHOD FOR PRODUCING PROTEIN**

(30) Priority: 04.03.2022 JP 2022033316
(71) Applicant: Japan Science and Technology Agency, Kawaguchi-shi, Saitama 332-0012 (JP); National University Corporation Tokai National Higher Education and Research System, Nagoya-shi, Aichi 464-8601 (JP)
(72) Inventor: ABE, Hiroshi, Nagoya-shi, Aichi 464-8601 (JP); ABE, Naoko, Nagoya-shi, Aichi 464-8601 (JP); INAGAKI, Masahito, Nagoya-shi, Aichi 464-8601 (JP); NAKASHIMA, Yuko, Nagoya-shi, Aichi 464-8601 (JP); KIMURA, Yasuaki, Nagoya-shi, Aichi 464-8601 (JP); HASHIYA, Fumitaka, Nagoya-shi, Aichi 464-8601 (JP)
(74) Representative: Hoffmann Eitle
(86) International application number: PCT/JP2023/007759
(87) International publication number: WO 2023/167276

(57) **Abstract**

The present invention is a capped RNA which has a translated region beginning with a start codon and encoding a protein and an upstream region upstream of the start codon, and a branch part having a cap structure. This branch part preferably has at least one structure of the following (a) to (c):
(a) a capped probe having a sequence complementary to a sequence of a part or all of the untranslated region and having the cap structure is hybridized to the untranslated region;
(b) a branched chain branching from the middle of a main chain constituting the RNA is provided in a part of the untranslated region and the cap structure is provided in the branched chain; and
(c) the RNA has a branch structure in which the 5¹ end of a main chain of the untranslated region is branched into two or more branches, the cap structure being provided in each branch.

## Description

### Technical Field

The present invention relates to a capped RNA having a cap structure and a method for producing the same, an apparatus for producing a protein, and a method for producing a protein.

### Background Art

Eukaryotic mRNA and the like has been known to have a 5'-capped structure in which 7-methylguanosine forms 5'-5' bond with the 5' end of RNA via a triphosphate bond. The cap structure has been known to promote translation of mRNA, and a target protein can be efficiently synthesized by artificially introducing the cap structure into mRNA in a protein expression system or the like.

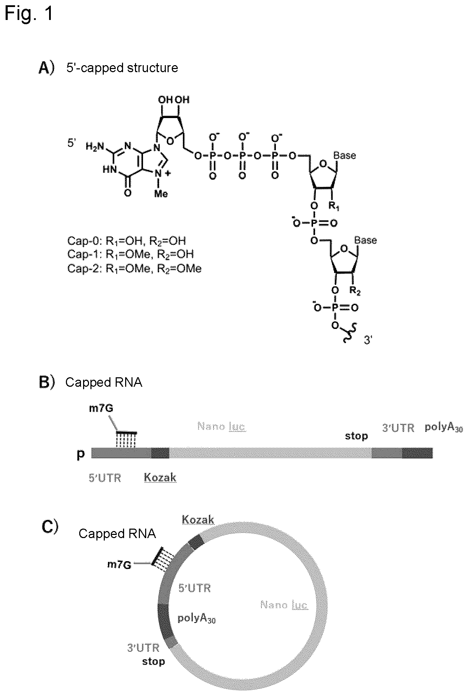

The present inventors have so far developed a method for producing a capped RNA which is RNA having the 5' end modified with a cap, the method including reacting an activated capping compound with a monophosphate RNA having the 5' end monophosphorylated (see, for example, PTL 1). By this method, a cap structure can be introduced into the 5' end of the RNA by a chemical method. A general RNA having a cap structure at the 5' end of the main chain of the RNA is shown in Fig. 1(A).

On the other hand, the present inventors have developed a circular RNA that encodes a protein, has a full-length base composed by number of 3 multiples, larger than 102 or more, has at least one start codon, does not have a terminator codon (stop codon) in the same reading frame as the start codon, and further does not contain an IRES (see, for example, PTL 2 and NPL 1). This circular RNA does not have a cap structure, but can perform rolling-circle-type protein translation with high efficiency.

As described above, there are a linear RNA and a circular RNA, and the linear RNA generally has one cap structure at the 5' end of the main chain, whereas the circular RNA is not known to have a cap structure. In general, it is known that a translation reaction is initiated from a start codon immediately after the introduction of a cap structure at the 5' end in the linear RNA. On the other hand, in the circular mRNA having no 5' end, since a cap structure cannot be introduced, it is common to introduce an IRES sequence in order to initiate a translation reaction. When two proteins are also encoded in a linear mRNA, in order to start a translation reaction in the second coding region, a method of introducing an IRES sequence is known (see, for example, NPL 2). In this literature, IRES is incorporated into eToehold, and eToehold is designed to be inactive until it is activated by a sense-antisense interaction with specific trRNA.

### Citation List

### Patent Literature

PTL 1: WO2021/172204A (Abstract, Claim 1, and the like)
PTL 2: WO2013/118878A (Abstract, Claim 1, paragraph 0004, and the like)

### Non-patent Literature

NPL 1: "Successful Mass Synthesis of Protein in Human Cultured Cells ∼Realization of Endless Rotary Protein Synthesis Reaction Using Circular mRNA~", Japan Science and Technology Agency, Press Release, November 10, 2015, Japan Science and Technology Agency (JST), Nagoya University, RIKEN (joint announcement), <ULR: https://www.jst.go.jp/pr/announce/20151110/index.html#ZU1>
NPL 2: "RNA-responsive elements for eukaryotic translational control", Evan M. Zhao et al., nature biotechnology, October 28, 2021, <URL:https://doi.org/10.1038/s41587-021-01068-2>

### Summary of Invention

### Technical Problem

In the linear RNA as in PTL 1, it is possible to chemically introduce one cap structure at the 5' end, but further improvement for improving translational activity is required.

In the circular RNA having no cap structure as in PTL 2, the rate at which translation initiation factors constituting a ribosome binds to RNA is slow, and there is room for further improvement in translational activity.

An object of the present invention is to provide a capped RNA having various types of structures not conventionally used and having translational activity, and a method for producing the same. Another object of the present invention is to provide a method for producing a protein and an apparatus for producing a protein capable of synthesizing a protein using such a capped RNA.

### Solution to Problem

[1] A capped RNA including:
   a translated region beginning with a start codon and encoding a protein; and
   an upstream region upstream of the start codon, a branch part having a cap structure being provided in the upstream region.
[2] The capped RNA, wherein the branch part has at least one structure of (a) to (c) below:
   (a) a capped probe having a sequence complementary to a sequence of a part or all of the upstream region and having the cap structure is hybridized to the upstream region;
   (b) a branched chain branching from a main chain constituting the RNA is provided in a part of the upstream region and the cap structure is provided in the branched chain; or
   (c) the RNA is a linear RNA and has a branch structure in which the 5' end of a main chain of the upstream region is branched into two or more branches, the cap structure being provided in each branch.
[3] The capped RNA according to [2], wherein
   in the (a), the RNA is a circular RNA, and
   a capped probe having a sequence complementary to a sequence of a part or all of the upstream region of the circular RNA and having a cap structure is hybridized to the upstream region of the circular RNA.
[4] The capped RNA according to [2], wherein
   in the (a), the RNA is a linear RNA, and
   a capped probe having a sequence complementary to a sequence of a part or all of the upstream region of the linear RNA and having a cap structure is hybridized to the upstream region of the linear RNA.
[5] The capped RNA according to [2], wherein
   in the (b), the RNA is a circular RNA, and
   a branched chain branching from a main chain constituting the RNA is provided in a part of the upstream region of the circular RNA, and the cap structure is provided in the branched chain.
[6] The capped RNA according to [2], wherein
   in the (b), the RNA is a linear RNA, and
   a branched chain branching from a main chain constituting the RNA is provided in a part of the upstream region of the linear RNA, and the cap structure is provided in the branched chain.
[7] The capped RNA according to [2], which has a structure represented by Formula (3) below in the (b): 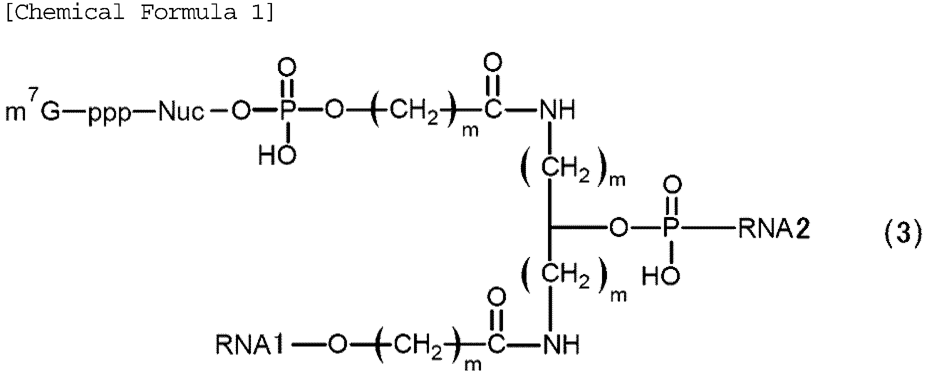 where m⁷G represents a cap, and p represents a phosphate group; Nuc represents a polynucleotide in which one or more natural or non-natural nucleotides are linked; m represents an integer of 1 to 5, and a plurality of m's may be the same as or different from each other; RNA1 and RNA2 represent RNAs constituting a main chain, and RNA1 and RNA2 are linked to a portion of a branched structure at the 3' end side and the 5' end side, respectively; and a RNA of Formula (3) may be a circular RNA in which the 5' end of RNA1 and the 3' end of RNA2 are linked to form a circular structure, or may be a linear RNA without the 5' end of RNA1 and the 3' end of RNA2 being linked.
[8] The capped RNA according to [2], which has a structure represented by Formula (1) or Formula (2) below in the (c): 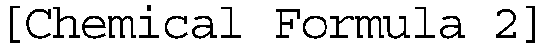 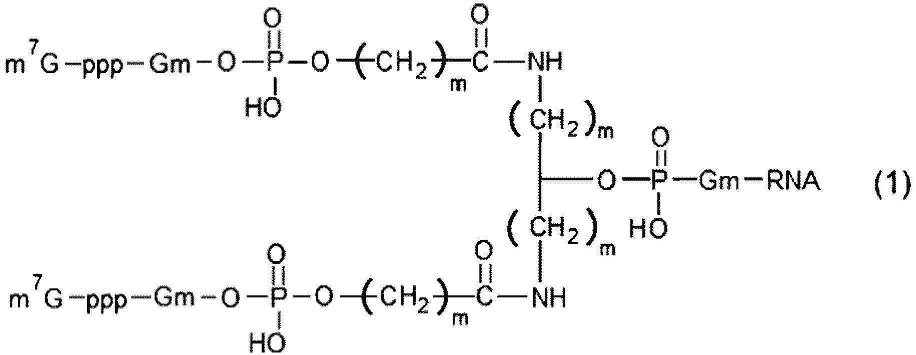 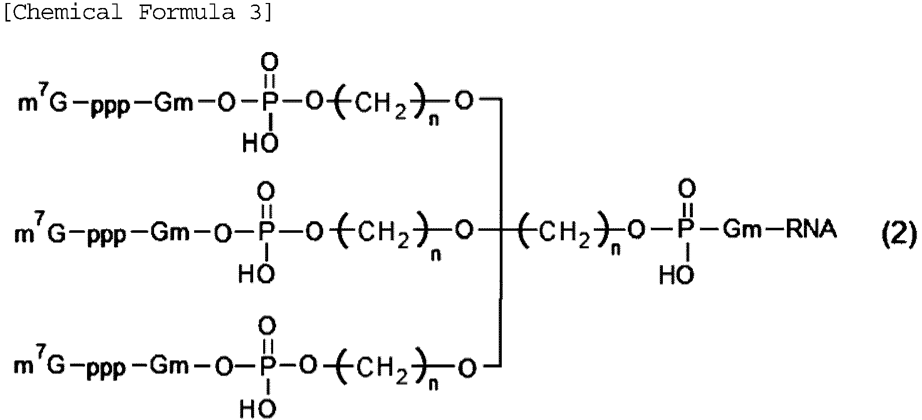 where m⁷G represents a cap, p represents a phosphate group, Gm represents a structure in which a 2'-hydroxyl group of a guanine nucleotide is methylated, m represents an integer of 1 to 5, a plurality of m's may be the same as or different from each other, n represents an integer of 1 to 5, and a plurality of n's may be the same as or different from each other.
[9] The capped RNA according to [8], wherein the Formula (1) has a structure represented by Formula (1-1) below, and the Formula (2) has a structure represented by Formula (2-1) below. 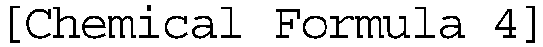 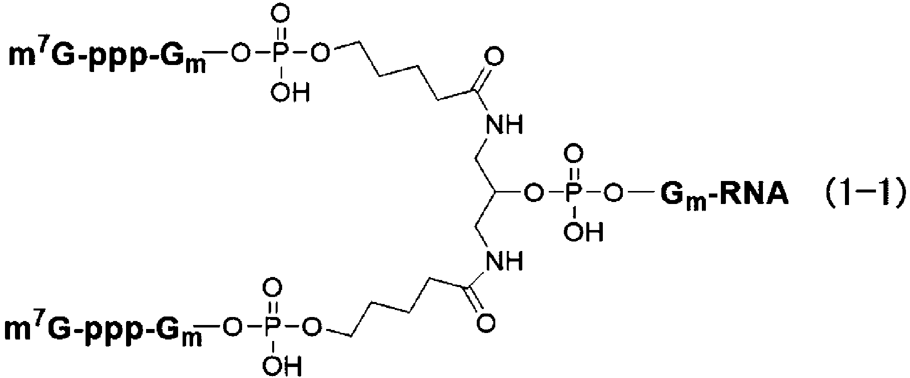 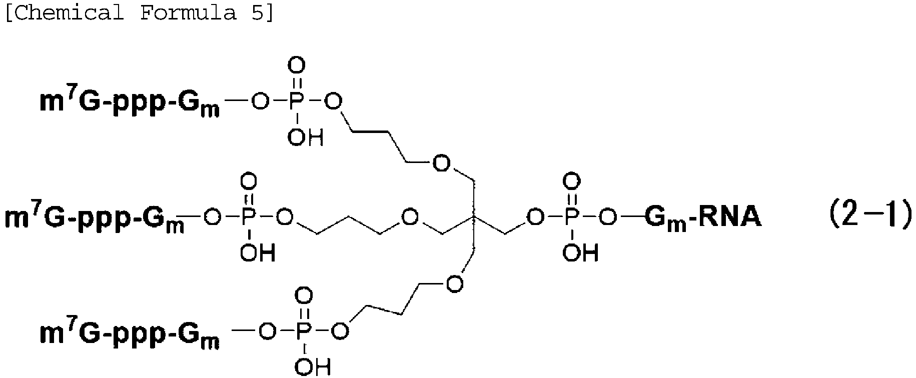
[10] The capped RNA according to [1], wherein the RNA has a plurality of translated regions.
[11] A method for producing the capped RNA according to [1], the method including at least one of steps (a) to (c) below:
   (a) hybridizing a capped probe having a sequence complementary to a sequence of a part or all of the upstream region and having a cap structure to the upstream region; or
   (b) synthesizing the RNA having a branched chain branching from a main chain constituting the RNA in a part of the upstream region, and then binding the cap structure to the branched chain; or
   (c) with the RNA being a linear RNA, synthesizing RNA having a branch structure in which the 5' end of a main chain of the upstream region is branched into two or more branches, and then binding the cap structure to each branch.
[12] An apparatus for producing a protein, including:
   the capped RNA according to [1]; and
   an expression system of a eukaryotic cell capable of expressing the protein encoded by the translated region using the capped RNA as a template.
[13] A method for producing a protein, including adding the capped RNA according to [1] to an expression system of a eukaryotic cell capable of expressing the protein encoded by the translated region using the capped RNA as a template to express the protein.
[14] A detection device of a target RNA having a cap structure at the 5' end of RNA, the detection device including:
   a detection probe containing a circular RNA having a translated region beginning with a start codon and encoding a labeled protein and an upstream region upstream of the start codon, the upstream region having a sequence complementary to a sequence of a part or all of the target RNA to be detected;
   a means for hybridizing the target RNA to the upstream region of the circular RNA by adding the circular RNA to a sample containing the target RNA to generate a capped RNA;
   an expression system of a eukaryotic cell capable of expressing the labeled protein by adding the capped RNA; and
   a means for detecting the labeled protein expressed in the expression system.
[15] A detection method of a target RNA having a cap structure at the 5' end of RNA, the detection method including:
   a step of preparing a detection probe containing a circular RNA having a translated region beginning with a start codon and encoding a labeled protein and an upstream region upstream of the start codon, the upstream region having a sequence complementary to a sequence of a part or all of the target RNA to be detected;
   a step of hybridizing the target RNA to the upstream region of the circular RNA by adding the circular RNA to a sample containing the target RNA to generate a capped RNA;
   a step of adding the capped RNA to an expression system of a eukaryotic cell capable of expressing the labeled protein to express the labeled protein; and
   a step of detecting the labeled protein expressed in the expression system.
[1 (Pre)] A capped RNA having a cap structure in an untranslated region of RNA having a translated region beginning with a start codon and encoding a protein and the untranslated region upstream of the start codon, the capped RNA including at least one structure of (a) to (c) below:
   (a) a capped probe having a sequence complementary to a sequence of a part or all of the untranslated region and having a cap structure is hybridized to the untranslated region;
   (b) a branched chain branching from a main chain constituting the RNA is provided in a part of the untranslated region and the cap structure is provided in the branched chain; or
   (c) the RNA has a branch structure in which the 5' end of a main chain of the untranslated region is branched into two or more branches, the cap structure being provided in each branch.
[9 (Pre)] A method for producing the capped RNA according to [1 (Pre)], the method including at least one of the following steps (a) to (c):
   (a) hybridizing a capped probe having a sequence complementary to a sequence of a part or all of the untranslated region and having a cap structure to the untranslated region; or
   (b) synthesizing the RNA having a branched chain branching from the main chain constituting the RNA in a part of the untranslated region, and then binding the cap structure to the branched chain; or
   (c) synthesizing RNA having a branch structure in which the 5' end of a main chain of the untranslated region is branched into two or more branches, and then binding the cap structure to each branch.
[12 (Pre)] A detection device of a target RNA having a cap structure at the 5' end of RNA, the detection device including:
   a probe containing a circular RNA having a translated region beginning with a start codon and encoding a labeled protein and an untranslated region upstream of the start codon, the untranslated region having a sequence complementary to a sequence of a part or all of the target RNA to be detected;
   a means for hybridizing the target RNA to the untranslated region of the circular RNA by adding the circular RNA to a sample containing the target RNA to generate a capped RNA;
   an expression system of a eukaryotic cell capable of expressing the labeled protein by adding the capped RNA; and
   a means for detecting the labeled protein expressed in the expression system.
[13 (Pre)] A detection method of a target RNA having a cap structure at the 5' end of RNA, the detection method including:
   a step of preparing a probe containing a circular RNA having a translated region beginning with a start codon and encoding a labeled protein and an untranslated region upstream of the start codon, the untranslated region having a sequence complementary to a sequence of a part or all of the target RNA to be detected;
   a step of hybridizing the target RNA to the untranslated region of the circular RNA by adding the circular RNA to a sample containing the target RNA to generate a capped RNA;
   a step of adding the capped RNA to an expression system of a eukaryotic cell capable of expressing the labeled protein to express the labeled protein; and
   a step of detecting the labeled protein expressed in the expression system.

### Advantageous Effects of Invention

According to the present invention, it is possible to provide a capped RNA having various types of structures not conventionally used and having translational activity, and a method for producing the same. Another object of the present invention can provide a method for producing a protein and an apparatus for producing a protein capable of synthesizing a protein using such a capped RNA.

### Brief Description of Drawings

Fig. 1 is a view showing an outline of a capped RNA (hybridization type) of the present invention.
Fig. 2 is a view showing an outline of a capped RNA (branched chain type) of the present invention.
Fig. 3 is a view showing base sequences and the like of a circular RNA and a capped probe used in this experiment in Examples.
Fig. 4 is a view showing the sequences of a template DNA and transcribed RNA in Examples.
Fig. 5 is a view showing the results of synthesis experiment of a capped probe in Examples.
Fig. 6 is a view showing the results of synthesis experiment of a linear RNA in Examples.
Fig. 7 is a view showing the results of circularization experiment of a linear RNA in Examples.
Fig. 8 is a view showing the results of relative evaluation of translational activity in Examples.
Fig. 9 is a view showing the evaluation results of the translational activity in the case of using various probes in Examples.
Fig. 10 is a view showing the evaluation results of the translational activity depending on the presence or absence of a polyA sequence in Examples.
Fig. 11 is a view showing the results of evaluating the translational activity by changing the ratio of a probe RNA in Examples.
Fig. 12 is a schematic view showing the synthesis scheme of a capped branched-chain-containing RNA oligonucleotide in Examples.
Fig. 13 is a view showing a chemical capping reaction of a branched chain in Examples.
Fig. 14 is a view showing the synthesis reaction of a circular RNA in Examples.
Fig. 15 is a view showing evaluation of translational activity of capped branched-chain-containing circular mRNA in human-derived cultured cells (HeLa) in Examples.
Fig. 16 is a view showing the structure of a 107-base-long RNA including a synthesized branch structure in Examples.
Fig. 17 is a view showing the evaluation results of the translational activity in HeLa cells including a synthesized 107-base-long RNA with branch structure/a plurality of cap structures in Examples.
Fig. 18 is a view showing the results of HPLC analysis of synthesized RNA in Examples.
Fig. 19 is a view showing the results of absorbance measurement of deprotected RNA and the results of electrophoresis of a capped RNA in Examples.
Fig. 20 is a view showing the results of absorbance measurement of a capped RNA in Examples.
Fig. 21 is a view showing the sequence of RNA after annealing in Examples.
Fig. 22 is a view showing the results of evaluating the structure and translational activity of bicistronic mRNA in Examples.
Fig. 23 is a view showing the overall structure of multi-uORF-mRNA and the sequence of probe RNA in Examples.
Fig. 24 is a view showing the overall structure and the sequence of multi-uORF-mRNA in Examples.
Fig. 25 is a view showing results of HPLC analysis of multi-uORF-mRNA in Examples.
Fig. 26 is a view showing results of HPLC analysis of multi-uORF-mRNA in Examples.
Fig. 27 is a photograph of denaturing PAGE of multi-uORF-mRNA in Examples and is a view showing capping efficiency.
Fig. 28 is a view showing evaluation results of the translational activity of multi-uORF-mRNA in Examples.
Fig. 29 is a view showing the sequence of a plasmid fragment containing IRES in Examples.
Fig. 30 is a view showing the evaluation results of the translational activity of capped circular RNA and IRES in Examples.
Fig. 31 is a view showing the experimental results showing that the translational activity of a circular mRNA in which a cap structure is introduced into a branched chain is greatly improved as compared with that in which a cap structure is not introduced.
Fig. 32 is a view showing the experimental results showing the relationship between the branched chain length of a circular mRNA in which a cap structure is introduced into a branched chain and translational activity (that the branched chain length is variable).
Fig. 33 is a view showing the experimental results showing that the translational activity of a circular mRNA having a cap structure in a branched chain is higher than that of mRNA or the like containing an EMCV-derived IRES sequence.
Fig. 34 is a view showing the results of synthesis of ATF4-Renilla mRNA by in vitro transcription.
Fig. 35 is a view showing the results of chemical synthesis of RNA.
Fig. 36 is a view showing the results of chemical capping of RNA.
Fig. 37 is a view showing the results of synthesis of circular NanoLuc mRNA by in vitro transcription.
Fig. 38 is a view showing the results of circularization reaction of circular NanoLuc mRNA.
Fig. 39 is a view showing the results of csRNA with respect to circular Nluc mRNA.
Fig. 40 is a view showing the results of chemical synthesis of RNA.
Fig. 41 is a view showing the results of chemical capping of RNA.
Fig. 42 is a view showing the results of chemical capping of RNA.
Fig. 43 is a view showing the sequences and secondary structures of ATF4-Renilla mRNA and ATF4-csRNA.
Fig. 44 is a view showing a translation reaction in HeLa cells of ATF4-Renilla mRNA and ATF4-csRNA5 to csRNA8.
Fig. 45 is a view showing the experimental results of a translation reaction in a HeLa cell extract of csRNA into which chemical modification was introduced.
Fig. 46 is a view showing the results of a translation reaction in HeLa cells of csRNA and a circular mRNA into which LNA modification was introduced.

### Description of Embodiments

A capped RNA of the present invention has a translated region beginning with a start codon (for example, AUG) and encoding a protein and an upstream region upstream (on the 5' end side) of the start codon. This upstream region may be an untranslated region (5'UTR) or may be within the reading frame of another open reading frame (ORF). This capped RNA has a branch part having a cap structure in the upstream region. Here, the "branch part having a cap structure" includes various forms in which the cap structure is branched from the RNA of the main chain. This includes, for example, a molecule such as a nucleic acid having a cap structure (DNA, RNA, or the like) hybridized to RNA of the main chain, a molecule in which nucleotides constituting the main chain are branched, a molecule covalently bound to nucleotides constituting the main chain, and the like. Note that, in the case of the circular RNA, 3'UTR and polyA are also included in the upstream region, but the branch part is preferably located at 5'UTR or uORF close to the start codon.

Specifically, the capped RNA has at least one of the following structures (a) to (c):
(a) a capped probe having a sequence complementary to a sequence of a part or all of an upstream region and having a cap structure is hybridized to the upstream region (hereinafter, referred to as "hybridization type");
(b) a structure in which a branched chain branching from the middle of a main chain constituting the RNA is provided in a part of an upstream region and a cap structure is provided in the branched chain (hereinafter, referred to as "branched chain type"); and
(c) a structure which has a branch structure in which the 5' end of a main chain of an upstream region is branched into two or more branches, the cap structure being provided in each branch (hereinafter, referred to as "multi-capped type").

Note that, the capped RNA of the present invention includes not only a natural RNA (that is, a polynucleotide to which nucleotides composed of ATP, GTP, CTP, and UTP are linked) but also a non-natural nucleic acid strand (modified nucleic acid strand) in which an mC4 linker, 2'OMe, LNA, or the like is introduced into a nucleotide of the RNA. Hereinafter, each type of capped RNA will be described.

### 1. Hybridization type

Hereinafter, the hybridization-type capped RNA will be described. (B) of Fig. 1 shows a hybridization-type capped RNA in a linear RNA and (C) of Fig. 1 shows a hybridization-type capped RNA in a circular RNA with the structures described in Examples, respectively. In both the linear RNA and the circular RNA, a capped probe, which is RNA having a cap structure, is hybridized with an upstream region (for example, an untranslated region (5'UTR)) of RNA encoding a protein to be translated (hereinafter, referred to as "coding RNA").

The coding RNA has a translated region ("Nano Luc" region in the drawing) beginning with a start codon and encoding a protein and an upstream region (for example, an untranslated region ("5'UTR" region) upstream (on the 5' end side) of the start codon. In the present embodiment, there may be a stop codon at the 3' end of the translated region, and an untranslated region (3'UTR) downstream (on the 3' end side) of the stop codon. The 3'UTR contains a polyA sequence. The coding RNA is linear in the linear RNA, and has a circular structure in which the 5' end and the 3' end of the linear RNA are linked in the circular RNA. Note that, in the present embodiment, an embodiment in which the coding RNA includes a stop codon is shown, but the present invention is not limited thereto, and for example, as in PTL 2 and NPL 1, the coding RNA may not have a stop codon. In the case of not including a stop codon, as disclosed in these prior arts, when a circular coding RNA having the number of bases of the entire length as a multiple of 3, which is 102 or more, is used as a circular RNA, a target protein can be generated with high translation efficiency by rotary protein translation.

The coding RNA may have only one translated region or two or more translated regions. When there are two or more translated regions, a capped probe having an upstream region on the upstream side of each translated region and hybridizing to any of the upstream regions can be used. In this case, the translational activity of the protein in the translated region starting from the start codon immediately below the upstream region is improved. It may have a normal cap structure at the 5' end of the main chain of the coding RNA. In the case of having a normal cap structure at the 5' end, the translation efficiency of the capped RNA is further improved by using the capped probe in combination with the cap structure.

The capped probe is composed of RNA shorter than the coding RNA, and has a base sequence complementary to a sequence of a part or all of 5'UTR of the coding RNA. The position at which the capped probe hybridizes in 5'UTR of the coding RNA is not particularly limited, but in the case of having a Kozak sequence, the position is preferably on the upstream side thereof. The length of the capped probe is not particularly limited, but is preferably about 10 to 50 bases (nt). The capped probe may have a cap bound to the 5' end or a cap bound to the 3' end. The capped probe may have a polyA sequence on the 3' end side. The length (number of bases) of the polyA sequence is not particularly limited, and is preferably 10 to 50 bases. The capped probe may have a locked nucleic acid (LNA) in which 2' carbon and 4' carbon of ribose are crosslinked, a C4 linker, or the like.

In the capped RNA, the capped probe is hybridized to 5'UTR of the coding RNA. Here, "hybridize" means to hybridize under stringent conditions. The "stringent conditions" refer to conditions under which a specific hybrid is formed and a nonspecific hybrid is hardly formed. Examples of RNA that "hybridizes under stringent conditions" include RNA having a certain or more homology to a sequence complementary to a sequence in a predetermined range of 5'UTR of the coding RNA. The homology in this case is, for example, usually 60% or more, preferably 80% or more, more preferably 90% or more, particularly preferably 95% or more, and most preferably 100%, in the BLAST algorithm.

Next, a method for producing a hybridization-type capped RNA will be described. A coding RNA constituting a capped RNA can be produced by using DNA having a sequence complementary to RNA having a target sequence and transcribing the DNA with RNA polymerase. Alternatively, the coding RNA may be chemically synthesized using a DNA synthesizer or the like. A circular coding RNA can be produced by producing a linear coding RNA and then circularizing the linear coding RNA by connecting ends using T4 RNA ligase 2 or the like.

The probe RNA can be produced by the following method. First, a non-capped RNA as a base is produced by an RNA solid phase synthesis method or the like using a phosphoramidite, a phosphorylation reagent, a 2'-OMe RNA amidite, an LNA amidite, a C4 linker amidite, a CPG column, or the like. Next, this RNA is reacted with m⁷GDP, 2-nitroimidazole, or the like to cap a phosphorylated RNA. Details of this reaction can be referred to PTL 1.

The capped RNA can be produced by annealing the coding RNA and the probe RNA. The ratio (molar ratio) between the coding RNA and the probe RNA during annealing is preferably in a range of 1 : 1 to 1 : 20, and more preferably in a range of 1 : 5 to 1 : 15 from the viewpoint of translational activity. The annealing temperature can be 80°C or higher, preferably 90°C or higher, and the reaction time can be 1 to 30 minutes, preferably about 3 to 10 minutes.

The hybridization-type capped RNA can add a cap structure at any position of 5'UTR regardless of whether the coding RNA is linear or circular. In RNA having a cap structure, the rate at which the translation initiation factor constituting a ribosome binds to the coding RNA is improved as compared with RNA having no cap structure, and as a result, the translational activity is improved. Alternatively, in a hybridization-type capped RNA, it is possible to enhance translation or induce a translation phenomenon from an arbitrary position by presenting a cap by hybridization with a probe in a circular RNA or a linear RNA.

In the linear RNA, by using a capped probe, a translation reaction can be induced from, for example, the second and subsequent start codons that are not originally used as a reading frame of protein synthesis. Thereby, translation can be induced from any RNA position by the capped probe.

A circular RNA having a cap structure has not been known so far, but, in the hybridization-type capped RNA of the present invention, a cap structure can also be introduced into a circular RNA. This makes it possible to significantly improve the translational activity as compared with a circular RNA having no cap structure.

### 2. Branched chain type

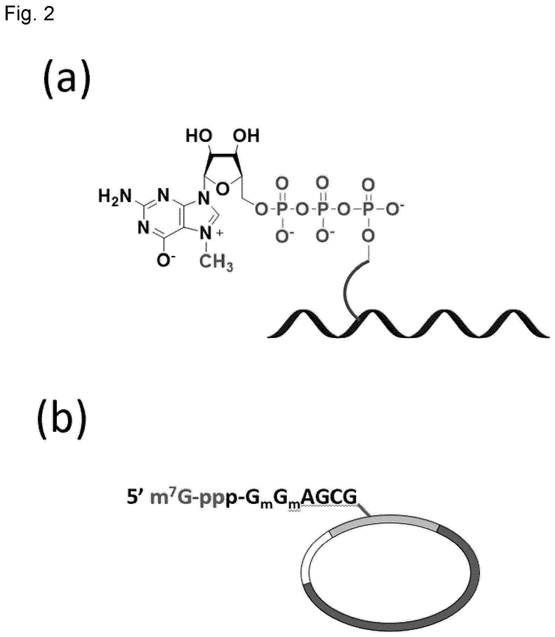

Hereinafter, the branched chain type capped RNA will be described. (a) of Fig. 2 shows a branched chain type capped RNA in a linear RNA and (b) of Fig. 2 shows a branched chain type capped RNA in a circular RNA with the structures described in Examples, respectively. In both the linear RNA and the circular RNA, the upstream region of the coding RNA has a branched chain branching from the main chain constituting the coding RNA, and the cap structure is provided in this branched chain.

The overall configuration of the coding RNA is as described above in "1. Hybridization type". In the branched chain type, a part of 5'UTR of the coding RNA has a branched chain branching from the main chain, and has a cap structure at the 5' end of this branched chain. The 5' end of the main chain of the coding RNA is phosphorylated in a linear RNA. The coding RNA may have only one translated region or two or more translated regions. When the coding RNA is linear, the coding RNA may have a normal cap structure at the 5' end.

As the branched chain type RNA, Formula (3) below can be exemplified. 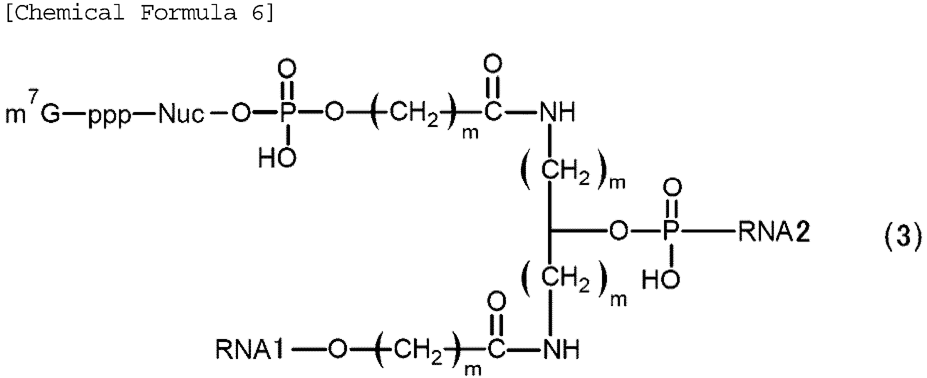 where m⁷G represents a cap, and p represents a phosphate group; Nuc represents a polynucleotide in which one or more natural or non-natural nucleotides are linked; m represents an integer of 1 to 5, and a plurality of m's may be the same as or different from each other; RNA1 and RNA2 represent RNAs constituting a main chain, and RNA1 and RNA2 are linked to a portion of a branched structure at the 3' end side and the 5' end side, respectively; and a RNA of Formula (3) may be a circular RNA in which the 5' end of RNA1 and the 3' end of RNA2 are linked to form a circular structure, or may be a linear RNA without the 5' end of RNA1 and the 3' end of RNA2 being linked.

Next, a method for producing a branched chain type capped RNA will be described. A coding RNA having a branched chain in 5'UTR can be synthesized using a normal phosphoramidite reagent used for RNA synthesis, a phosphoramidite reagent having a branched chain, and a chemical phosphorylation reagent, and using an automated nucleic acid synthesizer. Examples of the phosphoramidite having a branched chain may include 1-[5-(4,4'-dimethoxytrityloxy)pentylamide]-3-[5-levulinyloxypentylamide]-propyl-2-[(2-cyanoethyl)-(N,N-diisopropyl)]-phosphoramidite used in Examples described below. Examples of the chemical phosphorylation reagent may include Compound 1 (Formula (3) below) of Examples described below.

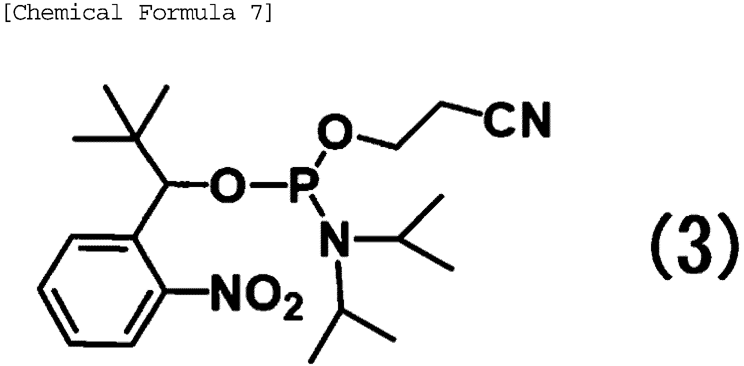

The sequence of 5'UTR of the coding RNA is synthesized from the upstream side using a normal phosphoramidite reagent or device, and a branched chain can be introduced at a desired position by using a phosphoramidite reagent having a branched chain at a position where the branched chain is to be introduced. Since a hydrophobic protective group is introduced into the 5' end of the branched chain of the coding RNA by a chemical phosphorylation reagent, this hydrophobic protective group is decomposed by photo-irradiation or the like to form a phosphate group at the 5' end of the branched chain. Then, the 5' end of the branched chain is capped using a heteroaromatic compound such as 7-methylguanosine 5'-diphosphate imidazolide salt (Im-m⁷GDP) or 2-nitroimidazole. Finally, the 5' end of the main chain of the coding RNA is phosphorylated using T4 polynucleotide kinase or the like. Thereby, a linear capped RNA is obtained. A circular coding RNA can be produced by producing a linear capped RNA and then cyclizing the linear capped RNA by connecting ends using T4 RNA ligase 2 or the like.

The branched chain type capped RNA can add a cap structure at any position of 5'UTR regardless of whether the coding RNA is linear or circular, similarly to the hybridization-type capped RNA. When the coding RNA has two or more translated regions, the translational activity of the translated region can be improved by introducing a branched chain cap into 5'UTR located upstream of a target translated region. Thereby, for example, a translation reaction can be induced from a start codon that is not used as a reading frame for protein synthesis. In the branched chain type capped RNA, a cap structure can be introduced even in a circular RNA that does not conventionally have a cap structure. This makes it possible to significantly improve the translational activity as compared with a circular RNA having no cap structure.

As shown in Examples described below, the branched chain type capped RNA tends to have higher translational activity and lower immunogenicity in a circular RNA than in a linear RNA.

The closer the position of the cap in the upstream region is to the start codon, the higher the translational activity tends to be. The position of the branch in the upstream region (that is, the position of the structure between RNA1 and RNA2 in Formula (3)) is not particularly limited, but since a cap structure is provided at the end of the branch, the translational activity tends to increase as the position of the branch is closer to the start codon. The position of the branch is preferably within a range of 1 to 50 nt, more preferably within a range of 1 to 30 nt, and particularly preferably within a range of 1 to 15 nt, on the 5' side based on the start codon.

The chain length of the branched portion of the branched chain (that is, the length of the polynucleotide of Nuc in Formula (3)) is not particularly limited, but the translational activity tends to decrease when the chain length is too long or too short. Here, the chain length of the branched chain is defined as a distance between the branched point and the 5' cap. The chain length of the branched chain is preferably within a range of 1 to 20 nt, more preferably within a range of 3 to 10 nt, and particularly preferably within a range of 5 to 8 nt. When the chain length is too short, the distance between the position of the cap structure and the branched portion is shortened, and the formation of the translation initiation factor complex, the binding of ribosomes, and the like are easily inhibited by the structure of the branched portion, and it is considered that the translational activity is lower in the branched RNA than in normal RNA.

### 3. Multi-capped type

Hereinafter, the multi-capped type capped RNA will be described. The multi- type capped capped RNA has a branch structure in which the 5' end of 5'UTR of a linear coding RNA is branched into two or more branches and the cap structure is provided in each branch.

Examples of the multi-capped type capped RNA may include a two-branch type structure represented by Formula (1) below and a three-branch type structure represented by Formula (2). 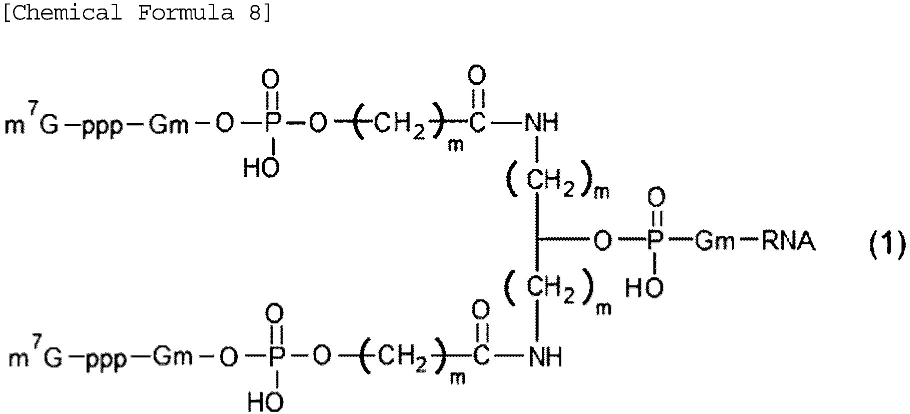 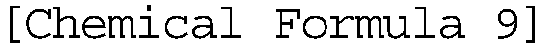 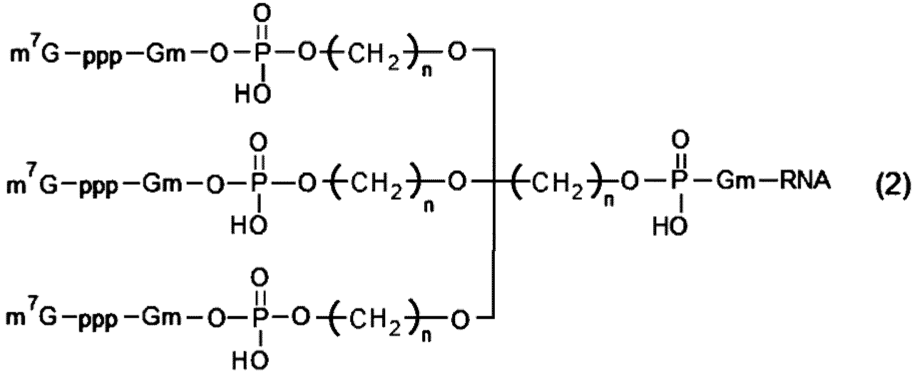 where m⁷G represents a cap, p represents a phosphate group, Gm represents a structure in which a 2'-hydroxyl group of a guanine nucleotide is methylated, m represents an integer of 1 to 5, a plurality of m's may be the same as or different from each other, n represents an integer of 1 to 5, and a plurality of n's may be the same as or different from each other.

In this case, it is preferable that Formula (1) above has a structure represented by Formula (1-1) below, and Formula (2) above has a structure represented by Formula (2-1) below.

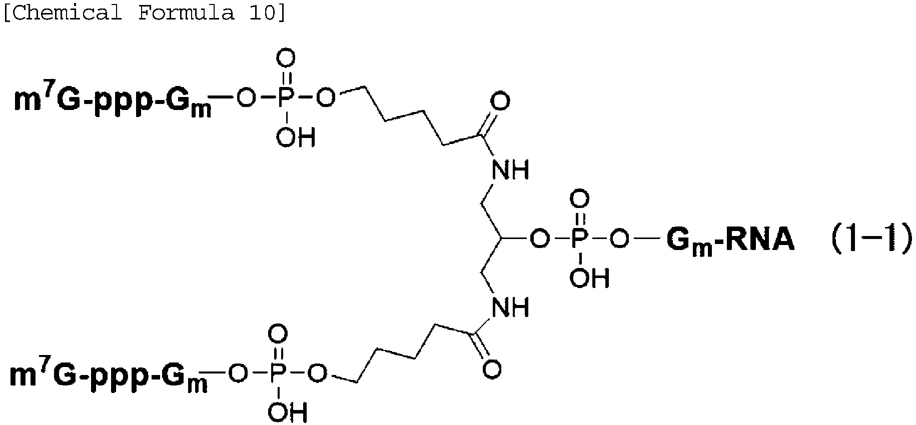

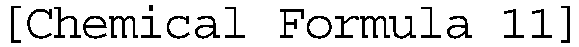

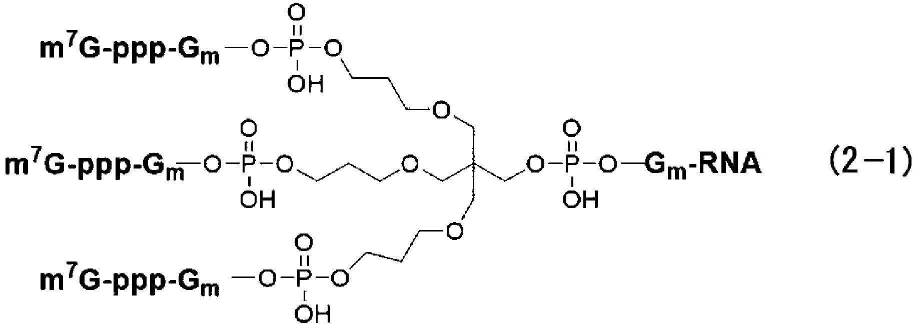

Next, a method for producing a multi-capped type capped RNA will be described. RNA before being capped can be synthesized using a normal phosphoramidite reagent used for RNA synthesis, a phosphoramidite reagent having a branched chain, and a chemical phosphorylation reagent, and using an automated nucleic acid synthesizer. Examples of the phosphoramidite having a branched chain may include 1,3-bis-[5-(4,4'-dimethoxytrityloxy)pentylamide]propyl-2-[(2-cyanoethyl)-(N,N-diisopropyl)]-phosphoramidite (two-branch type), and tris-2,2,2-[3-(4,4'-dimethoxytrityloxy)propyloxymethyl]ethyl-[(2-cyanoethyl)-(N,N-diisopropyl)]-phosphoramidite (three-branch type) used in Examples described below. Examples of the chemical phosphorylation reagent may include Compound 1 (Formula (3)) of Examples described below.

A branch structure is formed at the 5' end using a phosphoramidite reagent having a branched chain, and the sequence of 5'UTR of the coding RNA is synthesized from the upstream side using a normal phosphoramidite reagent or device, thereby synthesizing a coding RNA having a plurality of branches at the 5' end. A hydrophobic protective group is introduced into the 5' end of each branch of the coding RNA by a chemical phosphorylation reagent, and this hydrophobic protective group is decomposed by photo-irradiation or the like to form a phosphate group at the 5' end of the branched chain. Then, the 5' end of each branch of the branch structure is capped using a heteroaromatic compound such as 7-methylguanosine 5'-diphosphate imidazolide salt (Im-m⁷GDP) or 2-nitroimidazole. Thereby, a linear capped RNA in which a cap is bound to each branch is obtained.

Since the multi-capped type capped RNA has a plurality of caps at the 5' end of the main chain, the translation initiation factor constituting a ribosome is more likely to bind to the cap as compared with a normal capped RNA in which the cap is one, and the translational activity can be greatly improved by the multi-ligand effect.

### 4. Apparatus and method for producing protein

An apparatus for producing a protein of the present invention includes the above-described capped RNA, and an expression system of a eukaryotic cell capable of expressing the protein encoded by the translated region using the capped RNA as a template. In a method for producing a protein of the present invention, a capped RNA is added to an expression system of a eukaryotic cell to express a protein. Examples of the eukaryotic cell may include fungi such as yeast and filamentous fungi, plant cells, insect cells, and animal cells of human and the like, and specific examples thereof may include mouse L-cells, Ehrlich ascites cancer cells, HeLa cells, CHO cells, and Saccharomyces cerevisiae. The expression system of a eukaryotic cell may be either a cell expression system or a cell-free expression system. The introduction of the capped RNA into the cell expression system can be performed by a method such as a calcium phosphate method, a DEAE dextran method, a lipofection method, or an electroporation method. In the cell expression system, after the capped RNA is introduced into the cell, the cell is cultured under conditions such as an appropriate temperature, the cell is lysed after the culture, and then a target protein is separated and purified. In the cell-free expression system, lysates of eukaryotic cells can be used.

### 5. Detection device and detection method of target RNA

As described above, since the circular RNA does not have a cap structure, the circular RNA is not translated by itself, but a protein encoded by the circular RNA can be translated by hybridizing RNA having a cap structure. By utilizing this property, a target RNA can be detected using the circular RNA as a detection probe. Examples of the target RNA may include RNA derived from eukaryotes, viruses, and the like. Hereinafter, a detection device and a detection method of a target RNA of the present invention will be described.

The detection device of a target RNA of the present invention is a device that detects a target RNA having a cap structure at the 5' end of RNA. This detection device includes a detection probe containing a circular RNA, a means for generating a capped RNA, an expression system of a eukaryotic cell, and a means for detecting a labeled protein.

The detection probe has a translated region beginning with a start codon and encoding a labeled protein and an upstream region upstream of the start codon. This upstream region has a sequence complementary to a sequence of a part or all of a target RNA to be detected. Examples of the labeled protein may include fluorescently labelled proteins such as luciferase and green fluorescent protein (GFP), and enzyme-linked proteins such as horseradish peroxidase (HRP) and alkaline phosphatase (AP) used in Examples described below.

The means for generating a capped RNA is a means for hybridizing the above-described detection probe (circular RNA) and a target RNA. For this means, a normal reagent or device for annealing RNA can be used. Examples of such a reagent may include buffers such as Tri-HCl. Examples of the device may include a device such as a heat block or a thermal cycler that heats the reagent to 80°C or higher and then cools the reagent to room temperature.

The expression system of a eukaryotic cell is a system capable of expressing the labeled protein encoded by the translated region using the capped RNA generated above as a template, and examples thereof may include the HeLa cells described above.

As the means for detecting the labeled protein, various devices can be used depending on the type of the labeled protein. Examples of such a means may include a fluorescence measuring device when the labeled protein is a fluorescently labeled protein, and a luminescent substrate and a fluorescence measuring device when the labeled protein is an enzyme-linked protein.

The detection method of a target RNA of the present invention can be performed using the above-described detection device. First, a detection probe containing the circular RNA is prepared, and added to a sample containing a target RNA to generate a capped RNA. Next, the capped RNA is added to an expression system of a eukaryotic cell capable of expressing a labeled protein to express a labeled protein, and then the expressed labeled protein is detected. By such a method, the target RNA can be efficiently detected.

### Examples

Hereinafter, the present invention will be specifically described by way of Examples; however, the object of the present invention is not limited to these Examples. In the following Examples, the expression "%" is on a mass basis (mass percent) unless otherwise particularly specified.

### 1. Capped RNA (hybridization type)

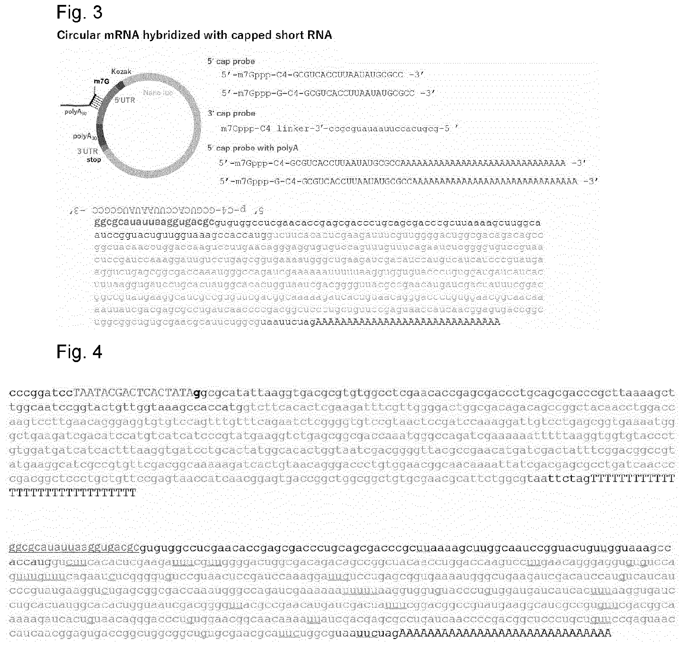

In the following, experiments on a capped RNA of a hybridization type are shown. Fig. 3 is a view showing base sequences and the like of a circular RNA and a capped probe used in this experiment. The upper left view in the drawing shows the structure of the capped RNA. As the circular RNA (coding RNA), the RNA has the 5'-side upstream region (untranslated region (denoted as "5'UTR") in the drawing) and a Kozak sequence (denoted as "Kozak") on the downstream side, and has a region encoding Nano Luc luciferase ("Nano Luc"), a stop codon ("stop"), the 3'-side untranslated region ("3'UTR"), and a polyA sequence ("polyA₃₀"). The capped probe in the drawing has a cap structure (m⁷G) at the 5' end, has a sequence complementary to a partial region of 5'UTR of the circular RNA, and has polyA30 at the 3' end. In the capped RNA, the capped probe is hybridized to 5'UTR of the circular RNA.

The upper right side in the drawing shows the arrangement of the capped probe used in this example. As the capped probe, three types of "5' cap probe" having a cap structure at the 5' end, "3' cap probe" having a cap structure at the 3' end, and "5' cap probe with polyA" having a cap structure at the 5' end and having polyA at the 3' end were used. The lower part in the drawing shows the sequence of the circular RNA. Bolded regions represented by "ggcg... cgc" are the portions where the capped probe hybridizes.

### (1) Probe RNA synthesis

RNA synthesis was performed on a 0.2 µmol scale, and an NTS M-2-MX DNA/RNA synthesizer or an NRs-4A10R7 DNA/RNA synthesizer manufactured by NIHON TECHNO SERVICE CO., LTD. was used. As the phosphoramidite used for RNA solid phase synthesis, rA(5'-DMT-2'-TOM-ribo-Adenosine (n-acetyl) OP), rC(5'-DMT-2'-TOM-ribo-Cytidine (n-acetyl) OP), rG(5'-DMT-2'-TOM-ribo-Guanosine (n-acetyl) OP), and U(5'-DMT-2'-TOM-ribo-Uridine OP) (ChemGenes) were used. As the phosphorylation reagent, 5' phosphate-ON Reagent (ChemGenes) was used. As the 2'-OMe RNA amidite, 2'-OMe-C-CE-phosphoramidite and 2'-OMe-G(iBu)-CE-phosphoramidite (ChemGenes) were used. As the LNA amidite, MBN-Ganosine(N,N-DMF)-phosphoramidite (ChemGenes) and MBN-5'-Methyl-Cytidine(N-bz)-phosphoramidite (ChemGenes) were used. As the C4 linker amidite, one synthesized according to the previous report (Angew. Chem. Int. Ed. 2011, 50, 8922-8926) was used. As the CPG, 3'-TOM-ribo Cytidine (n-acetyl) 2'-lcaa CPG 1000Å, 3'-TOM-ribo Adenosine (n-acetyl) 2'-lcaa CPG 1000Å (ChemGenes), and 3'-phosphate ON lcaa CPG 1000A were used.

After the synthesis of RNA, the CPG was transferred to a screw tube with 1.5 mL loop (SARSTEDT AG & Co. KG), 1.0 mL of 40% aq. methylamine/28% aq. ammonia (1 : 1) was added, and the mixture was incubated at 65°C for 20 minutes. The mixture was filtered using a Millex LH 0.45 µm filter, washed with sterile water (500 µL), and dried and solidified on a centrifugal evaporator. 1.0 mL of 1 M TBAF in THF was added and dissolved, and the mixture was allowed to stand still at room temperature overnight. 1.0 mL of a 1 M Tris-HCl buffer (pH 7.5) was added, and the organic solvent was distilled off with a centrifugal evaporator. Desalination was performed using a NAP-25 column (GE Healthcare). RNA was purified by reverse phase HPLC.

HPLC (HITACHI) conditions; Column: hydrosphere C18 column (4.6 × 250 mm; YMC), Mobile phase: A) 50 mM TEAA buffer (pH 7.0), 5% acetonitrile, B) acetonitrile, Gradient: 0 to 20 min B conc. 0% to 20%, Flow rate: 1.0 mL/min

RNA synthesized by 15% denatured polyacrylamide gel electrophoresis (PAGE) (acrylamide : bisacrylamide = 19 : 1 (wt/wt), 7.5 M urea) was analyzed. For PAGE, a TBE buffer (89 mM Tris-borate, 2 mM EDTA, pH 8.3) was used. Analysis by LC/MS was performed. LC/MS (agilent) conditions; ACQUITY UPLC BEH C18 Column, 0.3 mL/min, A; 8.6 mM TEA/100 mM HFIP solution, B; MeOH, 0 to 30%/20 min

Information on the sequence, molecular weight, and the like of the synthesized RNA is shown in the following table (RNA1 to RNA12 correspond to SEQ ID NOS: 1 to 12).

**[Table 1]**

| Sequence name | Sequence | Molecular weight | Measured value |
|---|---|---|---|
| RNA1 | pGCGUCACCUUAAUAUGCGCC | 6382.8 | 6383.7 |
| RNA2 | pG-C4 -GCGUCACCUUAAUAUGCGCC | 6880.1 | 5881.0 |
| RNA3 | pC4-GCGUCACCUUAAUAUGCGCC | 6534.9 | 6535.8 |
| RNA4 | GCGUCACCUUAAUAUGCGCC-C4-p | 6534.9 | 6535.2 |
| RNA5 | pCGCCGUGGAACUAUCUCCUA | 6382.8 | 6383.7 |
| RNA6 | pGCGUCACCUUAAUAUGCGCCAAAAAAAAAAAAAAAAAAAAAAAAAAAAAA | 16259.1 | 16261.4 |
| RNA7 | pG-C4-GCGUCACCUUAAUAUGCGCCAAAAAAAAAAAAAAAAAAAAAAAAAAAAAA | 16756.4 | 16759.7 |
| RNA8 | pC4-GCGUCACCUUAAUAUGCGCCAAAAAAAAAAAAAAAAAAAAAAAAAAAAAA | 16411.2 | 16414.3 |
| RNA9 | p-ₘGₘCGUCACCUUAAUAUGCGCCAAAAAAAAAAAAAAAAAAAAAAAAAAAAAA | 16287.1 | 16289.5 |
| RNA10 | pC4-mGmCGUCACCUUAAUAUGCGCCAAAAAAAAAAAAAAAAAAAAAAAAAAAAAA | 16439.2 | 16441.5 |
| RNA11 | pGCGUCACCUUAAUAUGCGCCAAAAAAAAAAAAAAAAAAAAAAAAAAAAAA | 16.373.2 | 16375.2 |
| RNA12 | pC4GCGUCACCUUAAUUAUGCGCCAAAAAAAAAAAAAAAAAAAAAAAAAAAAAA | 16525.3 | 16528.3 |
| C4; C4 link ker, m_; 2'OMe, Underlined; LNA | | | |

### (2) Capping reaction of phosphorylated RNA

The mixture was mixed in a 1.5 mL tube so as to have a final concentration of 20 µM RNA and 10 mM CaCl₂, respectively, and the mixture was freeze-dried and solidified and then dissolved in DMSO. Im (m⁷GDP) ·Na (Hongene Biotech) dissolved in DMSO was added thereto so as to have a final concentration of 10 mM and 2-nitroimidazole was added thereto so as to have a final concentration of 10 mM. This reaction solution was incubated at 55°C for 3 hours. The reaction solution was diluted two-fold with sterile water, and then isopropanol precipitation was performed. HPLC purification and analysis by LC/MS were performed.

HPLC conditions; Column: hydrosphere C18 column (4.6 × 250 mm; YMC), Mobile phase: A) 50 mM TEAA buffer (pH 7.0), 5% acetonitrile, B) acetonitrile, Gradient: 0 to 20 min B conc. 0% to 20%, Flow rate: 1.0 mL/min
LC/MS (agilent) conditions; ACQUITY UPLC BEH C18 Column, 0.3 mL/min, A; 8.6 mM TEA/100 mM HFIP solution, B; MeOH, 0 to 30%/20 min

The HPLC-purified capped RNA was precipitated using isopropanol and sodium acetate (pH 5.2).

Information on the sequence, molecular weight, and the like of the synthesized capped RNA is shown in the following table (capped RNA1 to RNA12 correspond to SEQ ID NOS: 13 to 24).

**[Table 2]**

| Sequence name | Sequence | Molecular weight | Measured value |
|---|---|---|---|
| Capped RNA1 | m7GpppGCGUCACCUUAAUAUGCGCC | 6823.0 | 6822.9 |
| Capped RNA2 | m⁷GpppG-C4-GCGUCACCUUAAUAUGCGCC | 7320.3 | 7320.2 |
| Capped RNA3 | m7Gppp-C4-GCGUCACCUUAAUAUGCGCC | 6975.1 | 6974.9 |
| Capped RNA4 | GCGUCACCUUAAUAUGCGCC-C4-pppm7G | 6975.1 | 6975.0 |
| Capped RNA5 | m7GpppCGCCGUGGAACUAUCUCCUA | 6823.0 | 6822.9 |
| Capped RNA6 | m7GpppGCGUCACCUUAAUAUGCGCCAAAAAAAAAAAAAAAAAAAAAAAAAAAAAA | 16699.3 | 16701.0 |
| Capped RNA7 | m7GgppG-C4-GCGUCACCUUAAUAUGCGCCAAAAAAAAAAAAAAAAAAAAAAAAAAAAAA | 17196.6 | 17198.7 |
| Capped RNA8 | m7Gppp-C4-GCGUCAAAUAUGCGCCAAAAAAAAAAAAAAAAAAAAAAAAAAAAAA | 16851. 4 | 16853.1 |
| Capped RNA9 | m7Gppp-ₘGₘCGUCACCUUAAUAUGCGCCAAAAAAAAAAAAAAAAAAAAAAAAAAAAAA | 16727.4 | 16728.7 |
| Capped RNA10 | m7Gppp-C4-mGmCGUCACCUUAAUAUGCGCCAAAAAAAAAAAAAAAAAAAAAAAAAAAAAA | 16879.4 | 16881.3 |
| Capped RNA11 | m7GpppGCGUCACCUUAAUAUGCGCCAAAAAAAAAAAAAAAAAAAAAAAAAAAAAA | 16813.5 | 16814.9 |
| Capped RNA12 | m7Gppp-C4-GCGUCACCUUAAUAUGCGCCAAAAAAAAAAAAAAAAAAAAAAAAAAAAAA | 16965.5 | 16966.8 |
| C4; C4 linker, m_;2'OMe, Underlined;LNA m_; 2'OMe, Underlined; LNA | | | |

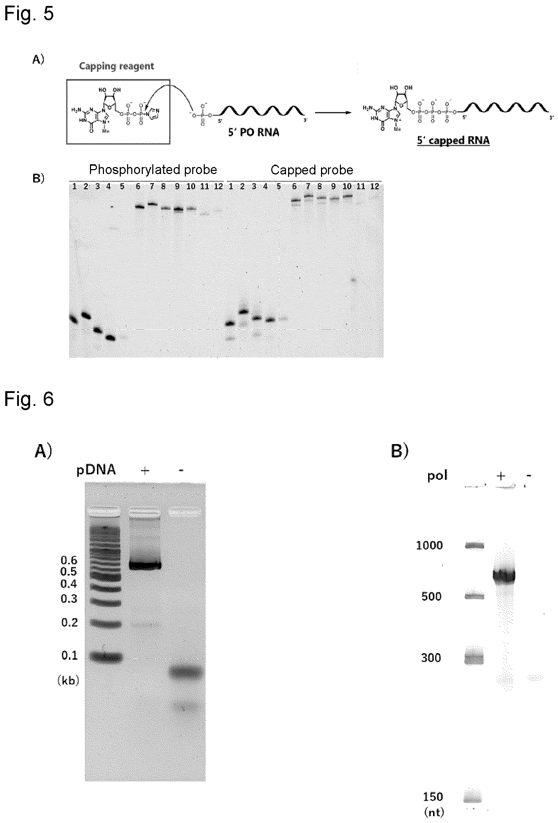

Fig. 5 is a view showing the results of synthesis experiment of a capped probe. (A) of Fig. 5 shows the synthesis scheme of the capped probe. The chemically synthesized monophosphorylated RNA was freeze-dried with calcium chloride. Im (m⁷GDP) ·Na (chemical capping reagent) and 2-nitroimidazole were added and reacted in a DMSO solution. (B) of Fig. 5 shows the results of the capped probe, and the RNA subjected to capping reaction was analyzed by PAGE and stained by SYBR Green II. As the capping reaction proceeded, the single base band shifted up due to the addition of methylguanine. As a result of PAGE analysis, it is found that the capping reaction proceeded with an efficiency of about 80 to 90%.

### (3) Synthesis of linear RNA by transcription reaction

The linear RNA was transcriptionally synthesized by T7 RNA polymerase using double-stranded DNA as a template. The upper part of Fig. 4 shows the sequence of the template DNA for the transcription synthesis of RNA (SEQ ID NO: 32) and the lower part shows the sequence of the transcribed RNA (SEQ ID NO: 31). The template double-stranded DNA was obtained by amplifying pNL-TK1.1 (Promega) by PCR reaction using PrimeSTAR HS DNA Polymerase (Takara Bio) (2.5 ng/µL DNA vector, 1× PrimeSTAR buffer, 0.2 mM dNTPs, 0.5 µM primers, 0.025 units/uL polymerase).

A transcription reaction solution (template DNA 2.5 ng/µL, 1×T7 RNA Polymerase buffer (Takara) (40 mM Tris-HCl (pH 8.0), 8 mM MgCl₂, 2 mM spermidine), 2.5 U/µL T7 RNA polymerase (Takara), 5 mM DTT, 2 mM ATP, 2 mM UTP, 2 mM, CTP, 0.4 mM GTP, 2 mM GMP) was prepared and incubated at 37°C for 2 hours. Recombinant DNase (Takara) was added to the reaction solution at 0.1 U/µL, and the mixture was incubated at 37°C for 30 minutes to decompose the template DNA.

After the reaction, phenol/chloroform extraction was performed. Subsequently, ultrafiltration was performed using Amicon Ultra 10K (Millipore) to perform alcohol precipitation.

Fig. 6 is a view showing the results of synthesis experiment of a linear RNA. A) of Fig. 6 shows the results of synthesis of double-stranded DNA as a template for a linear RNA by PCR reaction, and is a photograph of a gel analyzed on an agarose gel after the PCR reaction and stained by Gel Red. B) of Fig. 6 shows the results of transcriptional synthesis of the linear RNA and is a photograph of a gel analyzed by PAGE after the transcription reaction and stained by SYBR Green.

### (4) Cyclization reaction using T4 RNA ligase 2

The transcriptionally synthesized linear RNA was cyclized using T4 RNA ligase 2 (New England Biolabs) and 20-nt DNA (guide DNA). 0.5 µM linear RNA and 1.0 µM guide DNA were annealed in T4 RNA ligase 2 buffer (50 mM Tris-HCl (pH 7.5), 2 mM MgCl₂, 1 mM DTT and 0.4 mM ATP), then 0.1 U/µL T4 RNA ligase 2 and 10% PEG8000 were added, and the mixture was incubated at 37°C for 1 hour. The circular RNA of the ligated product and the linear RNA of the raw material were isolated by 5% denaturing PAGE.

### (5) Confirmation of cyclization using RNase R

RNA (0.18 µg/µL) was incubated in RNase R (1 unit/µL; Epicenter), 20 mM Tris-HCl (pH 8.0), 0.1 M KCl, and 0.1 mM MgCl₂ at 37°C for 10 minutes. The reaction solution was analyzed by 5% denaturing PAGE.

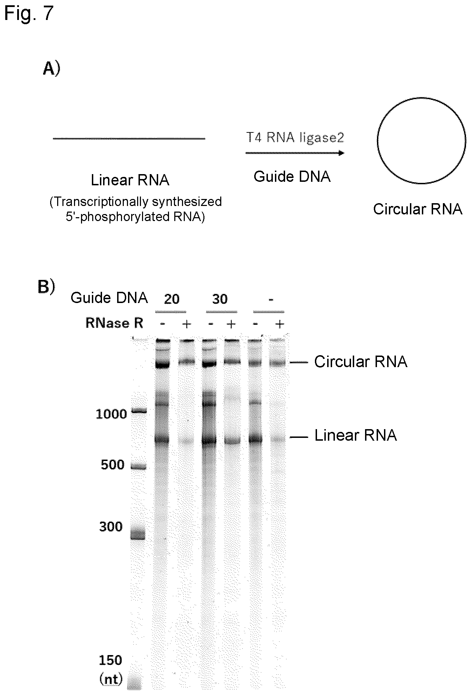

Fig. 7 is a view showing the results of cyclization experiment of a linear RNA. A) of Fig. 7 shows the synthesis scheme of the circular RNA. The transcriptionally synthesized linear RNA was annealed to the guide DNA, and a circular RNA was synthesized using T4 RNA ligase 2. B) of Fig. 7 shows the confirmation result of the circularized form, and RNase R was added to the RNA subjected to the circularization reaction and was incubated. Analysis by PAGE and staining by SYBR Green II were performed. The transcriptionally synthesized linear RNA was cyclized using T4 RNA ligase 2. The identification of circularized forms was performed by PAGE analysis with the action of an enzyme (RNase R) that selectively degrades the linear RNA. The efficiency of circularization was about 60%. The linear RNAs of the cyclized form and the raw material were cut out from the gel, and isolated and purified.

### (6) Annealing

0.2 µM capped probe and 0.1 µM linear or 0.1 µM circular RNA were annealed in a buffer (10 mM Tris-HCl (pH 7.5), 50 mM NaCl) (90°C, 3 min).

### (7) Evaluation of intracellular translational activity

HeLa cells (RIKEN Cell Bank) were cultured in Dulbecco's modified Eagle's medium (DMEM; WAKO) added with 10% fetal bovine serum (FBS; Invitrogen) (37°C, 5% CO₂). HeLa cells were seeded in a 96-well plate the day before transfection (1.0×10⁴ cell/well). The next day, the medium was removed and replaced with 100 µL/well Opti-MEM (registered trademark) (Thermo Fisher SCIENTIFIC). 0.15 µL Lipofectamine (registered trademark) MessengerMAX (registered trademark) and 20 ng mRNA were diluted in 10 µL Opti-MEM (registered trademark) and introduced into cells. After incubation at 37°C for 5 hours, 20 µL/well 1×Cell Lysis Buffer (Promega) was added to lyse the cells. Luminescence measurement was performed using Nano-Glo (registered trademark) luciferase assay (registered trademark) (Promega).

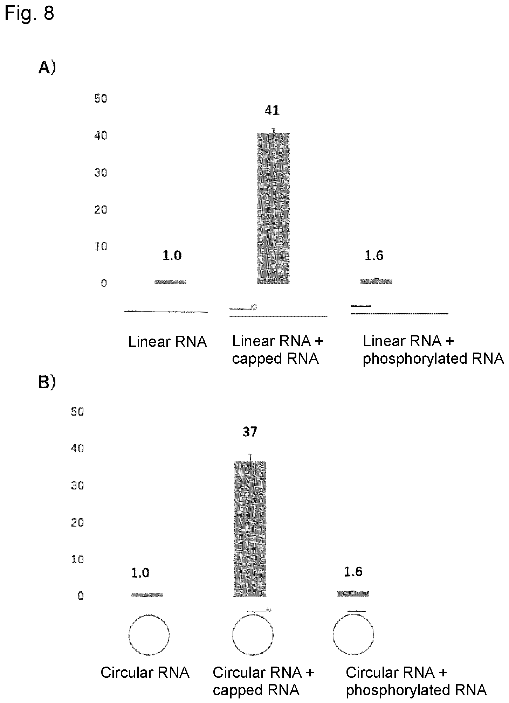

Fig. 8 is a view showing the results of relative evaluation of translational activity. A) of Fig. 8 shows the result of the translational activity of the linear RNA, and B) of Fig. 8 shows the result of the translational activity of the circular RNA. As the capped RNA probe, "capped RNA8" was used, and as the non-capped RNA probe, "RNA8" was used. The translational activity of a sample in which the RNA probe was not hybridized is relatively evaluated as "1". For any RNAs, the capped RNA was annealed and transfected into HeLa cells. After 5 hours, the cells were lysed and luminescence of NanoLuc luciferase was measured. From these results, when both the linear RNA and the circular RNA were hybridized with the capped RNA, about 40 times the translational activity promoting effect was observed as compared with the case of the RNA alone. In the mRNA hybridized with phosphorylated RNA having no cap structure, the translational activity promoting effect was not observed. This showed that the translation initiation stage was promoted in a cap-dependent manner.

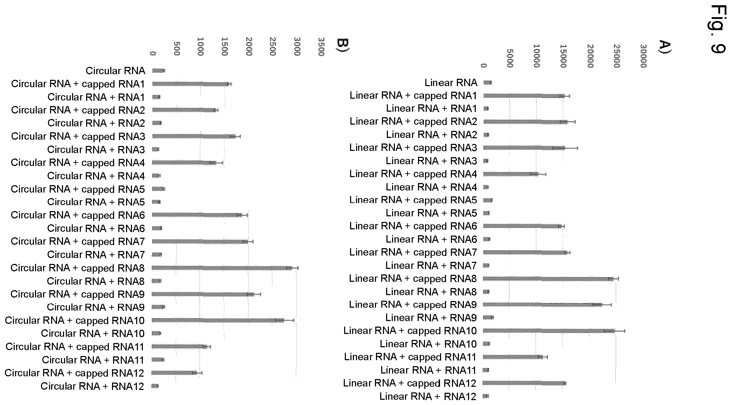

Fig. 9 is a view showing the evaluation results of the translational activity in the case of using various probes. A) of Fig. 9 shows the result of the translational activity of the linear RNA, and B) of Fig. 9 shows the result of the translational activity of the circular RNA. For any RNAs, the capped RNA was annealed and transfected into HeLa cells. After 5 hours, the cells were lysed and luminescence of NanoLuc luciferase was measured. From these results, when both the linear RNA and the circular RNA were hybridized with the capped RNA, the translational activity was promoted (capped RNA1 to RNA4, RNA6 to RNA12). In the capped RNA having a sequence not complementary to mRNA, since the translation promoting effect was not observed (capped RNA5), it was shown that the capped RNA was hybridized with mRNA to promote translation. When a C4 linker and a polyA sequence were introduced into the cap structure, the promoting effect was increased (capped RNA8 to RNA10).

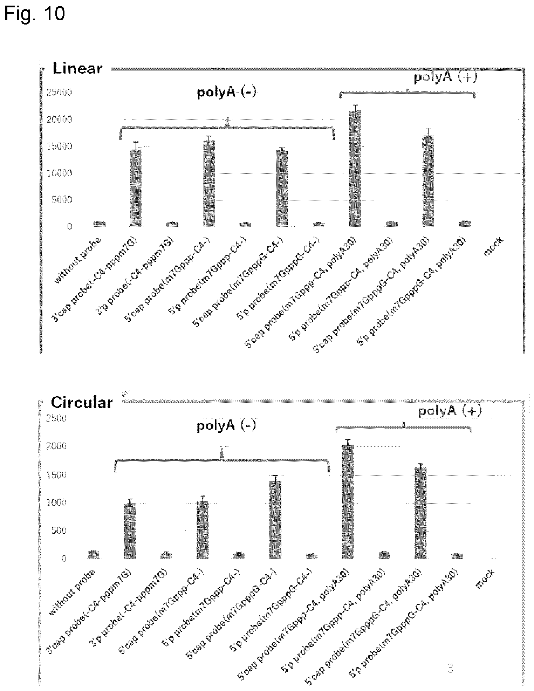

Fig. 10 is a view showing the evaluation results of the translational activity depending on the presence or absence of a polyA sequence. In the drawing, the upper part (Linear) shows the results using a linear RNA, and the lower part (Circular) shows the results using a circular RNA. "Cap probe" indicates the result of annealing the capped probe, and "p probe" indicates the result of annealing the non-capped phosphate-terminated probe. From these graphs, it is found that the translational activity is improved by annealing the capped probe in both cases of a linear RNA and a circular RNA as compared with a case where the probe is not capped. It is also found that the translational activity is slightly improved in the case of adding the polyA as compared with the case of not adding the polyA.

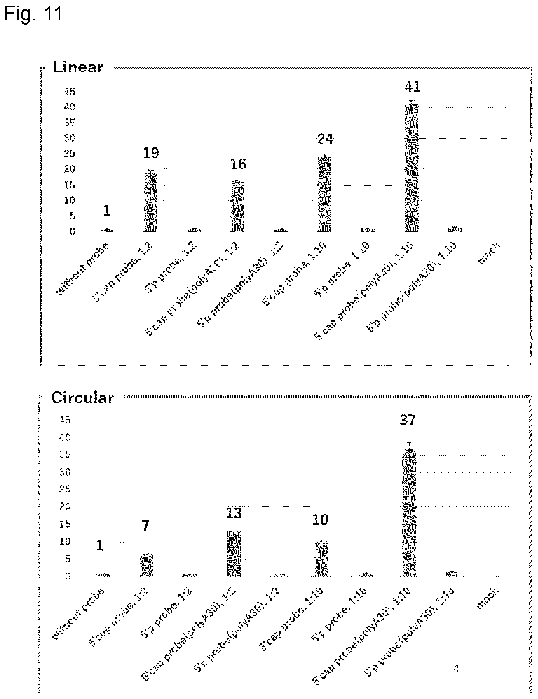

Evaluation of intracellular translational activity was performed in the same manner as in the above "(6) Evaluation of intracellular translational activity" except that the concentration of the capped probe was changed to two kinds (0.2 µM, 1 µM). The results are shown in Fig. 11. Fig. 11 is a view showing the results of evaluating the translational activity by changing the ratio of a probe RNA. From this drawing, it is found that the translational activity is higher at mRNA : probe = 1 : 10 than at mRNA : probe = 1 : 2.

### 2. Cap-containing circular RNA (branched chain type)

### (1) Chemical synthesis, purification, and chemical capping of RNA

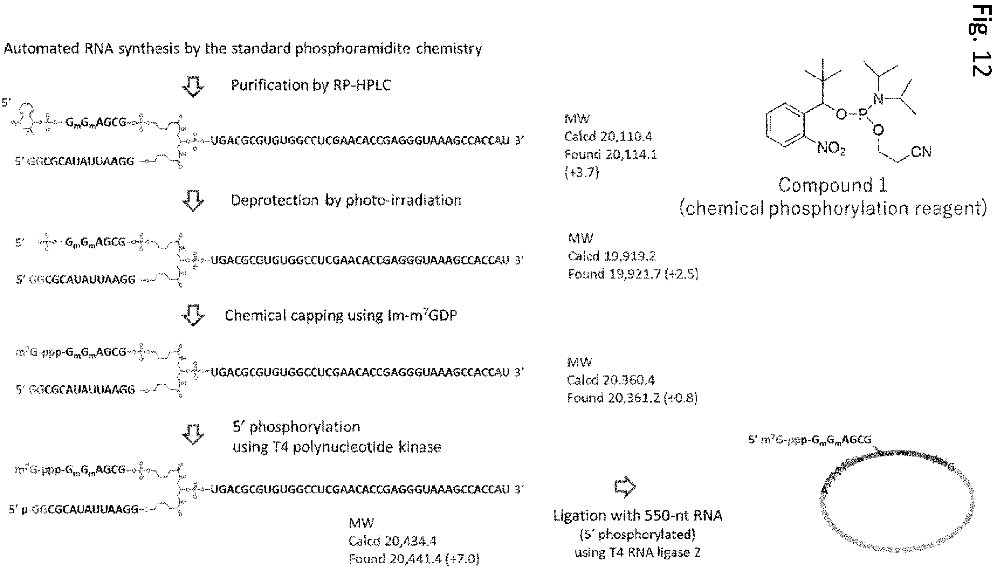

Fig. 12 is a schematic view showing the synthesis scheme of a capped branched-chain-containing RNA oligonucleotide. Hereinafter, a capped branched-chain-containing RNA oligonucleotide was synthesized based on this synthesis scheme.

### (a) RNA chemical synthesis

An oligoribonucleotide having a 54-base-long main chain and containing a branched chain phosphorylated at the 5' end was synthesized according to a conventional method with an automated nucleic acid synthesizer (NR-2A 7MX, NIHON TECHNO SERVICE CO., LTD.) using Compound 1 (Formula (3) above: see Japanese Patent Application No. 2021-111420), a commercially available phosphoramidite reagent [amidite for RNA synthesis is a product of ChemGenes (ANP-3201, ANP-3202, ANP-3203, ANP-3205), Asymmetric Doubler (Lev) Phosphoramidite (10-1981) purchased from Glen Research for branched chain introduction], and a solid phase carrier (3'-TOM-ribo Uridine CPG 1000A, ChemGenes). In order to synthesize a circular RNA not containing a branched chain, a 54-base-long RNA sequence composed only of a main chain was synthesized, and the 5' end thereof was phosphorylated by Compound 1.

After completion of the synthesis of RNA, 1 mL of a mixed solution of equal amounts of a 40% methylamine aqueous solution and concentrated ammonia water was added to the solid phase carrier, and heated at 65°C for 20 minutes. The supernatant was filtered through a Millex LH filter (0.45 µm, Merck), and then dried under reduced pressure using a centrifugal evaporator. A 1 M tetrabutylammonium fluoride/THF solution was further added to the residue, and the mixture was incubated overnight at room temperature. After desalination using a NAP-25 gel filtration column (Merck), RNA contained in the eluate was recovered by an alcohol precipitation method. This was redissolved in ultrapure water, and the target product was isolated and purified by reverse phase HPLC [system, Hitachi Chromaster (Pump 5110 and others, Hitachi High-Tech Science Corporation); column, YMC Triart Bio C4 (250 mm × 10 mm I.D., YMC CO., LTD.); Solvent A, 50 mM triethylammonium acetate (pH 7.0), 5% acetonitrile; Solvent B, acetonitrile; linear gradient 0 to 20% Solvent B (0 to 20 min); flow rate: 3 mL/min; detection wavelength, 260 nm; column temperature, 50°C].

The protective group on the phosphate group of the obtained target product was removed by irradiation with 365-nm light. MAX-305 light source device (Asahi Spectra Co., Ltd.) was used for photo-irradiation. An RNA solution (74 µM RNA, 1 mM Tris-HCl (pH 7.5), 0.1 mM EDTA) was added to each well of a transparent 96-well multi-well plate in an amount of 50 µL per well, and the plate was irradiated with light of 365 nm at a light quantity of 4 mW/cm² for 10 minutes. After irradiation, the RNA was recovered, and isolated and purified by alcohol precipitation. All the RNAs before and after deprotection of the phosphate group were analyzed by an LC/MS system (Agilent) to confirm that the target product was obtained [molecular weight theoretical value of protected phosphate group: 20,110.4, observed value: 20,114.1 (+3.7): molecular weight theoretical value of deprotected phosphate group: 19,919.2, observed value: 19,921.7 (+2.5)].

### (b) Capping reaction to 5' end of branched chain

7-Methylguanosine 5'-diphosphate imidazolide sodium salt (Im-m⁷GDP) was reacted with the phosphate group at the 5' end of the branched chain to obtain a capped product (see Japanese Patent Application No. 2021-111420). To 2 nmol (26 µL) of a raw material RNA containing a phosphate group at the 5' end of the branched chain, 5 µL of a 200 mM calcium chloride aqueous solution was added, and the mixture was frozen with liquid nitrogen and then freeze-dried. After 3 hours, 45 µL of dimethyl sulfoxide (DMSO) was added to and dissolved in a mixture of dried RNA and calcium chloride, 50 µL of an Im-m⁷GDP/DMSO solution and 5 µL of a 200 mM 2-nitroimidazole/DMSO solution were added thereto, and the mixture was warmed at 55°C for 3 hours. 122 µL of a 0.541 M sodium acetate (pH 5.2) aqueous solution was added, a part thereof was fractionated, and the efficiency of the capping reaction was evaluated. Each 5 pmol of the raw material and the RNA after the reaction was analyzed by 10% denaturing PAGE containing 7.5 M urea as a denaturant, and the band shift of the capped product was confirmed.

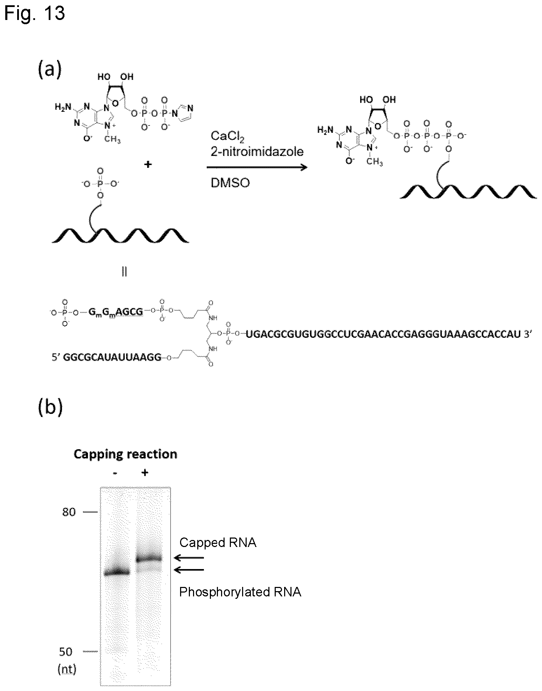

Fig. 13 is a view showing a chemical capping reaction of a branched chain. (a) of Fig. 13 shows a schematic view of the reaction (SEQ ID NO: 33). (b) of Fig. 13 shows the analysis results in 10% denaturing PAGE of the RNA before and after the reaction (containing 7.5 M urea as a denaturant). The same molar amount of RNA (5 pmol) was analyzed from the raw material RNA before the capping reaction and the reaction solution. The gel after electrophoresis was stained by a nucleic acid staining reagent SYBR Green II, and the bands were visualized. The product of the capping reaction was observed at a higher electrophoresis position than the raw material, and the reaction yield calculated from the band intensity ratio was 78%.

220 µL of 2-propanol was added to the solution after adding the aqueous sodium acetate solution, and the mixture was cooled overnight at -30°C and centrifuged (20,000 g, 25 min) to obtain a pellet. This was redissolved in ultrapure water, and the target product was isolated and purified by reverse phase HPLC [system, Hitachi LaChrom Elite (Pump L-2130 and others, Hitachi High-Tech Science Corporation); column, YMC Hydrosphere C18 (250 mm × 4.6 mm I.D., YMC CO., LTD.); Solvent A, 50 mM triethylammonium acetate (pH 7.0), 5% acetonitrile; Solvent B, acetonitrile; linear gradient 0 to 20% Solvent B (0 to 20 min); flow rate: 1 mL/min; detection wavelength, 260 nm; column temperature, 50°C].

The target product was recovered from the eluate by alcohol precipitation to obtain 1.185 nmol of a capped RNA (isolation yield: 59%). The capped RNA was analyzed by an LC/MS system (Agilent), and it was confirmed that the target product was obtained [molecular weight theoretical value: 20,360.4, observed value: 20,361.2 (+0.8)].

### (c) Phosphorylation of 5' end of main chain using T4 polynucleotide kinase

For two types of RNA, one in which a cap structure was added to a phosphate group of a branched chain and one in which a cap structure was not added, the 5' end of the main chain was enzymatically phosphorylated under the same conditions. The composition of the phosphorylation reaction is shown below. 8 µM RNA, 1 mM ATP, 50 mM Tris-HCl (pH 8.0), 10 mM magnesium chloride, 5 mM dithiothreitol, 0.2 U/µL T4 polynucleotide kinase (Takara Bio) .

After 50 µL of this reaction solution was incubated at 37°C for 1 hour, deproteinization was performed using a mixed solution of equal amounts of TE saturated phenol : chloroform, and RNA was recovered by alcohol precipitation. The RNA after phosphorylation was analyzed by an LC/MS system (Agilent) to confirm that the target product was obtained [molecular weight theoretical value of non-capped RNA: 19,993,3, observed value: 20,002.3 (+9.0): molecular weight theoretical value of capped RNA: 20,434.4, observed value: 20,441.4 (+7.0)].

### (2) Synthesis of transcribed RNA

A PCR reaction product obtained using a commercially available plasmid DNA containing the coding sequence of NanoLuc luciferase and pNL1.1.TK (Promega) as a template and two synthetic oligo-DNAs (5'-CCCGGATCCTAATACGACTCACTATAGGTCTTCACACTCGAAGATTTC-3' (SEQ ID NO: 25)/5'-TTTTTTTTTTTTTTTTTTTTTTTTTTTTTTCTAGAATTACGCCAGAATGCG-3' (SEQ ID NO: 26)) as a primer was prepared as a transcription template. The composition of the PCR solution was as follows. 1 ng/µL pNL1.1.TK, 0.3 µM primers, 0.2 mM dNTPs, 1.5 mM MgSO₄, 1×PCR Buffer for KOD -Plus- Neo, 0.02 U/µL KOD-Plus-Neo (Toyobo Co., Ltd.). The reaction solution was subjected to the following thermal cycling conditions by using a T100 thermal cycler (Bio-Rad). 94°C, 2 minutes → [98°C, 10 seconds - 55°C, 30 seconds - 72°C, 70 seconds] × 25 cycles.

The reaction solution (50 µL × 8 pieces) was collected, 0.4 mL of a mixed solution of equal amounts of TE saturated phenol : chloroform was added thereto, vigorously mixed, and centrifuged (20,000×g, 1 min) to separate an aqueous layer. 0.4 mL of chloroform was further added, and the mixture was vigorously mixed and centrifuged (20,000×g, 1 min) to separate an aqueous layer. 40 µL of a 3 M aqueous sodium acetate solution (pH 5.2) and 440 µL of 2-propanol were added thereto, and the mixture was cooled overnight at -30°C and then centrifuged (20,000×g, 20 min) to obtain DNA of a target product as a pellet. The pellet was redissolved in 200 µL of ultrapure water, the absorbance (A₂₆₀) of the solution at 260 nm was measured using NanoDrop2000 spectrometer, and the DNA concentration of the solution was calculated (A₂₆₀ 5.632; DNA concentration: 281.6 ng/µL).

A 550-base-long RNA transcript whose 5' end was phosphorylated was prepared using the DNA obtained above as a template and T7 RNA polymerase. The composition of the reaction solution was as follows. 15 ng/µL template DNA, 2 mM ATP, 2 mM UTP, 2 mM CTP, 0.5 mM GTP, 2 mM GMP, 40 mM Tris-HCl (pH 8.0), 8 mM MgCl₂, 2 mM spermidine, 5 mM DTT, 0.001 U/µL Pyrophosphatase (New England Biolabs, M2403), 2.34 ng/µL T7 RNA polymerase. 1 mL of the reaction solution was incubated at 37°C for 2 hours, 10 µL of DNase I (5 U/uL, Takara Bio) was then added, and the mixture was further incubated at 37°C for 20 minutes.

After the reaction solution was extracted with an equal amount of a mixed solution of equal amounts of saturated phenol : chloroform, and deproteinization was performed by chloroform, 100 µL of a 3 M aqueous sodium acetate solution (pH 5.2) and 1.1 mL of 2-propanol were added to the aqueous layer, and the mixture was centrifuged to obtain a crude RNA purified product as a precipitate. In order to remove nucleotides and the like contained in the obtained crude RNA purified product, the RNA was further isolated and purified by reverse phase HPLC [Hitachi LaChrom Elite (Pump L-2130 and others, Hitachi High-Tech Science Corporation); column, YMC Hydrosphere C18 (250 mm × 4.6 mm I.D., YMC CO., LTD.); Solvent A, 50 mM triethylammonium acetate (pH 7.0), 5% acetonitrile; Solvent B, acetonitrile; linear gradient 5 to 15% Solvent B (0 to 20 min); flow rate: 1 mL/min; detection wavelength, 260 nm; column temperature, 50°C], and finally 420 µg of the RNA transcript was obtained.

### (3) Circular RNA synthesis (ligase ligation, isolation)

A chemically synthesized RNA fragment whose main chain portion was composed of 54 bases and a transcriptionally synthesized RNA having a length of 550 bases were annealed in the presence of two types of template DNAs, and ligated and cyclized using an enzyme T4 RNA ligase 2. The sequences of the two types of template DNAs each composed of 30 bases were as follows.

Splint_1, 5'-ACCTTAATATGCGCCTTTTTTTTTTTTTTT-3' (SEQ ID NO: 27); Splint_2, 5'-TCGAGTGTGAAGACCATGGTGGCTTTACCC-3' (SEQ ID NO: 28) .

The composition of the enzyme reaction solution was as follows.

1 µM chemically synthesized RNA, 0.5 µM transcriptionally synthesized RNA, 2 µM Splint DNA_1, 2 µM Splint DNA_2, 20 (w/v)% PEG6000, 0.05 µg/µL T4 RNA ligase 2.

The mixture was heated at 90°C for 3 minutes and slowly cooled to room temperature over about 20 minutes before adding PEG6000 with a stock solution dose of 60 (w/v)% each and 1 µg/µL of T4 RNA ligase 2. Thereafter, PEG6000 and ligase were added so as to have the final concentration shown above, and the mixture was incubated at 37°C for 1 hour. 0.4 mL of a mixed solution of equal amounts of TE saturated phenol : chloroform was added to 320 µL of the reaction solution, vigorously mixed, and centrifuged (20,000×g, 1 min) to separate an aqueous layer. 0.4 mL of chloroform was further added, and the mixture was vigorously mixed and centrifuged (20,000×g, 1 min) to separate an aqueous layer. 40 µL of a 3 M aqueous sodium acetate solution (pH 5.2) and 440 µL of 2-propanol were added thereto, and the mixture was cooled overnight at -30°C and then centrifuged (20,000×g, 20 min).

The obtained pellet was dissolved in 10 µL of ultrapure water and separated by denaturing polyacrylamide gel electrophoresis (PAGE) [6% polyacrylamide (acrylamide/N,N'-methylenebisacrylamide, 19/1) containing 7.5 M urea as a denaturant and 25 (v/v)%formamide was prepared using a TBE buffer (89 mM Tris-boric acid, 2 mM EDTA)], a gel part containing a target cyclized RNA was cut out and crushed, and then ultrapure water was added thereto for extraction. RNA contained in the gel extract was recovered by an alcohol precipitation method and redissolved in ultrapure water. The RNA concentration was calculated based on the absorbance at 260 nm. The isolation yield of the circular RNA calculated based on the amount of the transcribed RNA used was 5 to 6%. The purity of the circular RNA after purification was confirmed by denaturing PAGE analysis.

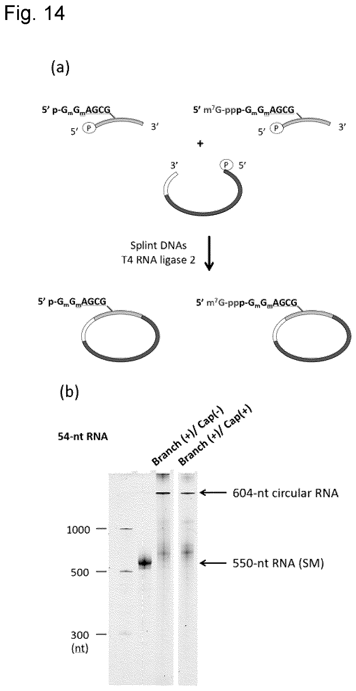

Fig. 14 is a view showing the synthesis reaction of a circular RNA. A) of Fig. 14 shows a schematic view of the reaction. Circled P represents a phosphate group at the 5' end of the RNA strand. b) of Fig. 14 shows the analysis results of a ligase ligation reaction product in 6% denaturing PAGE (containing 7.5 M urea as a denaturant, 25 (v/v)% formamide). The gel after electrophoresis was stained by a nucleic acid staining reagent SYBR Green II, and the bands were visualized.

### (4) Translation reaction in HeLa cells

HeLa cells (RIKEN Cell Bank) were seeded at 1×10⁴ cells per well of a 96-well multi-well plate the day before, and 10 ng of RNA per well was introduced into HeLa cells based on the recommended conditions of a transfection reagent (Lipofectamine MessengerMAX, Thermo). After 3 hours from transfection, the supernatant was replaced with Dulbecco's Modified Eagle Medium (Wako) medium containing 10 (w/w)% fetal bovine serum (Hyclone), and the cells were further cultured under the conditions of 37°C and 5% CO₂ for 24 hours, and then lysed. The amount of NanoLuc luciferase contained in the lysate was measured using Nano-Glo (registered trademark) Luciferase Assay System (Promega) and Mithras LB 940 plate reader (Berthold).

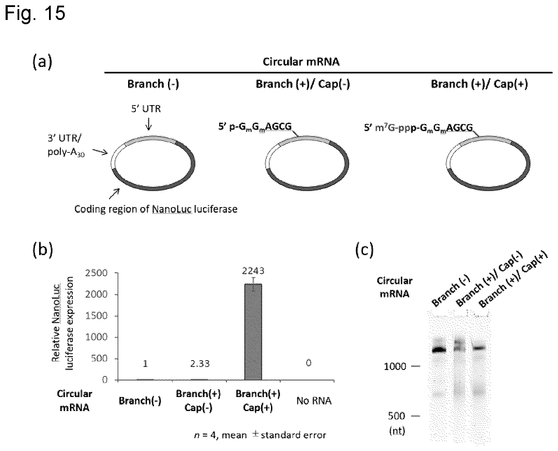

Fig. 15 is a view showing evaluation of translational activity of capped branched-chain-containing circular mRNA in human-derived cultured cells (HeLa). Fig. 15 is a view showing the results of a translation reaction using a circular RNA. (a) of Fig. 15 is a schematic view showing the structures of three types of synthesized circular mRNAs (604 bases in length).

(b) of Fig. 15 is a graph showing the results of comparing the translational activity in HeLa cells of three kinds of RNAs (unbranched, branched without a cap structure, branched with a cap structure) encoding NanoLuc luciferase. HeLa cells were seeded at 1×10⁴ cells per well of a 96-well multi-well plate the day before, and 10 ng of RNA per well was introduced into cells based using a transfection reagent (Lipofectamine MessengerMAX, 0.15 µL per well). The cells were cultured for 24 hours, then lysed, and the amount of NanoLuc luciferase contained in the lysate was evaluated using Nano-Glo (registered trademark) Luciferase Assay System (Promega).

(c) of Fig. 15 is a photograph showing the results of modified polyacrylamide electrophoresis analysis of the circular RNA used in translational activity measurement. 50 ng of RNA was heated and denatured in a loading solution containing formamide, and then electrophoresed using a 5% polyacrylamide gel containing 7.5 M urea as a denaturant. The gel after electrophoresis was stained by a nucleic acid staining reagent SYBR Green II, and the bands of the RNA were visualized.

From these drawings, it is found that the translational activity of the cap-containing circular RNA (Branch(+) Cap(+)) is 2000 times or more higher than that of the circular RNA (Branch(-)) having neither a branched chain nor a cap.

### 3. Multi-capped RNA

### (1) Synthesis of multi-capped RNA

An RNA strand and a non-nucleotide chain introduction site were synthesized using a commercially available RNA amidite (TOM-protected) reagent and Trebler Phosphoramidite, Symmetric Doubler Phosphoramidite, and Spacer Phosphoroamidite 9 (all manufactured by Glen Research) with an automated nucleic acid synthesizer. At this time, the 5' end was phosphorylated on a synthesizer using a chemical phosphorylation reagent Nb-CPR (Compound 1: Formula (3)). The deprotected and purified RNA was reacted with a chemical capping reagent (Im-m⁷GDP) to introduce a cap structure of mRNA.

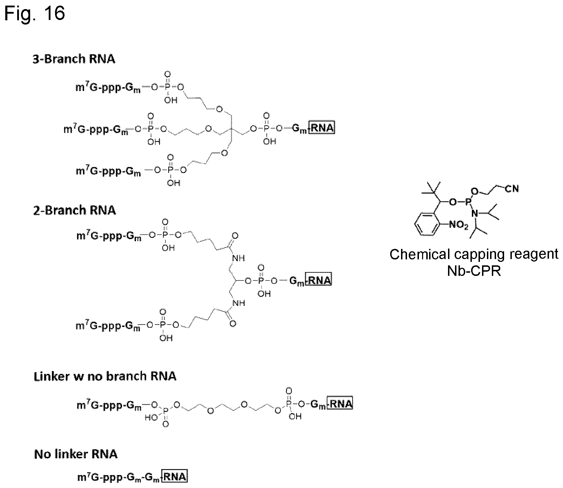

Fig. 16 is a view showing the structure of a 107-base-long RNA including a synthesized branch structure. The sequence is as follows. "p" at the 5' end indicates that it is phosphorylated, and "Gm" indicates that the 2'-hydroxyl group of the G nucleotide is methylated. "X" indicated three non-nucleotide linker structures. The structure of each different 5' end vicinity site is shown in the drawing.

5'_p_Gm_X_Gm_AGCCACCAUGGACUACAAGGACGACGACGAUAAGAUCAUCGACUAUA
AAGACGACGACGAUAAACACCACCACCACCACUGAAAAAAAAAAAAAAAAAAAA_3'

### (2) Evaluation of translational activity

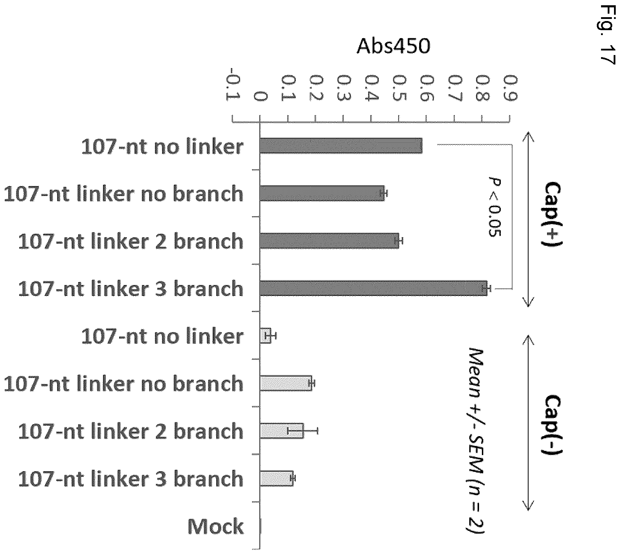

Fig. 17 is a view showing the evaluation results of the translational activity in 107-base-long HeLa cells including a synthesized branch structure/a plurality of cap structures. RNA was introduced into HeLa cells seeded on a 12-well plate at 1×10⁵ cells per well the day before by a lipofection method (Lipofectamine MessengerMAX reagent), the cells were cultured for 6 hours and then lysed, and the amount of the expressed peptide contained in the lysate was quantified by ELISA (immobilized anti-FLAG antibody HRP complex using an anti-His tag antibody as an immobilized antibody and an anti-FLAG antibody HRP complex as a detection antibody). As a result, in RNA including three cap structures, about 1.4 fold improvement in translational activity was confirmed as compared with the unmodified control sequence.

### 4. Bicistronic mRNA (linear type: Fluc-Nluc-based)

### (1) Preparation of Fluc-Nluc plasmid DNA by HiFi DNA assembly

PCR was performed using the following plasmid DNA and primer, and then the template plasmid was digested with DpnI, thereby synthesizing Fluc (Firefly luciferase) DNA, Nanoluc (Nano luciferase: Nluc) DNA, and vector DNA having a homologous sequence of 20 bases at the end. 20 µL of a reaction solution (100 ng/µL Fluc DNA, 100 ng/µL Nluc DNA, 100 ng/µL vector DNA, 1×NE Builder (registered trademark) HiFi DNA Assembly Master Mix (NEW ENGLAND BioLabs), scaled-up with MQ water) was prepared, incubated at 50°C for 1 hour, and then cooled on ice. 2 µL of the reaction solution was transformed into E. coli competent cells (ECOS (registered trademark) Competent E. coli JM109 (NIPPON GENE CO., LTD.)) and applied to an LB agar medium (containing 100 µg/mL ampicillin sodium). After overnight culture at 37°C, colonies were picked and added to 5 mL of an LB liquid medium (containing 50 ug/mL of ampicillin sodium). The mixture was cultured overnight at 37°C and 160 rpm, and the next day, the plasmid was extracted with Wizard (registered trademark) Plus SV Minipreps DNA Purification Systems (Promega) to obtain a target plasmid DNA.

### (2) Preparation of 3×FLAG-Fluc-Nluc plasmid DNA by HiFi DNA assembly

PCR was performed using the following plasmid DNA and primer to synthesize 3×FLAG DNA and pGEM-Fluc-Nluc vector having a homologous sequence of about 20 bases at the end. The PCR product was analyzed by 0.8% agarose gel electrophoresis. A target band was cut out and purified by gel extraction using Wizard (registered trademark) SV Gel and PCR Clean-Up System (Promega). 10 µL of a reaction solution (20 nM 3×FLAG DNA, 4 nM pGEM-Fluc-Nluc vector, 1×NE Builder (registered trademark) HiFi DNA Assembly Master Mix, scaled-up with MQ water) was prepared, incubated at 50°C for 1 hour, and then cooled on ice. 5 µL of the reaction solution was transformed into E. coli competent cells (ECOS (registered trademark) Competent E. coli JM109 (NIPPON GENE CO., LTD.)) and applied to an LB agar medium (containing 100 µg/mL ampicillin sodium). After overnight culture at 37°C, colonies were picked and added to 5 mL of an LB liquid medium (containing 50 ug/mL of ampicillin sodium). The mixture was cultured overnight at 37°C and 180 rpm, and the next day, the plasmid was extracted with Wizard (registered trademark) Plus SV Minipreps DNA Purification Systems (Promega) to obtain a target plasmid DNA.

### (3) Synthesis of transcription template DNA of 3×FLAG-Fluc-Nluc mRNA by PCR

50 µL of a PCR reaction solution (0.3 µM Forward primer, 0.3 µM Reverse primer, 1 ng/µL 3×FLAG-Fluc-Nluc plasmid DNA, 0.2 mM dNTPs, 1.5 mM MgSO₄, 1×PCR Buffer, 0.02 U/µL KOD-plus-Neo (TOYOBO)) was prepared, and a template DNA was synthesized by PCR. The PCR product was analyzed by 0.8% agarose gel electrophoresis. A target band was cut out and purified by gel extraction using Wizard (registered trademark) SV Gel and PCR Clean-Up System (Promega).

### (4) Synthesis of 3×FLAG-Fluc-Nluc mRNA by in vitro transcription

A transcription reaction solution (10 ng/µL template DNA, 1×T7 RNA Polymerase buffer (Takara) (40 mM Tris-HCl (pH 8.0), 8 mM MgCl₂, 2 mM spermidine), T7 RNA polymerase (Takara) 2.5 U/µL, 5 mM DTT, 2 mM ATP, 2 mM UTP, 2 mM CTP, 2 mM GTP or 0.4 mM GTP and 1.6 mM ARCA) was prepared and incubated at 37°C for 1 hour. As the non-capped mRNA, 2 mM GTP was used, and as the capped mRNA, 0.4 mM GTP and 1.6 mM ARCA were added to the reaction solution and synthesized. Recombinant DNaes I (TaKaRa) was added to the reaction solution at 0.3 U/µL, and the mixture was incubated at 37°C for 15 minutes to decompose the template DNA. After the reaction, purification was performed by phenol/chloroform extraction and alcohol precipitation.

### (5) Chemical synthesis of RNA

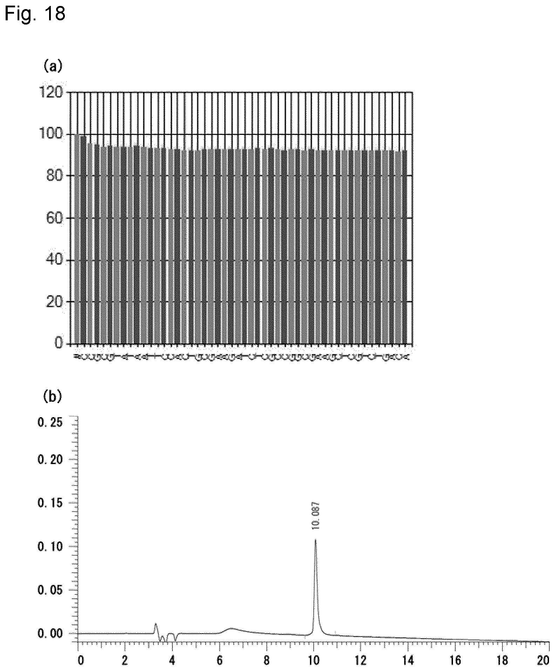

5'-GACAGUCUGCUCGAAGCGGCCGCUCUAGAAGCGTCACCTTAATATGCGCC-3' (SEQ ID NO: 30)
RNA was synthesized with an automated nucleic acid synthesizer using Universal UnyLinker Support 2000Å CPG (ChemGenes). Monophosphorylation of the 5' end was performed by synthetic amidite having a photoprotective group at the 5' end (Compound 1 described above). The synthesis of Compound 1 was performed based on the contents of "Onda, K. Master Thesis. Master thesis (Nagoya university) 2021". After the synthesis of Compound 1, the CPG was transferred to a screw tube with 1.5 mL loop (SARSTEDT AG & Co. KG), 1.0 mL of 40% aq. methylamine/28% aq. ammonia (1 : 1) was added, and the mixture was incubated at 65°C for 1 hour. The mixture was allowed to cool to room temperature, then filtered using a Millex LH 0.45 µm filter (Merck, Millipore), washed with sterile water (500 µL), and dried and solidified on a centrifugal evaporator. 1.0 mL of 1 M TBAF in THF was added and dissolved, and the mixture was allowed to stand still at room temperature overnight. 1.0 mL of a 1 M Tris-HCl buffer (pH 7.5) was added, and the organic solvent was distilled off with a centrifugal evaporator. Desalination was performed using a NAP-25 column (GE Healthcare). Purification of phosphorylated synthetic amidite-added RNA was performed by reverse phase HPLC. After acetonitrile and the TEAA buffer were removed with a centrifugal evaporator, the absorbance of the nucleic acid aqueous solution at 260 nm was measured to determine the concentration. Conditions of HPLC are shown below. The results of HPLC are shown in Fig. 18.
Column: Triart C4 (YMC) 10×250 mm, Mobile phase: A) 50 mM TEAA buffer (pH 7.0), 5% acetonitrile, B) acetonitrile, Gradient: 0 to 20 min B conc. 0% to 40%, 20 to 30 min B conc. 80%, Flow rate: 3.0 mL/min

### (6) Deprotection of RNA by photo-irradiation

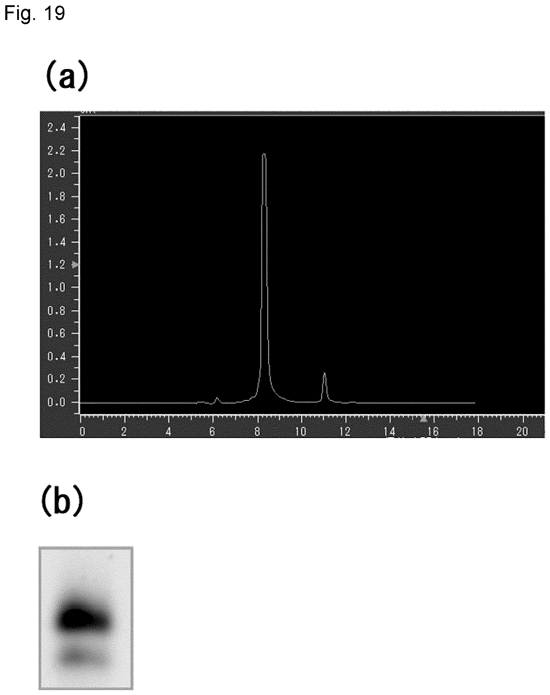

Approximately 100 µM of RNA in a 96-wel plate was irradiated with light at a wavelength of 365 nm at an intensity of 4000 µW/cm² for 10 minutes using MAX-305 (Asahi Spectra Co., Ltd.). The RNA solution after photo-irradiation was subjected to purification by confirming removal of a photoprotective group by reverse phase HPLC under the same conditions as those for purification of chemically synthesized RNA. After acetonitrile and the TEAA buffer were removed with a centrifugal evaporator, the absorbance of the nucleic acid aqueous solution at 260 nm was measured to determine the concentration. The results of the absorbance measurement are shown in Fig. 19(a).

### (7) Chemical capping of RNA

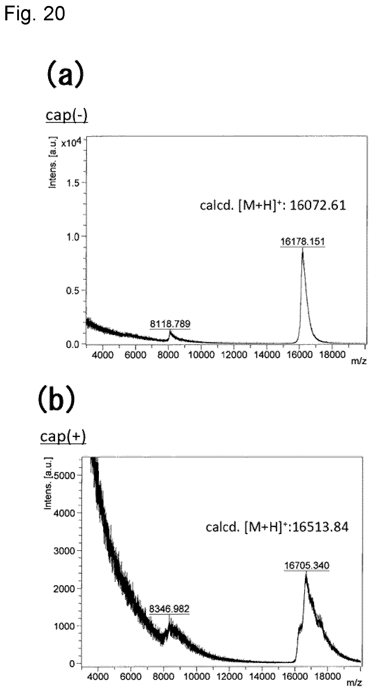

The mixture was mixed in a 1.5 mL tube so as to have a final concentration of 10 µM RNA and 10 mM CaCl₂, respectively, and the mixture was freeze-dried and solidified. A capping reagent (structure shown in the following formula) dissolved in DMSO was added thereto so as to have a final concentration of 10 mM and 2-nitroimidazole was added thereto so as to have a final concentration of 10 mM, and the mixture was scaled-up to 20 µL with DMSO. This reaction solution was incubated at 55°C for 3 hours. The reaction solution was diluted 10 times with sterile water, then 5 pmol of RNA was mixed with 2×formamide loading solution, and analysis was performed by 8% denaturing PAGE. The mixture was electrophoresed at a constant current of 20 W for 1.5 hours, stained by SYBR Green II, and the capping efficiency was calculated from the band intensity. The reaction solution diluted with sterile water was ultrafiltered with Amicon 3K (Merck, Millipore). The absorbance of the obtained RNA solution at 260 nm was measured to determine the concentration. A photograph of a gel obtained by electrophoresis of the capped RNA is shown in Fig. 19(b). The results of absorbance measurement of the capped RNA are shown in Fig. 20. As a result, the capping efficiency was 78%.

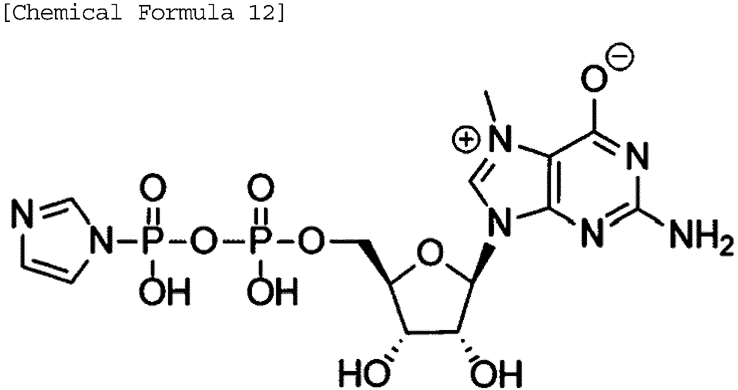

### (8) Annealing of mRNA and csRNA or sRNA

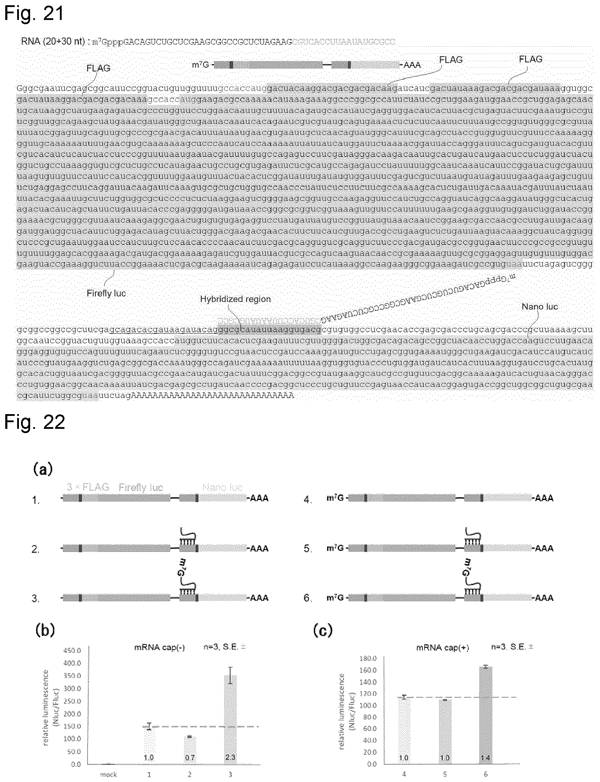

10 µL of an annealing solution (0.1 µM mRNA, 0.2 µM csRNA or sRNA 2, 50 mM NaCl) was prepared using mRNA (3×FLAG-Fluc-N1uc mRNA), csRNA (capped short RNA: capped RNA), and sRNA (short RNA: non-capped RNA) obtained above, and heated at 80°C for 5 minutes. Thereafter, the mixture was gradually cooled until the temperature reached room temperature. The sequence of the RNA after annealing (SEQ ID NO: 34) is shown in Fig. 21.

### (9) Transfection into HeLa cells and translation reaction

HeLa cells suspended in a 100 µL D-MEM medium (FUJIFILM, containing 10% FBS) at a concentration of 1×10⁴ cells/well were seeded in a 96-well plate the day before transfection. The next day, the medium was replaced with a 90 µL Opti-MEM (registered trademark) (Thermo Fisher SCIENTIFIC), and transfected with annealed mRNA-csRNA or mRNA-sRNA by using Lipofectamine (registered trademark) MessengerMAX (registered trademark) (Thermo Fisher SCIENTIFIC) (Lipofectamine (registered trademark) MessengerMAX (registered trademark) 0.3 µL, annealing solution 1 µL, scaled-up to 10 µL with Opti-MEM (registered trademark)). After incubation in an environment of 37°C and 5% CO₂ for 3.5 hours, the medium was replaced with 50 µL of a 10% FBS-containing D-MEM medium and incubated at 37°C for 3 hours. Luminescence measurement was performed using Nano-Glo (registered trademark) Dual-Luciferase (registered trademark) Reporter Assay System (Promega), and translation efficiency was determined by normalizing the luminescence amount of Nano luciferase with the luminescence amount of Firefly luciferase. The results are shown in Fig. 22. (a) of Fig. 22 shows the configuration of RNA, and (b) of Fig. 22 shows the results of translation efficiency corresponding thereto.

In bicistronic mRNA, hybridization of the Cap-probe before the start codon of the later coding region can induce translation of the coding region downstream thereof. This indicates that translation can be specifically induced in the coding region of the latter half with or without a normal cap at the 5' end.

### 5. Multi-uORF-mRNA (linear type: Rluc-based)

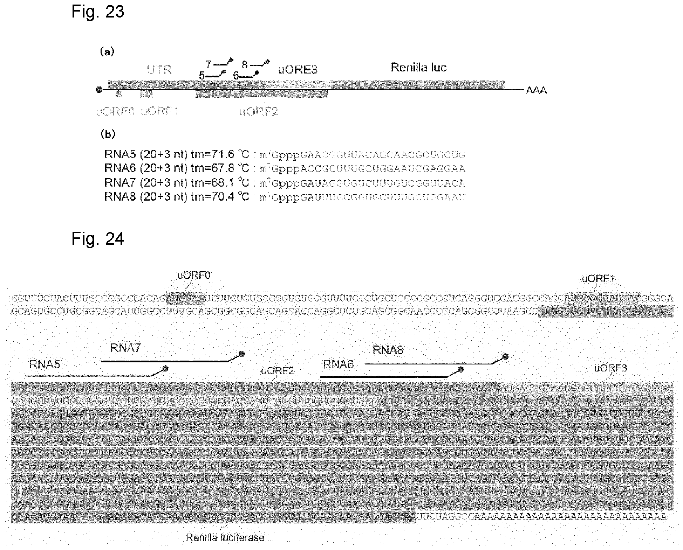

By binding a capped probe (RNA5 to RNA8 in Fig. 23(b)) to mRNA having a plurality of coding regions (Figs. 23(a) and 24: SEQ ID NO: 39), it was examined whether the translational activity of the downstream coding region to which the probe was bound was improved. A sequence of luciferase is inserted downstream of ORF3 of mRNA having a total length of 1338 nt. A complementary probe (RNA1 to RNA8) was designed and synthesized upstream of ORF3. The translational activity was evaluated by the expression level of luciferase. Details of the experiment will be described below.

### (1) Synthesis of transcription template DNA of Rluc mRNA by PCR

50 µL of a PCR reaction solution (0.3 µM Forward primer, 0.3 µM Reverse primer, 1 ng/µL Rluc plasmid DNA, 0.2 mM dNTPs, 1.5 mM MgSO₄, 1×PCR Buffer, 0.02 U/µL KOD-plus-Neo (TOYOBO)) was prepared, and a template DNA was synthesized by PCR. The PCR product was analyzed by 1% agarose gel electrophoresis. A target band was cut out and purified by gel extraction using Wizard (registered trademark) SV Gel and PCR Clean-Up System (Promega).

### (2) Synthesis of Rluc mRNA by in vitro transcription

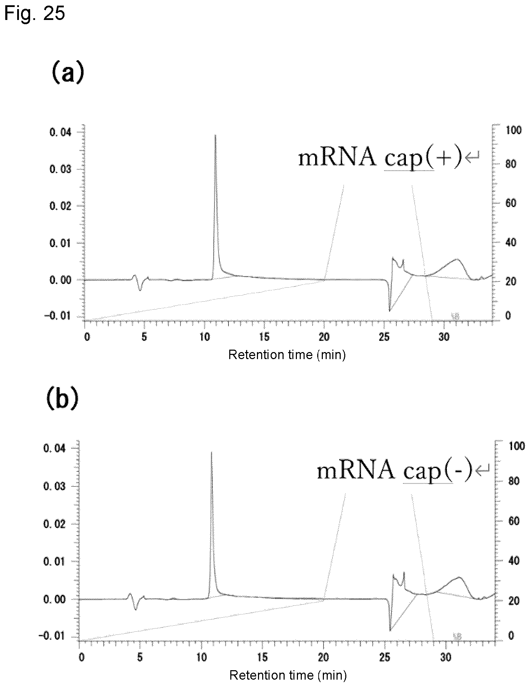

A transcription reaction solution (10 ng/µL template DNA, 1×T7 RNA Polymerase buffer (Takara) (40 mM Tris-HCl (pH 8.0), 8 mM MgCl₂, 2 mM spermidine), 2.5 U/µL T7 RNA polymerase (Takara), 5 mM DTT, 2 mM ATP, 2 mM UTP, 2 mM CTP, 2 mM GTP or 0.4 mM GTP and 1.6 mM ARCA) was prepared and incubated at 37°C for 1 hour. Recombinant DNaes I (TaKaRa) was added to the reaction solution at 0.3 U/µL, and the mixture was incubated at 37°C for 15 minutes to decompose the template DNA. After the reaction, purification was performed by phenol/chloroform extraction, alcohol precipitation, and reverse phase HPLC. Conditions of HPLC are as follows. The results of HPLC preparative post-analysis are shown in Fig. 25.
Column: Hydrosphere C18 (HS12S05-2546WT, 250 × 4.6 mm l.D. S-5 µm, 12 nm), Mobile phase: A) 50 mM TEAA buffer (pH 7.0), 5% acetonitrile, B) acetonitrile, Gradient: 0 to 20 min B conc. 0% to 20%, Flow rate: 1.0 mL/min, Column temperature: 50°C

### (3) Chemical synthesis of RNA

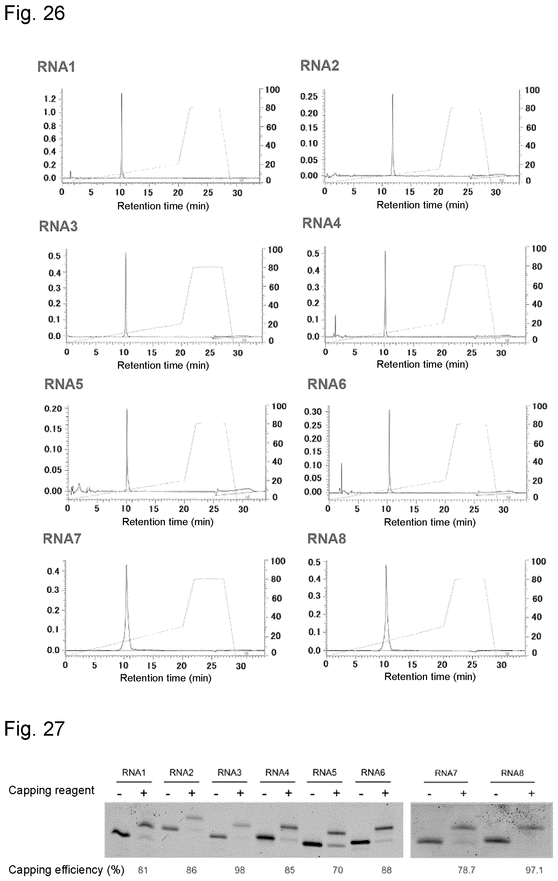

RNA was synthesized with an automated nucleic acid synthesizer using CPG and amidite shown below. After the synthesis, the CPG was transferred to a screw tube with 1.5 mL loop (SARSTEDT AG & Co. KG), 1.0 mL of 40% methylamine aq./28% ammonia aq. (1 : 1) was added, and the mixture was incubated at 65°C for 30 minutes. The mixture was allowed to cool to room temperature, then filtered using a Millex LH 0.45 µm filter (Merck, Millipore), washed with sterile water (500 µL), and dried and solidified on a centrifugal evaporator. 1.0 mL of 1 M TBAF in THF was added and dissolved, and the mixture was allowed to stand still at room temperature overnight. 1.0 mL of a 1 M Tris-HCl buffer (pH 7.5) was added, and the organic solvent was distilled off with a centrifugal evaporator. Desalination was performed using a NAP-25 column (GE Healthcare), and then purification was performed by reverse phase HPLC. The results of HPLC preparative post-analysis are shown in Fig. 26, and the results of LC-MS are shown in the following table.

### <CPG>

3'-TOM ribo Adenosine (n-acetyl) 2'-lcaa CPG 1000Å (N-3201-10, ChemGenes)/2'-TOM ribo Cytidine (n-acetyl) 3'-lcaa CPG 1000Å (N-3202-10, ChemGenes)/2'-TOM ribo Guanosine (n-acetyl) CPG 1000Å (N-3203-10, ChemGenes)/2'-TOM ribo Uridine 2'-lcaa CPG 1000Å (N-3205-10, ChemGenes)

### <Amidite>

5'-DMT- 2'-TOM ribo Adenosine (ANP-3201, ChemGenes)/2'-TOM ribo Cytidine (ANP-3202)/2'-TOM ribo Guanosine (ANP-3203, ChemGenes)/2'-TOM ribo Uridine (ANP-3205, ChemGenes)/5'-Phosphate-ON Reagent (CLP-1544, ChemGenes)

### <Conditions of HPLC>

Column: Hydrosphere C18 (HS12S05-2546WT, 250 × 4.6 mm l.D. S-5 µm, 12 nm), Mobile phase: A) 50 mM TEAA buffer (pH 7.0), 5% acetonitrile, B) acetonitrile, Gradient: 0 to 20 min B conc. 0% to 30%, Flow rate: 1.0 mL/min, Column temperature: room temperature

**[Table 3]**

| RNA5 (M.W.) | RNA6 (M.W.) | RNA7 (M.W.) | RNA8 (M.W.) |
|---|---|---|---|
| Calcd. 7465.4855 | Calcd. 7466.4695 | Calcd. 7422.3535 | Calcd. 7438.3525 |
| Obs. 7466.49 | Obs. 7467.48 | Obs. 7421.77 | Obs. 7437.76 |

### (4) Chemical capping of RNA

The mixture was mixed in a 1.5 mL tube so as to have a final concentration of 10 µM RNA and 10 mM CaCl₂, respectively, and the mixture was freeze-dried and solidified. A capping reagent (Chemical Formula 10 above) dissolved in DMSO was added thereto so as to have a final concentration of 10 mM and 2-nitroimidazole was added thereto so as to have a final concentration of 10 mM, and the mixture was scaled-up to 50 µL with DMSO. This reaction solution was incubated at 55°C for 3 hours. After the reaction, purification was performed by Amicon Ultra (0.5 mL, 3K, Merck Millipore) and alcohol precipitation, analysis was performed by 20% denaturing PAGE, and the capping efficiency was calculated from the band intensity. The results are shown in Fig. 26.

### (5) Annealing of mRNA and csRNA or sRNA

10 µL of an annealing solution (0.1 µM mRNA, 1.0 µM csRNA or sRNA, 10 mM Tris-HCl (pH 7.5), 50 mM NaCl, 10 mM EDTA (pH 8.0)) was prepared using mRNA (Rluc mRNA), csRNA (capped short RNA: capped RNA), and sRNA (short RNA: non-capped RNA) obtained above, and heated at 80°C for 5 minutes. Thereafter, the mixture was gradually cooled until the temperature reached room temperature.

### (6) Transfection into HeLa cells and translation reaction

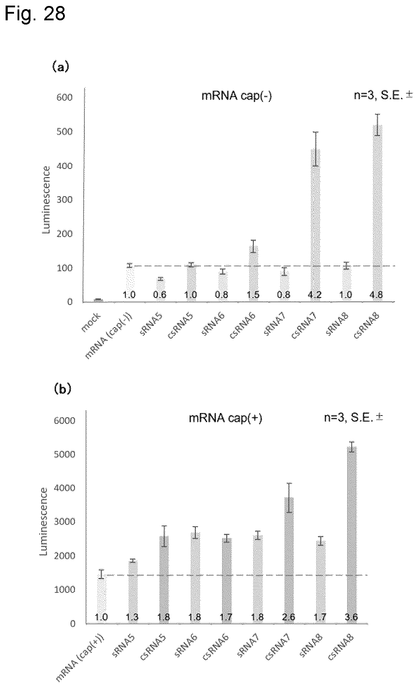

HeLa cells suspended in a 100 µL D-MEM medium (FUJIFILM, containing 10% FBS) at a concentration of 1×10⁴ cells/well were seeded in a 96-well plate the day before transfection. The next day, the medium was replaced with a 90 µL Opti-MEM (registered trademark) (Thermo Fisher SCIENTIFIC), and transfected with annealed mRNA-csRNA or mRNA-sRNA by using Lipofectamine (registered trademark) MessengerMAX (registered trademark) (Thermo Fisher SCIENTIFIC) (Lipofectamine (registered trademark) MessengerMAX (registered trademark) 0.3 µL, annealing solution 1 µL, scaled-up to 10 µL with Opti-MEM (registered trademark)). After incubation in an environment of 37°C and 5% CO₂ for 3 hours, the medium was replaced with 50 µL of a 10% FBS-containing D-MEM medium and incubated at 37°C for 3 hours. Luminescence measurement was performed using Renilla Luciferase Assay System (Promega). The results are shown in Fig. 28. From mRNA having no cap structure ((a) of Fig. 28), it is found that all of RNA5 to RNA8 have higher luminescence intensity, that is, higher translational activity in the capped RNA (csRNA) than in the non-capped RNA (sRNA). In both of the mRNA having no cap structure ((a) of Fig. 28) and the mRNA having a cap structure ((b) of Fig. 28), the translational activity was improved particularly when the cap probe 7 or 8 was bound.

### 6. Comparison between capped circular RNA and encephalomyocarditis virus (EMCV) IRES

### (1) Preparation of plasmid containing IRES

First, 1 µL of plasmid DNA containing 10 ng/µL of IRES was prepared and placed in a 1.5 mL tube. 10 µL of ECOS JM109 competent cells were placed thereto, and transformation was performed according to the ECOS 6-minute protocol. Under an alcohol lamp, the transformed sample was applied to an LB/agar medium (containing 100 µL/mL of ampicillin) and incubated in an incubator set at 37°C overnight. After 15 hours, it was confirmed that colonies were formed, and a single colony was added to 10 mL of an LB/ampicillin liquid medium. Then, the mixture was shaken overnight at 37°C. Thereafter, plasmid extraction was performed using Wizard Plus SV Minipreps DNA Purification system. The concentration of the plasmid was measured at an absorbance at 260 nm.

### (2) IRES fragment purification of purified plasmid with restriction enzyme

To 500 ng of the purified plasmid DNA containing IRES, 1 µL of 10×K buffer, 0.5 µL of EcoRI (Takara), and 0.5 µL of BamHI (Takara) were added, and MQ water was added so that the total amount was 10 µL. The sample was incubated at 37°C for 1 hour, and then it was confirmed that restriction enzyme treatment could be performed by 1% agarose electrophoresis (100 V, 30 min). Since the 645 bp band is a sequence fragment containing IRES, the band was isolated and purified by Wizard Plus SV Minipreps DNA Purification system.

### (3) Preparation of fragment containing NanoLuc luciferase

To 10 ng of plasmid DNA in which 5'UTR of pNL1.1TK was shortened by 50 nt, 10 µM to 25 pmol of each of a forward primer and a reverse primer was added. This sample was added to a PCR reaction solution (10×buffer 5 µL, 25 mM MgSO₄ 3 µL, 2 mM dNTPs 5 µL, KOD plus neo 1 µL) to prepare a sample having a total amount of 50 µL. Then, the adjusted sample was subjected to PCR under the conditions of 94°C for 2 minutes → (98°C for 10 seconds, 55°C for 30 seconds, 68°C for 3 minutes) × 25 → 68°C for 3 minutes → 4°C. The results were confirmed by 1% agarose electrophoresis (100 V, 30 min). To 48 µL of the PCR reaction solution, 0.5 µL of DpnI as a restriction enzyme was added in order to digest the plasmid before the reaction, and the mixture was incubated at 37°C for 1 hour. The PCR reaction solution was purified by a Wizard Plus SV Minipreps DNA Purification system.

### (4) In-fusion reaction

The IRES fragment and NanoLuc luciferase fragment obtained above were each measured at an absorbance at 260 nm. Then, 2.5 µL of IRES fragment (13.5 ng/µL), 1.5 µL of a linear vector containing NanoLuc luciferase (31.1 ng/µL), and 1 µL of 5×In-fusion HD Enzyme Premix (Takara) were mixed to have a total amount of 5 µL. As a negative control sample, a sample was also prepared in which 1.5 µL of a linear vector containing NanoLuc luciferase (31.1 ng/uL) and 1 µL of 5×In-fusion HD Enzyme Premix (Takara) were mixed, and the total amount was 5 µL with MQ water. Then, the adjusted sample was incubated at 50°C for 15 minutes. The incubated sample was subjected to the ECOS 6-minute protocol again, and then 50 µL of ECOS JM109 competent cells were added thereto. This sample was applied to an LB/agar medium (containing 100 µL/mL of ampicillin) under an alcohol lamp and incubated in an incubator set at 37°C overnight. After 15 hours, it was confirmed that colonies were formed, and a single colony was added to 10 mL of an LB/ampicillin liquid medium. Then, the mixture was shaken overnight at 37°C. Thereafter, plasmid extraction was performed using Wizard Plus SV Minipreps DNA Purification system. The concentration of the plasmid was measured at an absorbance at 260 nm.

### (5) Amplification of NanoLuc luciferase region from plasmid DNA

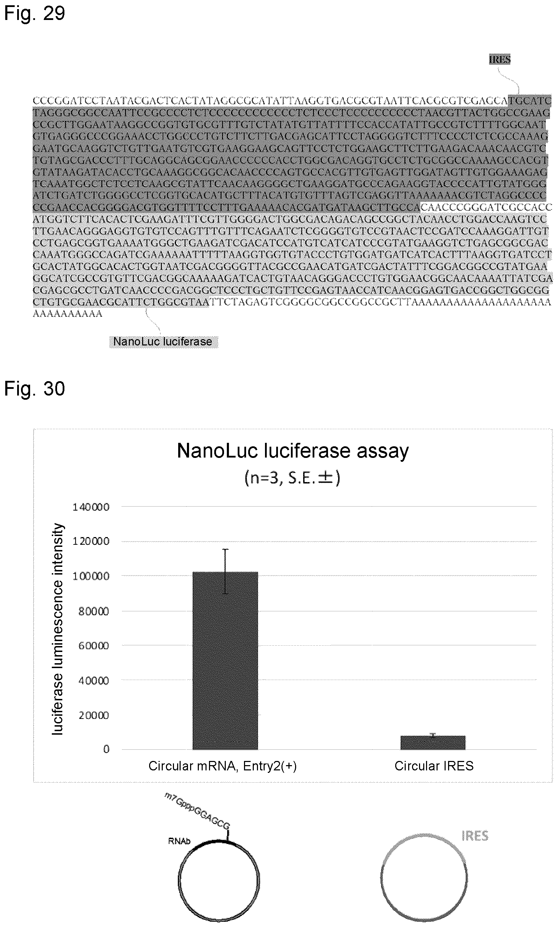

A PCR reaction solution (10×buffer 5 µL, 25 mM MgSO₄ 3 µL, 2 mM dNTPs 5 µL, forward primer 10 µM 1.5 µL, reverse primer 10 µM 1.5 µL, plasmid DNA 0.37 µL, KOD plus neo 1 µL) was prepared so as to be 50 µL. Then, the adjusted sample was subjected to PCR under the conditions of 94°C for 2 minutes → (98°C for 10 seconds, 55°C for 30 seconds, 72°C for 90 seconds) × 25 → 72°C for 3 minutes → 4°C. From the plasmid extracted by the above operation, the required NanoLuc luciferase region and IRES sequence were cut out by PCR. The sequence of the excised DNA fragment (SEQ ID NO: 40) is shown in Fig. 29. The product amplified by PCR was electrophoresed on an agarose gel (1.0%, 1×TAE, 100 V, 30 min). The PCR product was purified by a Wizard SV gel and PCR Clean-up System after PCR.

### (6) IVT (in vitro transcription) of purified DNA

The DNA (3'fragment, full length) purified above was subjected to IVT reaction. 600 µL of a transcription reaction solution (4000 ng of each purified DNA, 10×buffer 60 µL, 50 mM DTT 60 µL, 20 mM ATP, CTP, UTP, GMP 60 µL, 20 mM GTP 15 µL, T7 RNA polymerase (Takara) 30 µL, scaled-up with MQ water) was prepared and incubated at 37°C for 2 hours. Thereafter, this sample was electrophoresed by 5% denaturing PAGE (7.5 M urea, 1×TBE buffer, 26 mA, 60 min). Thereafter, the sample after electrophoresis was stained for 20 minutes using a stain prepared with 3 µL of SYBR Green II and 30 mL of MQ water. Denaturing PAGE was photographed using ChemiDoc to confirm an IVT product.

### (7) Purification of IVT product

To 600 µL of a 3'fragment and full-length IVT product, 20 µL of DNase I (Takara) was added, and the mixture was incubated at 37°C for 15 minutes. Thereby, DNA remaining in the IVT reaction was removed. Next, 600 µL of neutral phenol and 600 µL of chloroform were added, and the mixture was centrifuged with a centrifuge at 14500 rpm for 15 seconds. Thereafter, 600 µL of chloroform was added thereto, and centrifugation was performed in the same manner with a centrifuge. Thereby, proteins including an enzyme were removed. Next, centrifugation was performed at room temperature for 5 minutes using a centrifugal evaporator. Thereby, chloroform not suitable for Amicon was removed. Next, Amicon 10K was used. Thereby, low-molecular-weight one was removed. Finally, 100 µL of isopropanol and 20 µL of a 3 M aqueous sodium acetate solution (pH 5.2) were added to 100 µL of the obtained mixture using Amicon, and the mixture was allowed to stand still at -30°C for 1 hour. Thereafter, centrifugation was performed with a centrifuge under the conditions of 14500 rpm, room temperature, and 30 minutes. The supernatant was removed so that the pellet obtained by centrifugation was not sucked, and 1 mL of 80% ethanol was then added and washed. After centrifugation for 2 minutes under the same conditions, the supernatant was removed and allowed to stand still for 5 minutes. Finally, it was dissolved by centrifugation with 200 µL of MQ water three times to purify 3'fragment and full-length RNA.

### (8) HPLC purification of IVT product

As described above, the purification operation of the sample was performed after the IVT reaction, but double-stranded RNA, triphosphoric acid, and the like were not removed in this operation. These components produce effects such as a decrease in yield and translational activity when a ligation reaction or a translation reaction is verified in cells, and therefore, they were removed by HPLC. HITACHI HPLC system was used. The conditions were as follows: Hydrosphere C18: Solvent A, 50 mM TEAA (pH 7.0), 5% ACN; Solvent B, ACN; Gradient: 0 to 20% B/0 to 20 min; Flow rate, 1 mL/min; Column temperature, 50°C; Column size: 250 × 4.6 mm l.D, S-5 µL, 12 nm; detection wavelength, 260 nm. After fractionation by HPLC, acetonitrile was removed by concentration under reduced pressure. Then, after alcohol precipitation, the absorbance at 260 nm was measured and used for future experiments.

### (9) Isolation and purification of circular mRNA containing IRES

The target RNA was subjected to cyclization reaction under optimized conditions. The cyclization reaction was performed using 1 µM of a guide strand, 0.4 µM of 3'fragment, 0.4 µM of RNA, 10×T4 RNA ligase 2 buffer, 60% PEG, and 0.025 µg/µL of T4 RNA ligase 2. After electrophoresis with 6% dPAGE (7.5 µM urea, 1×TBE buffer, 30 mA, 180 min), bands thought to be a circular mRNA were cut with a cutter and placed in a 1.5 mL tube under ultraviolet radiation on a TLC plate. Thereafter, a total of 500 µL of MQ water was added to the tube and shaken with a shaker overnight. Then, after centrifugation at 14500 rpm for 1 minute at room temperature, the supernatant was recovered, 400 µL of MQ water was added to the remaining gel, and the same centrifugation was performed to recover the supernatant. Subsequently, the supernatant was filtered in two portions using Millex LCR 13 mm. Then, the sample after filtration was subjected to purification operation of circular mRNA by isopropanol precipitation. The concentration of the purified circular mRNA was measured at an absorbance of 260 nm to prepare a sample for cell experiment.

### (10) Confirmation of circular mRNA by ribonuclease

To 10 ng of the circular mRNA purified by the above isolation and purification operation, RNase R (Epicenter, 20 U/uL) was added so as to be 1 U/µL, and the mixture was scaled-up to 5 µL with MQ water and then incubated at 37°C for 15 minutes. Then, the circular mRNA was purified by phenol/chloroform extraction and isopropanol precipitation. Thereafter, the resultant product was electrophoresed by 5% dPAGE (7.5 µM urea, 1×TBE buffer, 30 mA, 240 min). The gel after electrophoresis was stained by SYBR Green II for 30 minutes, and the electrophoresis result was confirmed with ChemiDoc.

### (11) Seeding of human cultured cells

Human cultured cells (HeLa) cultured in the dish were washed twice with 3 mL of PBS (Phosphate buffered saline, nacalai tesque), and 1 mL of a 0.05% trypsin solution was added to degrade the cells, then the cells were incubated for 1 minute. Thereafter, 3 mL of the medium was added to stop tryptic degradation. The sample was centrifuged (Multipurpose refrigerated centrifuge EX-126, 200G, 3 min), the supernatant was removed, then the sample was resuspended in a medium, and cell counting was performed using 20 µL of the supernatant. Thereafter, the cells were adjusted to 10×10⁴ cells with a medium, and then 100 µL each was added thereto to make 10×10³ cells per well, and then incubated for 24 hours under the conditions of 37°C and 5% CO₂.

### (12) Transfection of circular mRNA into human cultured cells

First, an experiment was performed in which a sample whose translation amount was measured in human cultured cells was 5 ng per well and 3 wells per sample. After removing the medium from the incubated sample, the sample was washed once with 150 µL of PBS (Phosphate buffered saline, nacalai tesque). Cells were added to a multi-well plate for cell culture (96 well). Then, human cultured cells were cultured in a multi-well plate for cell culture (D-MEM, high glucose, containing phenol red, 37°C, 5% CO₂). Lipofectamine Messenger MAX transfection reagent (Invitrogen, 0.3 µL/well) and Opti-MEM (10 µL/well) were mixed with the cultured sample. 33 µL each of the mixed solution was added to the sample RNA. 89 µL each of Opti-MEM was added to each well, and then 11.3 µL each of the prepared RNA/Lipo reagent was added. After incubation under the conditions of 37°C and 5% CO₂ for 3 hours, the supernatant was removed, and the medium was replaced with D-MEM.

### (13) Luminescence measurement of circular mRNA in human cultured cells

Luminescence measurement of human cultured cells was performed 24 hours after the transfection. The medium was sucked from a multi-well plate for cell culture (96 well), and then washed once with 150 µL of PBS. Luminescence measurement of the sample after washing was performed using Nano Glo luciferase Assay System (Promega). First, 20 µL of 1×Glo Lysis Buffer per well was added. This was incubated at room temperature for 5 minutes, and the lysate of each cell was added to a 96-well white plate in an amount of 2 µL per well. To each well, 0.1 µL of Nano Glo luciferase assay substrate and 10 µL of Nano-Glo luciferase assay buffer were added. After the reagent was added, the measurement was performed with a plate reader (Comparative Example). The results are shown in Fig. 30.

An evaluation experiment using the above human cultured cells was also performed using the cap-containing circular RNA (Branch(+) Cap(+)) in the above "2. Cap-containing circular RNA (branched chain type)". The results are also shown in the drawing. From this drawing, it was found that the expression level of luciferase in the cap-containing circular RNA was 10 times that in the method using the existing IRES.

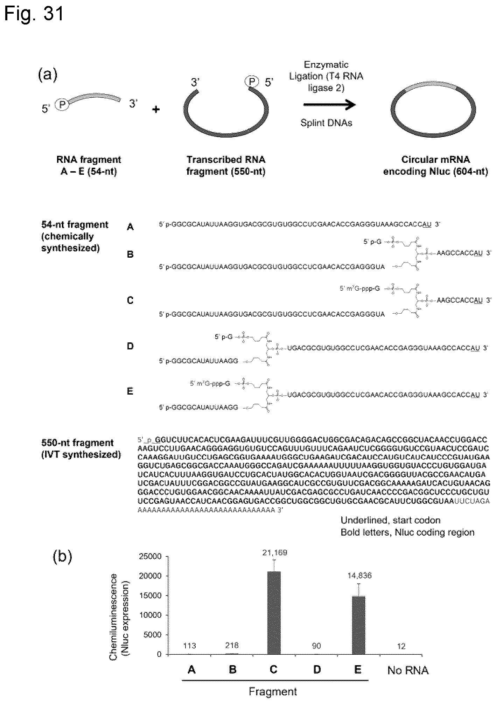

### 6. Synthesis of circular mRNA composed of two fragments of chemically synthesized RNA fragment (54 bases in length) and transcriptionally synthesized RNA fragment (550 bases in length) (Figs. 31 to 33)

An oligoribonucleotide having a 54-base-long main chain and containing a branched chain phosphorylated at the 5' end was synthesized according to a conventional method with an automated nucleic acid synthesizer (NR-2A 7MX or NRs-4A10R7NP, NIHON TECHNO SERVICE CO., LTD.) using a photodegradable protective group-containing chemical phosphorylation reagent (Japanese Patent Application No. 2021-111420), a commercially available phosphoramidite reagent [product from ChemGenes (ANP-3201, ANP-3202, ANP-3203, ANP-3205) for RNA strand synthesis, Asymmetric Doubler (Lev) Phosphoramidite (10-1981) purchased from Glen Research for branched chain introduction], and a solid phase carrier (3'-TOM-ribo Uridine CPG 1000A, ChemGenes). In order to synthesize a circular RNA not containing a branched chain, a 54-base-long RNA sequence (SEQ ID NO: 43) composed only of a main chain was synthesized. After completion of the synthesis, 1 mL of a mixed solution of equal amounts of a 40% methylamine aqueous solution and concentrated ammonia water was added to the solid phase carrier, and heated at 65°C for 20 minutes to performed cutting-out from the solid phase single substance and deprotection of the nucleobase moiety. The supernatant was filtered through a Millex LH filter (0.45 µm, Merck), and then dried under reduced pressure using a centrifugal evaporator. A 1 M tetrabutylammonium fluoride/THF solution was further added to the residue, and the mixture was incubated overnight at room temperature. After desalination using a NAP-25 gel filtration column (Merck), RNA contained in the eluate was recovered by an alcohol precipitation method. This was redissolved in ultrapure water, and the target product was isolated and purified by reverse phase HPLC.
[Hitachi LaChrom Elite or Chromaster system (Hitachi High-Tech Science Corporation); column, YMC Triart Bio C4 (250 mm × 10 mm I.D., YMC CO., LTD.); Solvent A, 50 mM triethylammonium acetate (pH 7.0), 5% acetonitrile; Solvent B, acetonitrile; linear gradient 0 to 20% Solvent B (0 to 20 min); flow rate: 3 mL/min; detection wavelength, 260 nm; column temperature, 50°C].

The protective group on the phosphate group of the obtained target product was removed by irradiation with 365-nm light. MAX-305 light source device (Asahi Spectra Co., Ltd.) was used for photo-irradiation. An RNA solution was added to each well of a transparent 96-well multi-well plate in an amount of 50 µL per well, and the plate was irradiated with light of 365 nm at a light quantity of 4 mW/cm² for 10 minutes. After irradiation, the RNA was recovered, and isolated and purified by alcohol precipitation. All the RNAs before and after deprotection of the phosphate group were analyzed by an LC/MS system (Agilent), and it was confirmed that the target product was obtained. Then, 7-methylguanosine 5'-diphosphate imidazolide sodium salt (Im-m⁷GDP) was reacted with the phosphate group at the 5' end of the branched chain to obtain a capped RNA strand (WO2021172204A1). To 2 nmol (26 µL) of a raw material RNA containing a phosphate group at the 5' end of the branched chain, 5 µL of a 200 mM calcium chloride aqueous solution was added, and the mixture was frozen with liquid nitrogen and then freeze-dried. After 3 hours, 45 µL of dimethyl sulfoxide (DMSO) was added to and dissolved in a mixture of dried RNA and calcium chloride, 50 µL of an Im-m⁷GDP/DMSO solution and 5 µL of a 200 mM 2-nitroimidazole/DMSO solution were added thereto, and the mixture was warmed at 55°C for 3 hours. 122 µL of a 0.541 M sodium acetate (pH 5.2) aqueous solution was added, a part thereof was fractionated, and the efficiency of the capping reaction was evaluated. Each 5 pmol of the raw material and the RNA after the reaction was analyzed by 10% denaturing PAGE containing 7.5 M urea as a denaturant, and the band shift of the capped product was confirmed. 220 µL of 2-propanol was added to the solution after adding the aqueous sodium acetate solution, and the mixture was cooled overnight at -30°C and centrifuged (20,000 g, 25 min) to obtain a pellet. This was redissolved in ultrapure water, and the target product was isolated and purified by reverse phase HPLC.
[Hitachi LaChrom Elite or Chromaster system (Hitachi High-Tech Science Corporation); column, YMC Hydrosphere C18 (250 mm × 4.6 mm I.D., YMC CO., LTD.); Solvent A, 50 mM triethylammonium acetate (pH 7.0), 5% acetonitrile; Solvent B, acetonitrile; linear gradient 0 to 20% Solvent B (0 to 20 min); flow rate: 1 mL/min; detection wavelength, 260 nm; column temperature, 50°C].

The target product was recovered from the eluate by alcohol precipitation to obtain a capped RNA. As described above, the cap structure was introduced into the branched chain, and then the 5' end of the main chain RNA was phosphorylated using an enzyme T4 polynucleotide kinase. The composition of the phosphorylation reaction is shown below. 8 µM RNA, 1 mM ATP, 50 mM Tris-HCl (pH 8.0), 10 mM magnesium chloride, 5 mM dithiothreitol, 0.2 U/µL T4 polynucleotide kinase (Takara Bio). After this reaction was incubated at 37°C for 1 hour, deproteinization was performed using a mixed solution of equal amounts of TE saturated phenol : chloroform, and RNA was recovered by alcohol precipitation.

The PCR reaction was performed by using a commercially available plasmid DNA containing the coding sequence of NanoLuc luciferase (Nluc) and pNL1.1.TK (Promega) as a template and two synthetic oligo-DNAs (5'-CCCGGATCCTAATACGACTCACTATAGGTCTTCACACTCGAAGATTTC-3' (SEQ ID NO: 25)/5'-TTTTTTTTTTTTTTTTTTTTTTTTTTTTTTCTAGAATTACGCCAGAATGCG-3' (SEQ ID NO: 26)) as a primer. The composition of the reaction solution was as follows. 1 ng/µL pNL1.1. TK, 0.3 µM oligo DNA, 0.2 mM dNTPs, 1.5 mM MgSO₄, 1×PCR Buffer for KOD-Plus-Neo, 0.02 U/µL KOD -Plus- Neo (Toyobo Co., Ltd.). The reaction solution was subjected to the following thermal cycling conditions by using a T100 thermal cycler (Bio-Rad). 94°C, 2 minutes → [98°C, 10 seconds → 55°C, 30 seconds → 72°C, 70 seconds] × 25 cycles. The reaction solution (50 µL × 8 pieces) was collected, 0.4 mL of a mixed solution of equal amounts of TE saturated phenol : chloroform was added thereto, vigorously mixed, and centrifuged (20,000×g, 1 min) to separate an aqueous layer. 0.4 mL of chloroform was further added, and the mixture was vigorously mixed and centrifuged (20,000×g, 1 min) to separate an aqueous layer. 40 µL of a 3 M aqueous sodium acetate solution (pH 5.2) and 440 µL of 2-propanol were added thereto, and the mixture was cooled overnight at -30°C and then centrifuged (20,000×g, 20 min) to obtain DNA of a target product as a pellet. A 550-base-long RNA transcript whose 5' end was phosphorylated was prepared using the DNA as a template and T7 RNA polymerase. The composition of the reaction solution was as follows.

15 ng/µL template DNA, 2 mM ATP, 2 mM UTP, 2 mM CTP, 0.5 mM GTP, 2 mM GMP, 40 mM Tris-HCl (pH 8.0), 8 mM MgCl₂, 2 mM spermidine, 5 mM DTT, 0.001 U/uL Pyrophosphatase (New England Biolabs, M2403), 2.34 ng/µL T7 RNA polymerase.

1 mL of the reaction solution was incubated at 37°C for 2 hours, 10 µL of DNase I (5 U/µL, Takara Bio) was then added, and the mixture was further incubated at 37°C for 20 minutes. After the reaction solution was extracted with an equal amount of a mixed solution of equal amounts of saturated phenol : chloroform, and deproteinization was performed by chloroform, a 1/10 amount of a 3 M aqueous sodium acetate solution (pH 5.2) and an equal amount of 2-propanol were added to the aqueous layer, and the mixture was centrifuged to obtain a crude RNA purified product as a precipitate. The obtained RNA transcript was isolated and purified by reverse phase HPLC [Hitachi LaChrom Elite or Chromaster system (Hitachi High-Tech Science Corporation); column, YMC Hydrosphere C18 (250 mm × 4.6 mm I.D., YMC CO., LTD.); Solvent A, 50 mM triethylammonium acetate (pH 7.0), 5% acetonitrile; Solvent B, acetonitrile; linear gradient 5 to 15% Solvent B (0 to 20 min); flow rate: 1 mL/min; detection wavelength, 260 nm; column temperature, 50°C], and finally 420 µg of the RNA transcript was obtained.

A chemically synthesized RNA fragment whose main chain portion was composed of 54 bases and a transcriptionally synthesized RNA having a length of 550 bases were annealed in the presence of two types of template DNAs, and ligated and cyclized using an enzyme T4 RNA ligase 2. The sequences of the two types of template DNAs each composed of 30 bases were as follows. Splint_1, 5'-ACCTTAATATGCGCCTTTTTTTTTTTTTTT-3' (SEQ ID NO: 27); Splint_2, 5'-TCGAGTGTGAAGACCATGGTGGCTTTACCC-3' (SEQ ID NO: 28).

The composition of the enzyme reaction solution was as follows. 1 µM chemically synthesized RNA, 0.5 µM transcriptionally synthesized RNA, 2 µM Splint DNA_1, 2 µM Splint DNA_2, 50 mM Tris-HCl (pH 7.5), 2 mM MgCl₂, 1 mM DTT, 400 µM ATP, 10 (w/v)% PEG6000, 0.05 µg/µL T4 RNA ligase 2. The mixture was heated at 90°C for 3 minutes and slowly cooled to room temperature over about 20 minutes before adding PEG6000 with a stock solution dose of 60 (w/v) % each and 1 µg/µL of T4 RNA ligase 2. Thereafter, PEG6000 and ligase were added so as to have the final concentration shown above, and the mixture was incubated at 37°C for 1 hour. An equal amount of a mixed solution of equal amounts of TE saturated phenol : chloroform was added to the reaction solution, vigorously mixed, and centrifuged (20,000×g, 1 min) to separate an aqueous layer. An equal amount of chloroform was further added, and the mixture was vigorously mixed and centrifuged (20,000×g, 1 min) to separate an aqueous layer. A 1/10 amount of a 3 M aqueous sodium acetate solution (pH 5.2) in the aqueous layer and an equal amount of 2-propanol were added thereto, and the mixture was cooled at -30°C for 1 hour or more and then centrifuged (20,000×g, 20 mi) to recover RNA. The obtained pellet was dissolved in a small amount of ultrapure water and separated by denaturing polyacrylamide gel electrophoresis (PAGE) [6% polyacrylamide (acrylamide/N,N'-methylenebisacrylamide, 19/1) containing 7.5 M urea as a denaturant and 25 (v/v)%formamide was prepared using a TBE buffer (89 mM Tris-boric acid, 2 mM EDTA)]. A gel part containing a target circular mRNA was detected by a UV shadowing method and cut out, and a gel piece was crushed, and then a 50 mM aqueous sodium acetate solution (pH 5.2) was added to extract RNA from the gel. RNA contained in the extract was recovered by an alcohol precipitation method and redissolved in ultrapure water. The RNA concentration was calculated based on the absorbance at 260 nm. The purity of the circular RNA after isolation and purification was confirmed by denaturing PAGE analysis (> 90%).

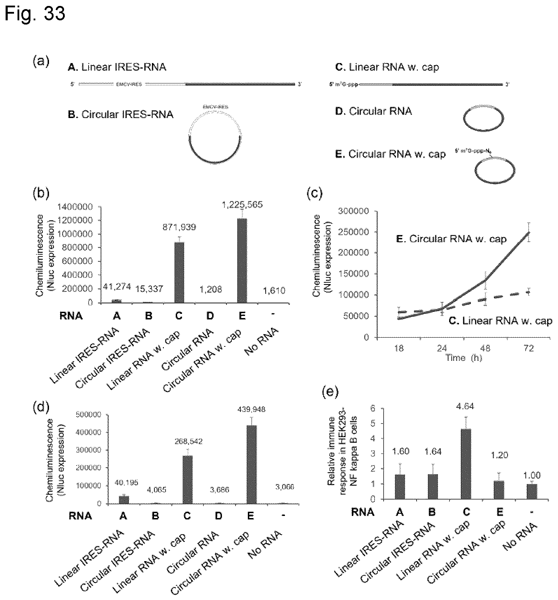

### 7. Synthesis of 5'-end capped linear mRNA composed of two fragments of chemically synthesized RNA fragment (54 bases in length) and transcriptionally synthesized RNA fragment (550 bases in length) (Fig. 33)

A 5' end phosphorylated chemically synthesized RNA oligomer having a 54-base-long main chain and not containing a branched chain was synthesized using an automated nucleic acid synthesizer according to a conventional method, similarly to the chemical synthesis containing a branched chain. A chemical capping reagent 7-methylguanosine 5'-diphosphate imidazolide sodium salt (Im-m⁷GDP) was reacted with the phosphate group at the 5' end thereof to obtain a capped RNA strand (WO2021172204A1). To 2 nmol (26 µL) of the 5' end phosphorylated RNA, 5 µL of a 200 mM calcium chloride aqueous solution was added, and the mixture was frozen with liquid nitrogen and then freeze-dried. After 3 hours, 45 µL of dimethyl sulfoxide (DMSO) was added to and dissolved in a mixture of dried RNA and calcium chloride, 50 µL of an Im-m⁷GDP/DMSO solution and 5 µL of a 200 mM 2-nitroimidazole/DMSO solution were added thereto, and the mixture was warmed at 55°C for 3 hours. 122 µL of a 0.541 M sodium acetate (pH 5.2) aqueous solution was added, a part thereof was fractionated, and the efficiency of the capping reaction was evaluated. Each 5 pmol of the raw material and the RNA after the reaction was analyzed by 10% denaturing PAGE containing 7.5 M urea as a denaturant, and the band shift of the capped product was confirmed. 220 µL of 2-propanol was added to the solution after adding the aqueous sodium acetate solution, and the mixture was cooled overnight at -30°C and centrifuged (20,000 g, 25 min) to obtain a pellet. This was redissolved in ultrapure water, and the target product was isolated and purified by reverse phase HPLC. [Hitachi LaChrom Elite or Chromaster system (Hitachi High-Tech Science Corporation); column, YMC Hydrosphere C18 (250 mm × 4.6 mm I.D., YMC CO., LTD.); Solvent A, 50 mM triethylammonium acetate (pH 7.0), 5% acetonitrile; Solvent B, acetonitrile; linear gradient 0 to 20% Solvent B (0 to 20 min); flow rate: 1 mL/min; detection wavelength, 260 nm; column temperature, 50°C].

The target product was recovered from the eluate by alcohol precipitation to obtain a capped RNA. The obtained capped RNA was ligated with T4 RNA ligase 2 in the presence of only Splint DNA_2 and the same transcribed RNA as that used in the synthesis of the above-described branched chain-containing circular mRNA. The composition of the enzyme reaction solution was as follows. 1 µM chemically synthesized 5'-capped RNA, 0.5 µM transcriptionally synthesized RNA, 2 µM Splint DNA_2, 50 mM Tris-HCl (pH 7.5), 2 mM MgCl₂, 1 mM DTT, 400 µM ATP, 10 (w/v)% PEG6000, 0.05 µg/µL T4 RNA ligase 2. The progress of the linking reaction was confirmed by denaturing polyacrylamide gel electrophoresis (PAGE) [6% polyacrylamide (acrylamide/N,N'-methylenebisacrylamide, 19/1) containing 7.5 M urea as a denaturant and 25 (v/v)%formamide was prepared using a TBE buffer (89 mM Tris-boric acid, 2 mM EDTA)], and separation and purification were performed. A gel part containing a linear mRNA as a target conjugate was detected by a UV shadowing method and cut out, and a gel piece was crushed, and then a 50 mM aqueous sodium acetate solution (pH 5.2) was added to extract RNA from the gel. RNA contained in the extract was recovered by an alcohol precipitation method and redissolved in ultrapure water. The RNA concentration was calculated based on the absorbance at 260 nm. The purity of RNA after isolation and purification was confirmed by denaturing PAGE analysis.

### 8. Synthesis of linear and circular mRNAs containing internal ribosome entry site (IRES) sequences (Fig. 33)

From a commercially available plasmid DNA pIRES Vector (Takara Bio 631605), a sequence including the IRES sequence was cut out at two restriction enzyme sites (EcoR I/BamH I), and this was inserted into pNL1.1 TK Vector (Promega N1501) to prepare a plasmid DNA. The sequence of the present plasmid DNA (4396-bp, pIRES_NL) used thereafter as a template for the PCR reaction is as shown in SEQ ID NO: 41.

The PCR reaction was performed by using the above-described pIRES_NL as a template and two synthetic oligo-DNAs (5'-CCCGGATCCTAATACGACTCACTATAGGCGCATATTAAGGTGACGCGT-3' (SEQ ID NO: 42)/5'-TTTTTTTTTTTTTTTTTTTTTTTTTTTTTTCTAGAATTACGCCAGAATGCG-3' (SEQ ID NO: 43)) as a primer. The composition of the reaction solution was as follows. 1 ng/µL pIRES_NL, 0.3 µM oligo DNA, 0.2 mM dNTPs, 1.5 mM MgSO₄, 1×PCR Buffer for KOD-Plus-Neo, 0.02 U/µL KOD-Plus-Neo (Toyobo Co., Ltd.). The reaction solution was subjected to the following thermal cycling conditions by using a T100 thermal cycler (Bio-Rad). 94°C, 2 minutes → 98°C, 10 seconds → 55°C, 30 seconds → 72°C, 90 seconds] × 25 cycles. The reaction solution was deproteinized, and then DNA was recovered by alcohol precipitation. Using the obtained DNA as a template, a transcription reaction using T7 RNA polymerase was performed in the same manner as in the case of the IRES sequence-free sequence to prepare a linear mRNA. The obtained linear mRNA was similarly purified using reverse phase HPLC. The circular mRNA containing the IRES sequence was subjected to a circularization reaction in the presence of only one splint DNA Splint DNA_1 in the same manner as the circular mRNA not containing IRES [0.5 µM transcriptionally synthesized IRES-containing RNA, 2 µM Splint DNA_1, 50 mM Tris-HCl (pH 7.5), 2 mM MgCl₂, 1 mM DTT, 400 µM ATP, 10 (w/v)% PEG6000, 0.05 µg/µL T4 RNA ligase 2], and isolated and purified by denaturing PAGE in the same manner. The purity of the circular RNA after isolation and purification was confirmed by denaturing PAGE analysis (> 90%).

### 9. Experiment for evaluating translational activity of mRNA using cultured HeLa cells (Figs. 31 to 33)

Cultured HeLa cells (RIKEN Cell Bank) were seeded at 1×10⁴ cells per well of a 96-well cell culture plate the day before. Here, 10 ng of RNA per well was introduced into HeLa cells based on the recommended conditions of the transfection reagent (Lipofectamine MessengerMAX, Thermo Fisher, 0.15 µL/well). After 3 hours from transfection, the supernatant was replaced with Dulbecco's Modified Eagle Medium (DMEM, Wako Pure Chemical Industries, Ltd.) medium containing 10 (w/w)% fetal bovine serum (FBS, Hyclone), and the cells were further cultured under the conditions of 37°C and 5% CO₂ for 24 hours, and then lysed. The amount of NanoLuc luciferase protein contained in the lysate was measured using Nano-Glo (registered trademark) Luciferase Assay System (Promega) and Mithras LB 940 plate reader (Berthold).

### 10. Experiment for evaluating translational activity of mRNA using experimental animal (mouse) (Fig. 33)

pH-responsive lipid (ALC-0315, MedChemExpress, Monmouth Junction, NJ, USA), phospholipid (1,2-distearoyl-sn-glycero-3-phosphocholine, DSPC, Fujifilm Wako, Osaka, Japan), cholesterol (Sigma Aldrich), and ALC-0159 (MedChemExpress) were prepared in an ethanol solvent at a molar ratio of 46.3 : 9.4 : 42.7 : 1.6. mRNA was dissolved in 50 mM of citrate buffer (pH 3). mRNA and lipid were mixed at a volume ratio of 2 : 1 in a microchannel (NanoAssemblr (registered trademark) Spark, Precision Nanosystems, Vancouver, Canada). Thereafter, the mixture was diluted 40 times with PBS, and ultrafiltered with Amicon Ultra-15-30K centrifugal units (Merck Millipore). LNP was administered from the tail vein so that mRNA was 0.33 ug/mouse. After 4 hours, each organ was collected. After homogenization in a passive lysis buffer (Promega), the amount of luciferase was quantified using Nano-Glo (registered trademark) Luciferase Assay System (Promega) and Lumat3 LB9508 luminometer (Berthold technologies, Bad Wildbad, Germany).

### 11. Experiment for evaluating immunogenicity of mRNA using cultured HEK293-NF kappa B reporter cells (Fig. 33)

NF-κB reporter (Luc)-HEK293 cells were purchased from BPS Biosciences. On the day before transfection, trypsinized cells were seeded at 3×10⁵ cells per well of a 48-well cell culture plate. Immediately before transfection, a growth medium (DMEM medium containing 10% FBS and 100 µg/mL hygromycin B) was removed and replaced with 240 µL Opti-MEM (registered trademark) I Reduced Serum Medium (Thermo Fisher) per well. 20 ng of mRNA per well was mixed with 0.15 µL Lipofectamine MessengerMAX (Thermo Fisher) and 10 µL Opti-MEM I medium and added to the wells and cells were cultured for 3 hours. The supernatant was replaced with a growth medium (250 µL), and the cells were cultured for 24 hours in total. Cells were lysed using 50 µL of Glo Lysis Buffer (Promega) per well. The total protein concentration in the cell lysate was measured using Pierce BCA Protein Assay Kit (Thermo Fisher), and the expression level of luciferase protein was measured using One-Glo Luciferase Assay System (Promega). The expression level of luciferase was standardized by the total protein concentration, and the relative value of the activation amount of the NF-κB information transduction pathway by each mRNA introduction was calculated.

Fig. 31 shows the experimental results showing that the translational activity of a circular mRNA in which a cap structure is introduced into a branched chain is greatly improved as compared with that in which a cap structure is not introduced. (a) shows a conceptual diagram showing a method for synthesizing a circular mRNA containing a 5'-capped structure in the branched chain. A chemically synthesized RNA fragment (SEQ ID NOS: 44 to 48) having a length of 54 bases and a transcriptionally synthesized RNA (SEQ ID NO: 49) were linked by enzyme ligase to prepare a circular mRNA. The sequences and structures of chemically synthesized RNA fragments and enzyme-synthesized RNA fragments are shown in the drawing. (b) shows the evaluation results of the translational activity of a circular mRNA synthesized by the method shown in (a) in cultured HeLa cells. The circular mRNA was introduced into cultured HeLa cells by a lipofection method and cultured for 24 hours, then the cells were lysed, and the NanoLuc luciferase activity of the lysate was measured. The activities of circular mRNAs having branched chains of three different chain lengths were compared. It is found that the translational activity of the circular mRNA having a cap structure in the branched chain was greatly improved as compared with that having no cap structure.

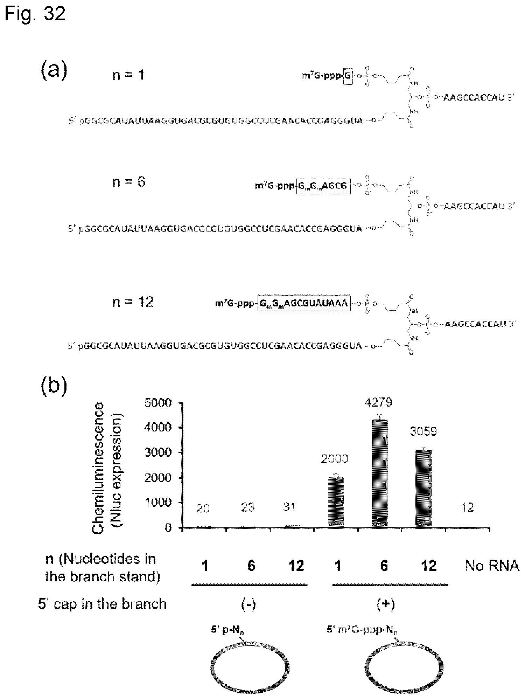

Fig. 32 shows the experimental results showing the relationship between the branched chain length of a circular mRNA in which a cap structure is introduced into a branched chain and translational activity (that the branched chain length is variable). (a) is a view showing the structure of chemically synthesized RNA fragments (SEQ ID NOS: 48, 50, and 51) having a 5'-capped structure in the branched chain. Three types of RNA strands having different branched chain lengths and sequences were prepared. (b) shows the evaluation results of the translational activity of a circular mRNA synthesized using the fragment shown in (a) as a substrate in cultured HeLa cells. This is the result of comparing a circular mRNA prepared using an RNA strand before introducing a cap structure at the 5' end of the branched chain and the activity thereof, and showing the importance of introducing a cap structure at the end of the branched chain. The circular mRNA was introduced into cultured HeLa cells by a lipofection method and cultured for 24 hours, then the cells were lysed, and the NanoLuc luciferase activity of the lysate was measured. When the activities of circular mRNAs having branched chains of three different chain lengths were compared, the circular mRNA having a 6-base-long branched chain showed the highest translational activity although the activities were not significantly different.

Fig. 33 shows the experimental results showing that the translational activity of a circular mRNA having a cap structure in a branched chain is higher than that of mRNA or the like containing an EMCV-derived IRES sequence. All RNAs contain a common Nano luciferase coding sequence, 3'UTR, and poly A30 tail. (a) is a schematic view showing the sequence and structure of the used mRNA. (b, c) show the evaluation results of the translational activity in cultured HeLa cells. The mRNA was introduced into cultured HeLa cells by a lipofection method and cultured for 24 hours (b) or 18, 24, 48, and 72 hours (c), then the cells were lysed, and the NanoLuc luciferase activity of the lysate was measured. (d) shows the results of measuring the expression level in the mouse spleen. (e) shows the experimental results of evaluating immunogenicity of mRNA in a HEK293-NF kappa B-luciferase reporter cell line. mRNA was introduced into cells by a lipofection method.

From (b-e), the translational activity of the circular mRNA (E) having a cap structure in the branched chain was higher than that of linear mRNA (A) and circular mRNA (B) containing EMCV-IRES, linear mRNA (C) having a cap structure, and circular mRNA (D) having no cap structure in both the human-derived cultured cell line (b) and the mouse animal experiment (d) (b). In the cultured cell line, the circular RNA (E) having a cap structure showed continuous translational activity for a longer time as compared with the linear mRNA (C) having a cap structure. This is an experimental result showing that the immunogenicity of the circular mRNA (E) having a cap structure in the branched chain is lower than that of the linear mRNA (C) having a cap structure.

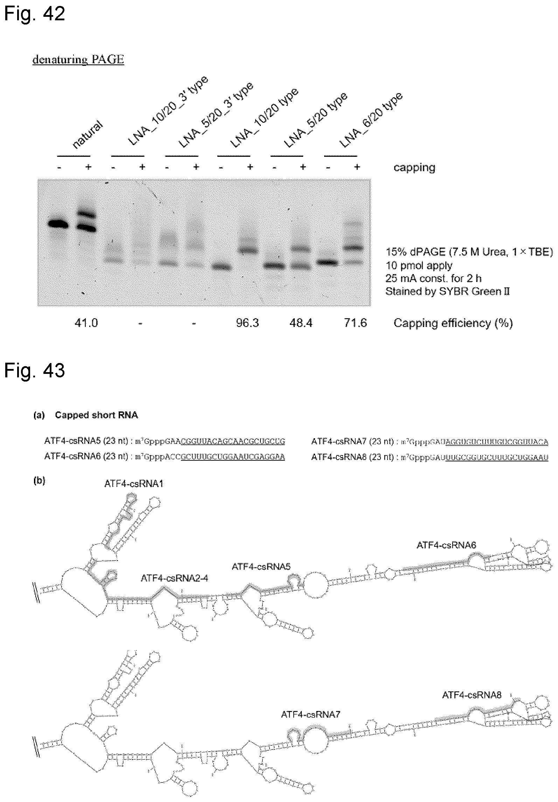

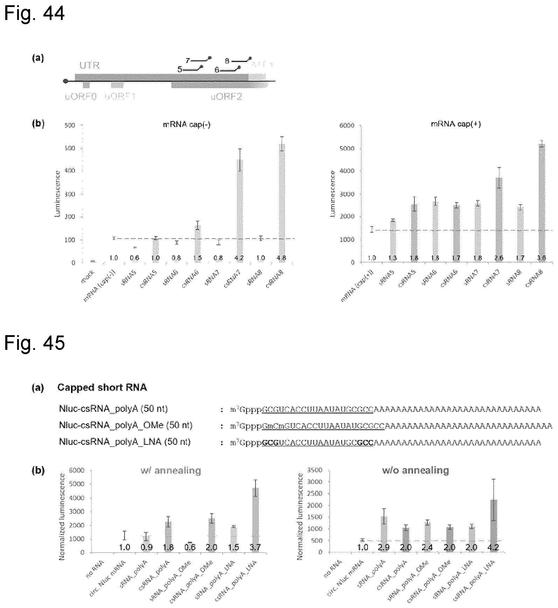

### 12. Translation reaction in HeLa cells of ATF4-Renilla mRNA and ATF4-csRNA5 to csRNA8 (Figs. 43 and 44)

### (1) Synthesis of transcription template DNA of ATF4-Renilla mRNA by PCR

A PCR reaction solution (0.3 µM Forward primer, 0.3 µM Reverse primer, 1 ng/µL plasmid DNA (psiCHECK2_ATF4-Renilla), 0.2 mM dNTPs, 1.5 mM MgSO₄, 1×PCR Buffer for KOD-Plus-Neo (TOYOBO), 0.02 U/µL KOD-Plus-Neo (TOYOBO), scaled-up with MQ water) was prepared, and a template DNA was synthesized by a PCR method using a thermal cycler. As PCR conditions, the initial denaturation cycle of 94°C for 2 minutes, 98°C for 10 seconds, 56°C for 30 seconds, and 72°C for 60 seconds was set to 30 cycles. A 1% agarose gel (Agarose S (NIPPON GENE CO., LTD.), 1×TAE buffer) was prepared, 6×Loading Buffer (takara) was added to the PCR reaction solution and applied, and electrophoresis was performed at 100 V for 30 minutes. After staining by EtBr, a target band was cut out, and transcription-type DNA was extracted and purified using Wizard (registered trademark) SV Gel and PCR Clean-Up System (Promega). The absorbance of the obtained DNA solution at 260 nm was measured to determine the concentration.

### (2) Synthesis of ATF4-Renilla mRNA by in vitro transcription

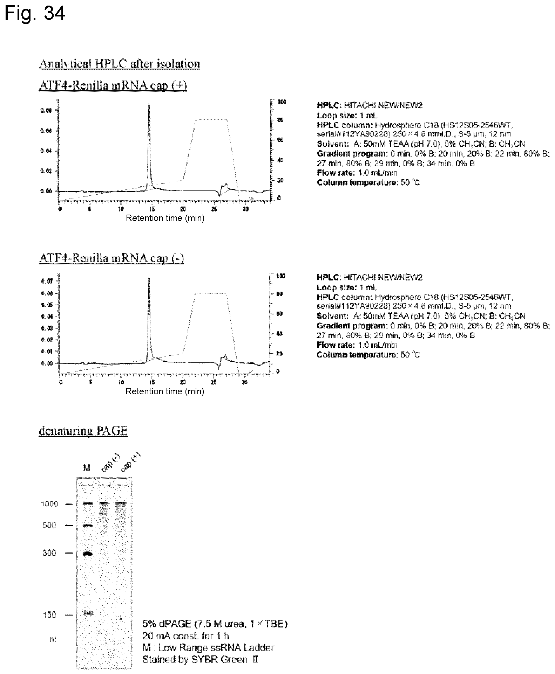

In order to introduce a cap structure at the 5' end, an in vitro transcription reaction was performed with the following composition in which the ratio of ARCA and GTP was set to 4 : 1. A transcription reaction solution (10 ng/µL template DNA, 1×T7 polymerase buffer (takara) (40 mM Tris-HCl (pH 8.0), 8 mM MgCl₂, 2 mM spermidine), 5 mM DTT, 2 mM ATP, 2 mM CTP, 2 mM UTP, 0.4 mM GTP, 1.6 mM ARCA, 2.5 U/µL T7 RNA polymerase, scaled-up with MQ water) was prepared. In the case of introducing no cap structure, a transcription reaction solution was prepared with the following composition (10 ng/µL template DNA, 1×T7 polymerase buffer (takara) (40 mM Tris-HCl (pH 8.0), 8 mM MgCl₂, 2 mM spermidine), 5 mM DTT, 2 mM ATP, 2 mM CTP, 2 mM UTP, 2 mM GTP, 2.5 U/µL T7 RNA polymerase, scaled-up with MQ water). After incubation at 37°C for 1 hour, Recombinant DNase I (takara) was added to the reaction solution at 0.1 U/µL, and the mixture was incubated at 37°C for 15 minutes to decompose the template DNA. After the reaction, the protein was removed by phenol/chloroform extraction, and the mixture was concentrated using Amicon Ultra (0.5 mL, 10K) (Merck Millipore). Thereafter, purification was performed by alcohol precipitation and reverse phase HPLC. Acetonitrile was removed with a centrifugal evaporator, and then alcohol precipitation was performed. The nucleic acid aqueous solution was subjected to absorbance measurement at 260 nm to determine the concentration. The results are shown in Fig. 34.

### (3) Chemical synthesis of RNA

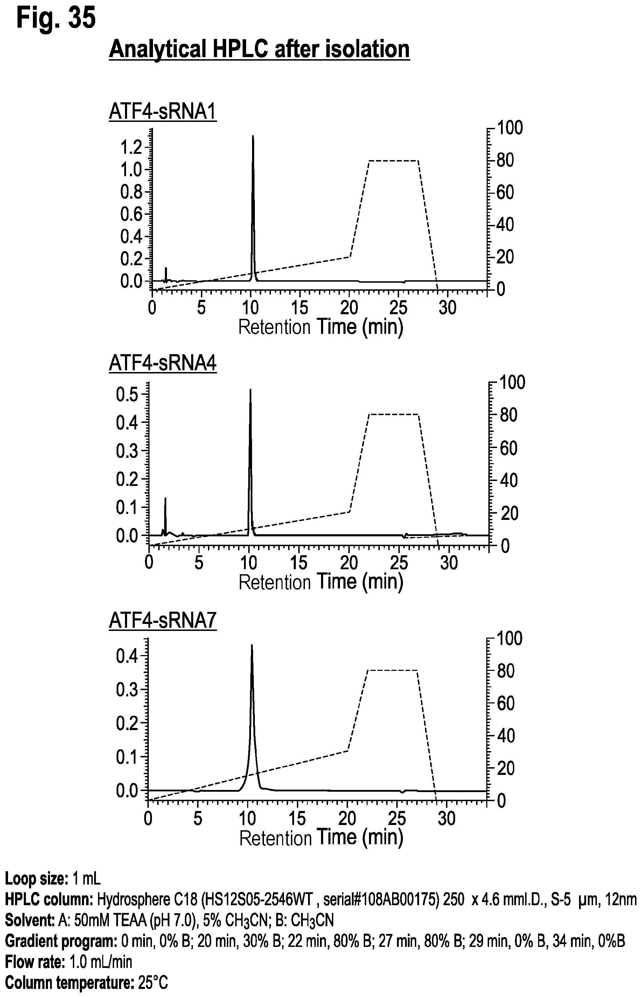

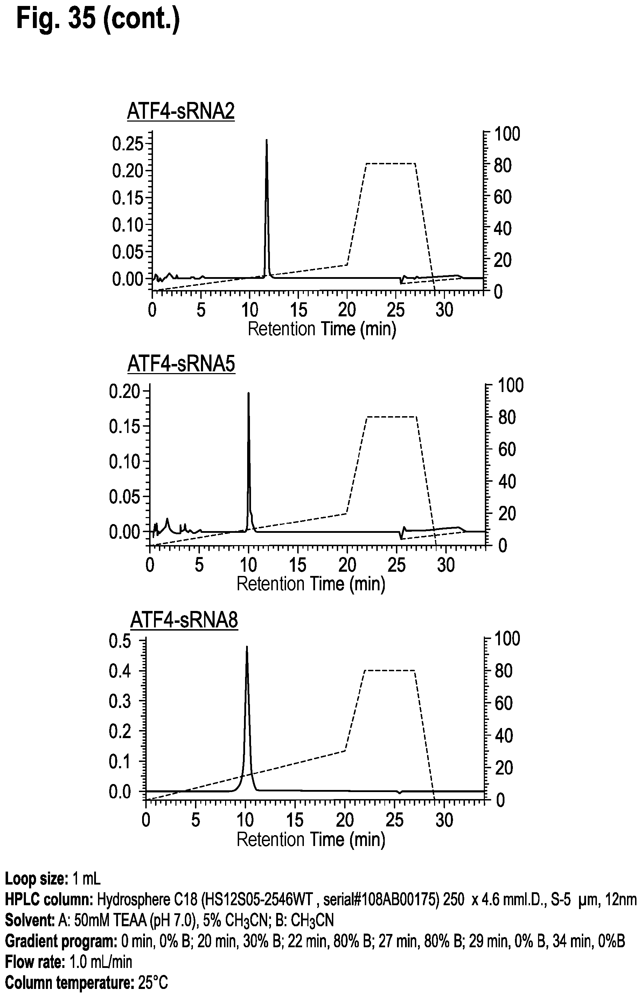

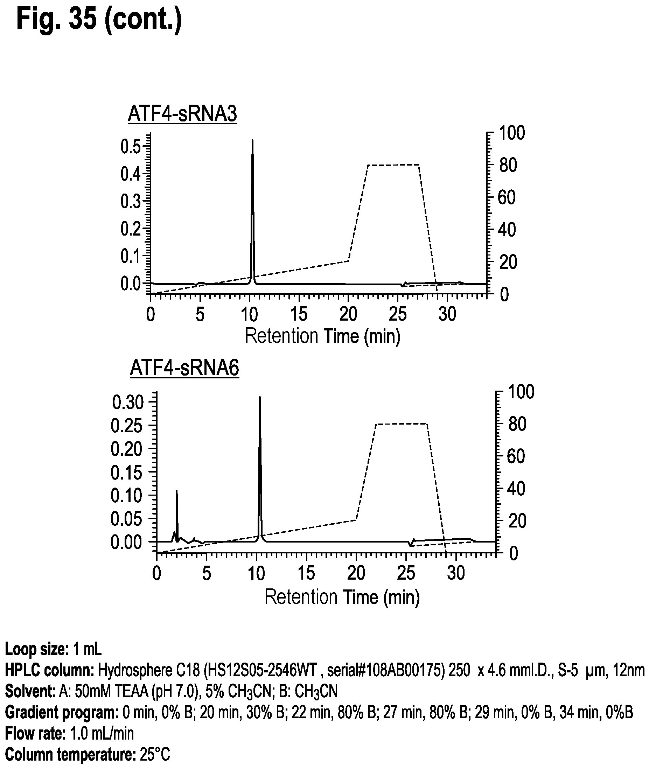

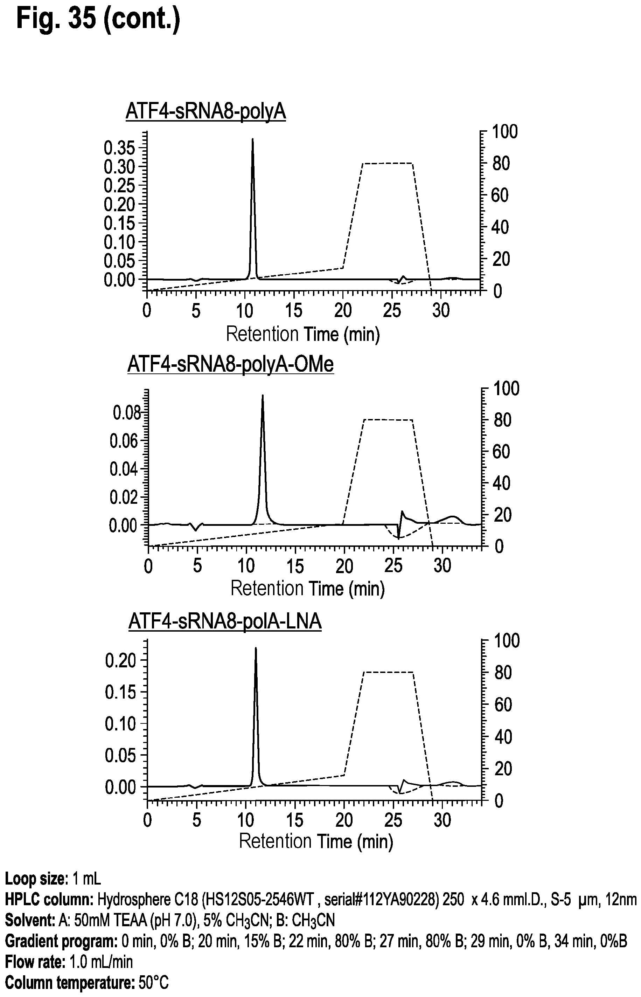

Synthesis was performed with an automated nucleic acid synthesizer using any one of Universal UnyLinker Support 2000Å CPG (ChemGenes), 3'-TOM ribo Adenosine (n-acetyl) 2'-lcaa CPG 1000Å (ChemGenes), 2'-TOM ribo Cytidine (n-acetyl) 3'-lcaa CPG 1000Å (ChemGenes), 2'-TOM ribo Guanosine (n-acetyl) CPG 1000Å (ChemGenes), and 2'-TOM ribo Uridine 2'-lcaa CPG 1000Å (ChemGenes). The 5' end was phosphorylated using 5'-Phosphate-ON Reagent (ChemGenes). After the synthesis, the CPG was transferred to a screw tube with 1.5 mL loop (SARSTEDT AG & Co. KG), 1 mL of 40% aq. methylamine/28% aq. ammonia (1 : 1) was added, and the mixture was incubated at 65°C for 30 minutes. After cooling on ice, the mixture was filtered using a Millex LH 0.45 µm filter, washed with 0.75 mL of MQ water, and dried and solidified on a centrifugal evaporator. 1 mL of 1 M TBAF in THF was added, and the mixture was allowed to stand still at room temperature overnight. 1 mL of a 1 M Tris-HCl buffer (pH 7.5) was added, and the organic solvent was distilled off with a centrifugal evaporator. Desalination was performed using a NAP-25 column (GE Healthcare), and then purification was performed by alcohol precipitation and reverse phase HPLC. After acetonitrile was removed with a centrifugal evaporator, alcohol precipitation was performed, and the concentration of the nucleic acid aqueous solution was determined from absorbance measurement at 260 nm. The results are shown in Fig. 35.

**[Table 4]**

| LC-MS | | | | |
|---|---|---|---|---|
| ATF4-sRNA1 | ATF4-sRNA2 | ATF4-sRNA3 | | ATF4-sRNA4 |
| Calcd. 7465.4855 | Calcd. 7498.4675 | Calcd. 7448.5025 | | Calcd. 7489.5115 |
| Obs. 7466.46 | Obs. 7499.49 | Obs. 7449.53 | | Obs. 7490.41 |
| | | | | |

| ATF4-sRNA5 | ATF4-sRNA6 | ATF4-sRNA7 | | ATF4-sRNA8 |
|---|---|---|---|---|
| Calcd. 7465.4855 | Calcd. 7466.4695 | Calcd. 7422.3535 | | Calcd. 7438.3525 |
| Obs. 7466.49 | Obs. 7467.48 | Obs. 7421.77 | | Obs. 7437.76 |
| | | | | |

| ATF4-sRNA8-polyA | ATF4-sRNA8-polyA_O Me | | ATF4-sRNA8-p olyA-LNA | |
|---|---|---|---|---|
| Calcd. 18012.6081 | Calcd. 18157.3559 | | Calcd. 18207.33 09 | |
| Obs. 18016.39 | Obs. 18156.25 | | Obs. 18206.1 2 | |

### (4) Chemical capping of RNA

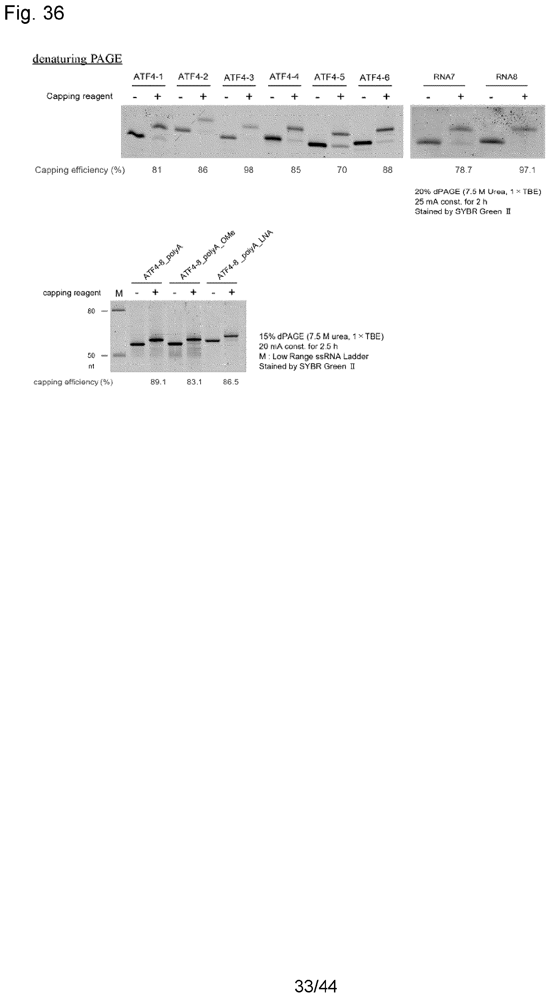

An aqueous RNA solution and an aqueous CaCl₂ solution were placed in a 1.5 mL tube, and then freeze-dried and solidified. A capping reagent dissolved in DMSO and 2-nitroimidazole were added thereto, and the mixture was scaled-up with DMSO. The composition of the final capping reaction solution is as follows (20 µM RNA, 10 mM CaCl₂, 10 mM capping reagent, 10 mM 2-nitroimidazole, scaled-up with DMSO). The reaction solution was incubated at 55°C for 3 hours, and then purified by Amicon Ultra (Merck Millipore, 0.5 mL, 3K) and alcohol precipitation, and the concentration of the nucleic acid aqueous solution was determined from absorbance measurement at 260 nm. Thereafter, analysis by denaturing PAGE was performed. 3 pmol of RNA was mixed with 2×formamide loading solution and loaded onto the gel after heating at 80°C for 5 minutes. The gel was electrophoresed at 25 mA for 2 hours or 20 mA for 2.5 hours, stained by SYBR Green II, and then photographed by ChemiDoc. The capping efficiency was calculated from the band intensity. The results are shown in Fig. 36.

**[Table 5]**

| LC-MS | | | | |
|---|---|---|---|---|
| ATF4-csRNA1 | ATF4-csRNA2 | ATF4-csRNA3 | | ATF4-csRNA4 |
| Calcd. 7904.6991 | Calcd. 7937.6811 | Calcd. 7887.7161 | | Calcd. 7928.7251 |
| Obs. 7905.57 | Obs. 7938.40 | Obs. 7888.59 | | Obs. 7929.46 |
| | | | | |

| ATF4-csRNA5 | ATF4-csRNA6 | ATF4-csRNA7 | | ATF4-csRNA8 |
|---|---|---|---|---|
| Calcd. 7904.6991 | Calcd. 7905.6831 | Calcd. 7861.5671 | | Calcd. 7877.5661 |
| Obs. 7905.50 | Obs. 7906.58 | Obs. 7861.10 | | Obs. 7876.90 |
| | | | | |

| ATF4-csRNA8-polyA | ATF4-csRNA8-polyA _OMe | | ATF4-csRNA8-polyA-LNA | |
|---|---|---|---|---|
| Calcd. 18456.2994 | Calcd. 18596.5694 | | Calcd. 18646.544 4 | |
| Obs. 18456.01 | Obs. 18596.14 | | Obs. 18645.35 | |

### (2) Annealing and luminescence measurement

An annealing solution (0.1 µM ATF4_Renilla mRNA, 1 µM csRNA (SEQ ID NOS: 52 to 55), 10 mM Tris-HCl (pH 7.5), 50 mM NaCl, 1 mM EDTA (pH 8.0)) was prepared and heated at 80°C for 5 minutes. Thereafter, annealing of mRNA and csRNA was performed by gradually cooling until the temperature reached room temperature. HeLa cells suspended in 100 µL of 10% FBS-containing D-MEM (Wako) at 1.5×10⁴ cells/well were seeded in a 96-well plate the day before transfection. The next day, the medium was removed and transfected with annealed mRNA by using Lipofectamine (registered trademark) MessengerMAX (registered trademark) (Thermo Fisher SCIENTIFIC) (Lipofectamine (registered trademark) MessengerMAX (registered trademark) 0.3 µL, annealing solution 1 µL, scaled-up to 100 µL with Opti-MEM (registered trademark) (Thermo Fisher SCIENTIFIC)). After incubation in an environment of 37°C and 5% CO₂ for 3 hours, the medium was removed and 100 µL of 10% FBS-containing D-MEM (Wako) was added. After incubation again in an environment of 37°C and 5% CO₂ for 3.5 hours, the medium was removed, and 20 µL of 1×Renilla Luciferase Assay Lysis Buffer (Promega) was added thereto for lysation. 10 µL of lysate was mixed with 30 µL of Renilla Luciferase Assay Reagent (Promega) and luminescence measurement was performed.

(a) of Fig. 43 shows the sequence of newly designed csRNA. The complementary strand region with mRNA is indicated by underline. The sequences were all described in the 5' → 3' direction. (b) shows the secondary structure of ATF4-Renilla mRNA 5'UTR. The binding position of csRNA is indicated by a marker in the drawing.

Fig. 44 is a view showing optimization of the binding position of csRNA. (a) is a schematic view of mRNA and csRNA used for translation. Red circles in the drawing indicate the cap structure. (b) is a view showing luciferase luminescence measurement in HeLa cells. A relative value when the luminescence intensity of mRNA (cap(-)) or mRNA (cap(+)) was taken as 1 was shown. Error bars were standard errors of three experimental averages.

From these results, the translation promoting effect was improved by designing csRNA so that mRNA binds to a region that does not seem to form a double strand in the molecule (both mRNA cap(-) and mRNA cap(+) showed a large increase in the amount of translation).

### 13. Translation reaction in HeLa cell extract of csRNA into which chemical modification (OMe, LNA) was introduced (Fig. 45)

### (1) Synthesis of circular NanoLuc mRNA by in vitro transcription

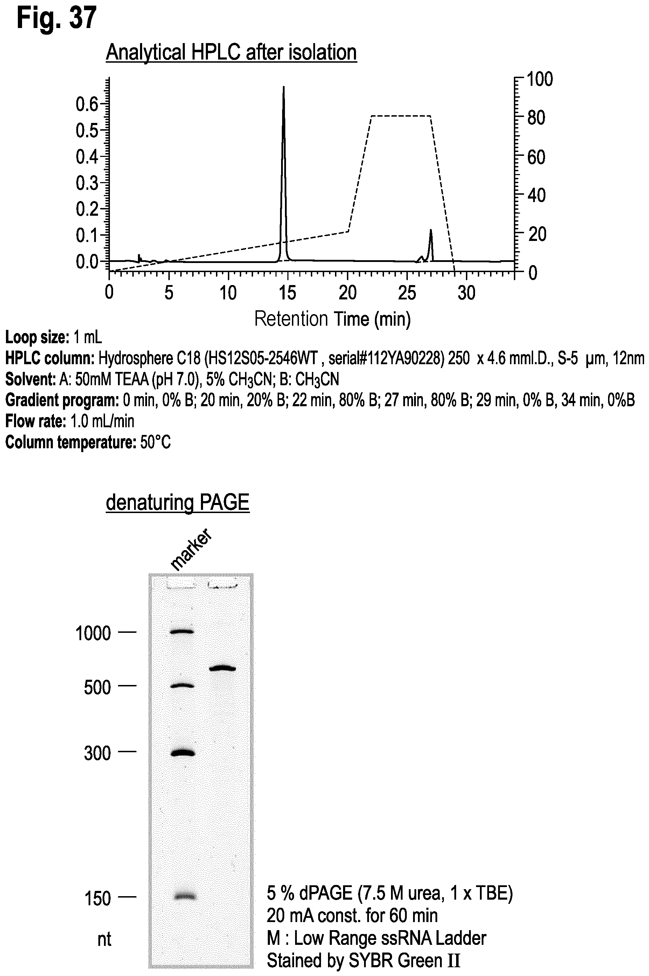

In order to introduce a monophosphate group at the 5' end, an in vitro transcription reaction was performed with the following composition in which the ratio of GMP and GTP was set to 4 : 1. A transcription reaction solution (10 ng/µL template DNA, 1×T7 polymerase buffer (takara) (40 mM Tris-HCl (pH 8.0), 8 mM MgCl₂, 2 mM spermidine), 5 mM DTT, 2 mM ATP, 2 mM CTP, 2 mM UTP, 2 mM GTP, 10 mM GMP, 2.5 U/µL T7 RNA polymerase, scaled-up with MQ water) was prepared and incubated at 37°C for 2 hours. Recombinant DNase I (takara) was added to the reaction solution at 0.1 U/µL, and the mixture was incubated at 37°C for 15 minutes to decompose the template DNA. After the reaction, the protein was removed by phenol/chloroform extraction, and the mixture was concentrated using Amicon Ultra (Merck Millipore, 0.5 mL, 10K). Thereafter, purification was performed by alcohol precipitation and reverse phase HPLC. Acetonitrile was removed with a centrifugal evaporator, and then alcohol precipitation was performed. The nucleic acid aqueous solution was subjected to absorbance measurement at 260 nm to determine the concentration. The results are shown in Fig. 37.

### (2) Cyclization reaction of circular NanoLuc mRNA

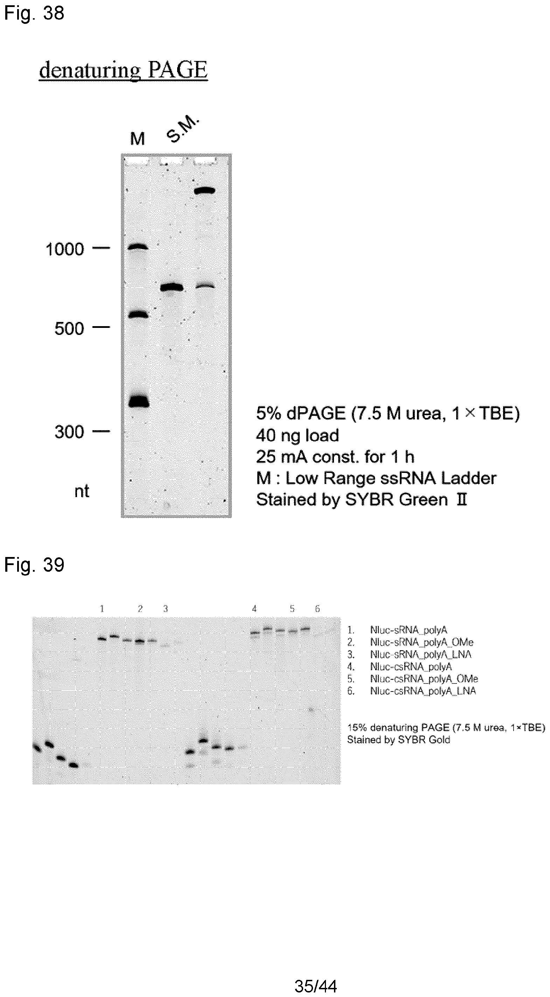

1 mL of a reaction solution (0.5 µM mRNA, 1.0 µM splint DNA, T4 RNA ligase II, 1×T4 RNA ligase II buffer, 10% PEG8000, scaled-up with MQ water) was prepared and incubated at 37°C for 1 hour. Recombinant DNase I (takara) was added to the reaction solution at 0.1 U/µL, and the mixture was incubated at 37°C for 15 minutes to decompose the splint DNA. After the reaction, the protein was removed by phenol/chloroform extraction, and the mixture was concentrated using Amicon Ultra (0.5 mL, 10K) (Merck Millipore). Subsequently, alcohol precipitation was performed, and progress of the cyclization reaction was confirmed by 5% denaturing PAGE. Thereafter, the circular mRNA was isolated and purified by denaturing PAGE. A gel containing a target band was cut out by UV shadowing, and the cut gel was crushed well. The gel piece was immersed in 2 mL of MQ water and permeated overnight at 4°C to extract RNA. Centrifugation was performed at 4°C and 10000 rpm for 5 minutes, and the supernatant was recovered. 500 µL of MQ water was added to the gel piece again, and the mixture was centrifuged in the same manner to recover the supernatant. All the recovered supernatants were filtered (Millex-LCR, 0.45 µm (Merck Millipore)) to remove the mixed gel piece. The filtrate was desalted and concentrated by ultrafiltration (Amicon Ultra (0.5 mL, 10K) (Merck Millipore)), and then alcohol precipitation was performed. The nucleic acid aqueous solution was subjected to absorbance measurement at 260 nm to determine the concentration. The results are shown in Fig. 38.

### (3) Synthesis of csRNA (Nluc-csRNA_polyA, Nluc-csRNA_polyA_OMe, Nluc-csRNA_polyA_LNA) with respect to circular Nluc mRNA

### (a) RNA synthesis

RNA synthesis was performed on a 0.2 µmol scale, and an NTS M-2-MX DNA/RNA synthesizer or an NRs-4A10R7 DNA/RNA synthesizer manufactured by NIHON TECHNO SERVICE CO., LTD. was used. As the phosphoramidite used for RNA solid phase synthesis, rA(5'-DMT-2'-TOM-ribo-Adenosine (n-acetyl) OP), rC(5'-DMT-2'-TOM-ribo-Cytidine (n-acetyl) OP), rG(5'-DMT-2'-TOM-ribo-Guanosine (n-acetyl) OP), and U(5'-DMT-2'-TOM-ribo-Uridine OP) (ChemGenes) were used. As the phosphorylation reagent, 5' phosphate-ON Reagent (ChemGenes) was used. As the 2'-OMe RNA amidite, 2'-OMe-C-CE-phosphoramidite and 2'-OMe-G(iBu) -CE-phosphoramidite (ChemGenes) were used. As the LNA amidite, MBN-Ganosine(N,N-DMF)-phosphoramidite (ChemGenes) and MBN-5'-Methyl-Cytidine(N-bz)-phosphoramidite (ChemGenes) were used. As the CPG, 3'-TOM-ribo Cytidine (n-acetyl) 2'-lcaa CPG 1000Å, 3'-TOM-ribo Adenosine (n-acetyl) 2'-lcaa CPG 1000Å (ChemGenes), and 3'-phosphate ON lcaa CPG 1000A were used. After the synthesis, the CPG was transferred to a screw tube with 1.5 mL loop (SARSTEDT AG & Co. KG), 1.0 mL of 40% aq. methylamine/28% aq. ammonia (1 : 1) was added, and the mixture was incubated at 65°C for 20 minutes. The mixture was filtered using a Millex LH 0.45 µm filter, washed with sterile water (500 µL), and dried and solidified on a centrifugal evaporator. 1.0 mL of 1 M TBAF in THF was added and dissolved, and the mixture was allowed to stand still at room temperature overnight. 1.0 mL of a 1 M Tris-HCl buffer (pH 7.5) was added, and the organic solvent was distilled off with a centrifugal evaporator. Desalination was performed using a NAP-25 column (GE Healthcare). Purification was performed by reverse phase HPLC. HPLC (HITACHI) conditions; Column: hydrosphere C18 column (4.6 × 250 mm; YMC), Mobile phase: A) 50 mM TEAA buffer (pH 7.0), 5% acetonitrile, B) acetonitrile, Gradient: 0 to 20 min B conc. 0% to 20%, Flow rate: 1.0 mL/min Analysis was performed by 15% denatured polyacrylamide gel electrophoresis (PAGE) (acrylamide : bisacrylamide = 19 : 1 (wt/wt), 7.5 M urea). For PAGE, a TBE buffer (89 mM Tris-borate, 2 mM EDTA, pH 8.3) was used. Analysis by LC/MS was performed. LC/MS (agilent) conditions; ACQUITY UPLC BEH C18 Column, 0.3 mL/min, A; 8.6 mM TEA/100 mM HFIP solution, B; MeOH, 0 to 30%/20 min. The results are shown in Fig. 39.

### LC-MS

Nluc-sRNA_polyA; calcd. 16259.1, obs. 16261.4
Nluc-sRNA_polyA_OMe; calcd. 16287.1, obs. 16289.5
Nluc-sRNA_polyA_LNA; calcd. 16373.2, obs. 16375.2

### (4) Capping reaction of phosphorylated RNA

The mixture was mixed in a 1.5 mL tube so as to have a final concentration of 20 µM RNA and 10 mM CaCl₂, respectively, and the mixture was freeze-dried and solidified and then dissolved in DMSO. Im (m7GDP) ·Na (Hongene Biotech) dissolved in DMSO was added thereto so as to have a final concentration of 10 mM and 2-nitroimidazole was added thereto so as to have a final concentration of 10 mM. This reaction solution was incubated at 55°C for 3 hours. The reaction solution was diluted two-fold with sterile water, and then isopropanol precipitation was performed. HPLC purification was performed. HPLC conditions; Column: hydrosphere C18 column (4.6 × 250 mm; YMC), Mobile phase: A) 50 mM TEAA buffer (pH 7.0), 5% acetonitrile, B) acetonitrile, Gradient: 0 to 20 min B conc. 0% to 20%, Flow rate: 1.0 mL/min

Analysis by LC/MS was performed. LC/MS (agilent) conditions; ACQUITY UPLC BEH C18 Column, 0.3 mL/min, A; 8.6 mM TEA/100 mM HFIP solution, B; MeOH, 0 to 30%/20 min

The HPLC-purified capped RNA was precipitated using isopropanol and sodium acetate (pH 5.2).

### LC-MS

Nluc-csRNA_polyA; calcd. 16699.3, obs. 16701.0
Nluc-csRNA_polyA_OMe; calcd. 16727.4, obs. 16728.7
Nluc-csRNA_polyA_LNA; calcd. 16813.5, obs. 16814.9

### (5) Chemical synthesis of RNA

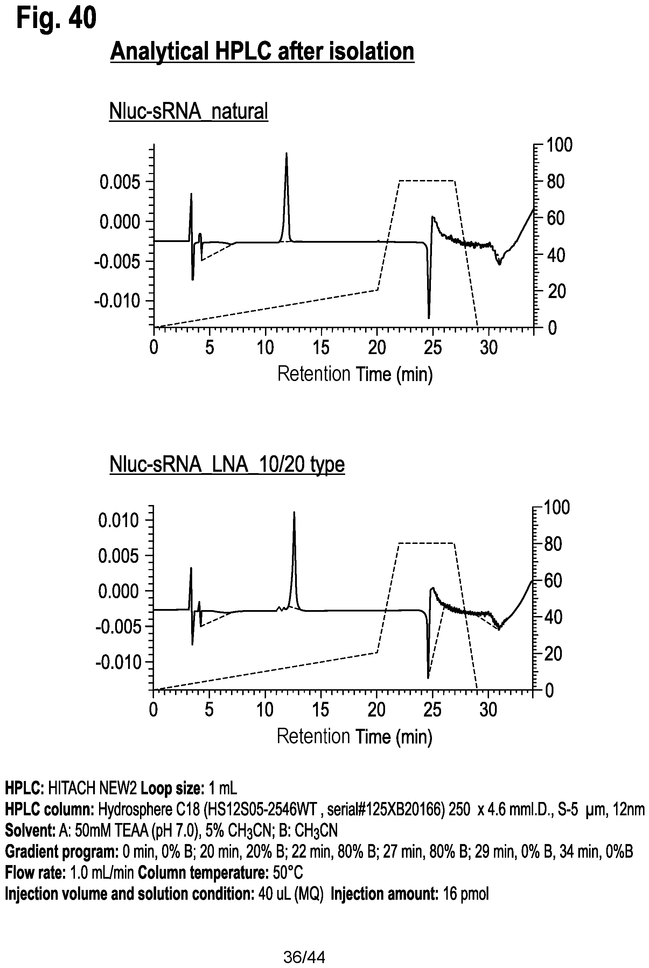

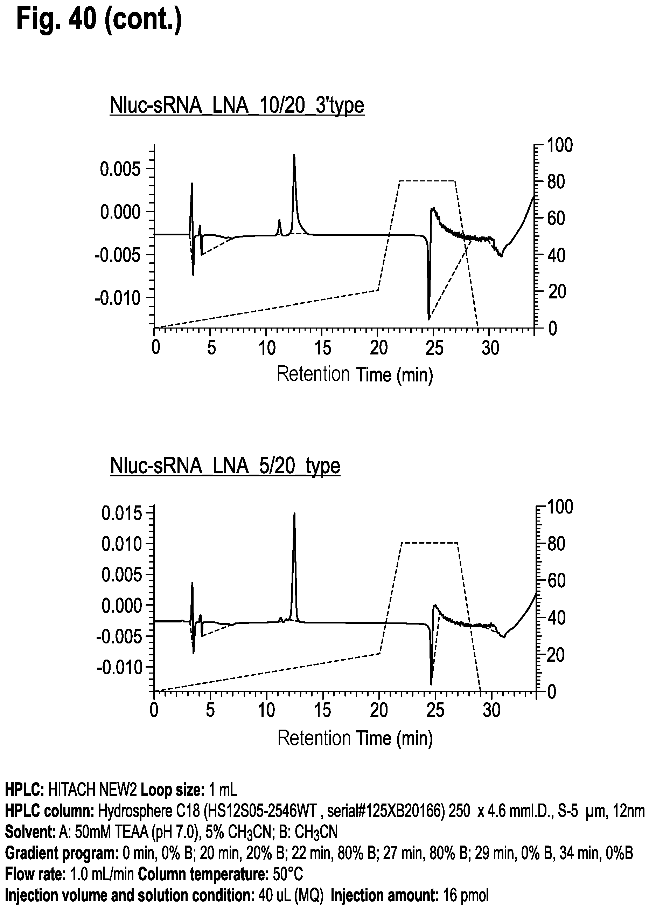

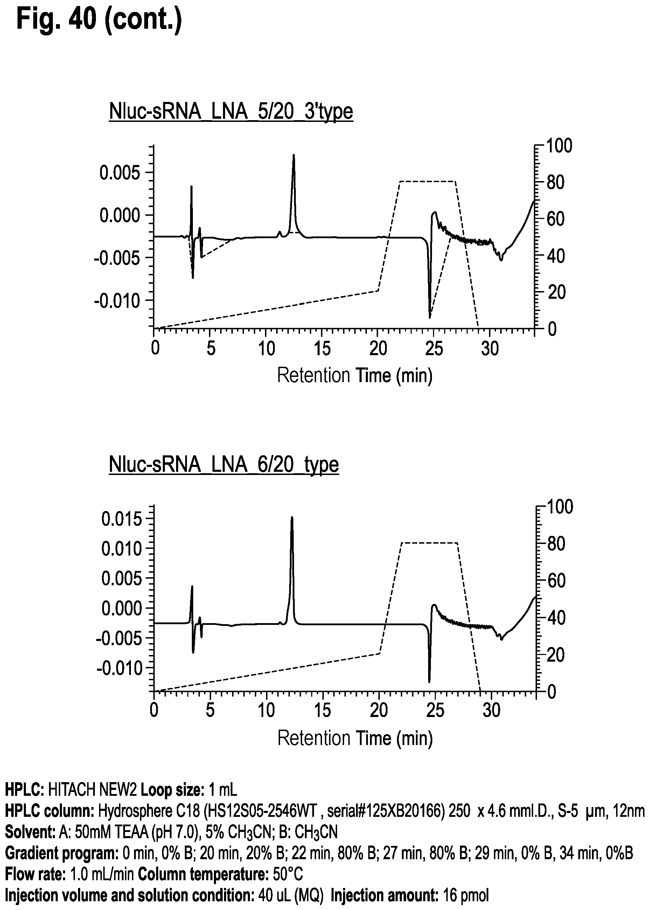

RNA was synthesized with an automated nucleic acid synthesizer using Universal UnyLinker Support 2000Å CPG (ChemGenes). The 5' end was phosphorylated using 5'-Phosphate-ON Reagent (ChemGenes). After the synthesis, the CPG was transferred to a screw tube with 1.5 mL loop (SARSTEDT AG & Co. KG), 1 mL of 40% aq. methylamine/28% aq. ammonia (1 : 1) was added, and the mixture was incubated at 65°C for 30 minutes. After cooling on ice, the mixture was filtered using a Millex LH 0.45 µm filter, washed with 1 mL of MQ water, and dried and solidified on a centrifugal evaporator. 1 mL of 1 M TBAF in THF was added, and the mixture was allowed to stand still at room temperature overnight. 1 mL of a 1 M Tris-HCl buffer (pH 7.5) was added, and the organic solvent was distilled off with a centrifugal evaporator. Desalination was performed using a NAP-25 column (GE Healthcare), and then purification was performed by alcohol precipitation and reverse phase HPLC. After acetonitrile was removed with a centrifugal evaporator, alcohol precipitation was performed, and the concentration of the nucleic acid aqueous solution was determined from absorbance measurement at 260 nm. The results are shown in Fig. 40.

**[Table 6]**

| LC-MS | | |
|---|---|---|
| Nluc-sRNA_natural | Nluc-sRNA_LNA_10/20_3' type | Nluc-sRNA_LNA_5/20_3' type |
| Calcd. 8052.8091 | Calcd. 8243.0541 | Calcd. 8154.9451 |
| Obs. 8051.69 | Obs. 8241.99 | Obs. 8153.64 |
| | | |

| Nluc-sRNA_LNA_10/20 type | Nluc-sRNA_LNA_5/20 type | Nluc-sRNA_LNA_6/20 type |
|---|---|---|
| Calcd. 8243.0541 | Calcd. 8126.8911 | Calcd. 8152.9291 |
| Obs. 8242.05 | Obs. 8125.75 | Obs. 8151.74 |

### (6) Chemical capping of RNA

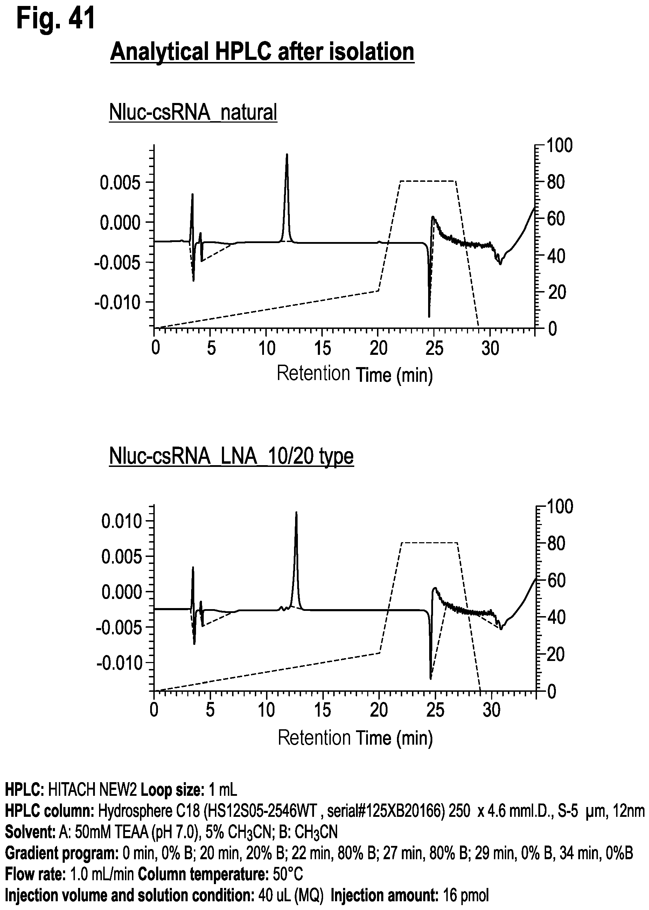

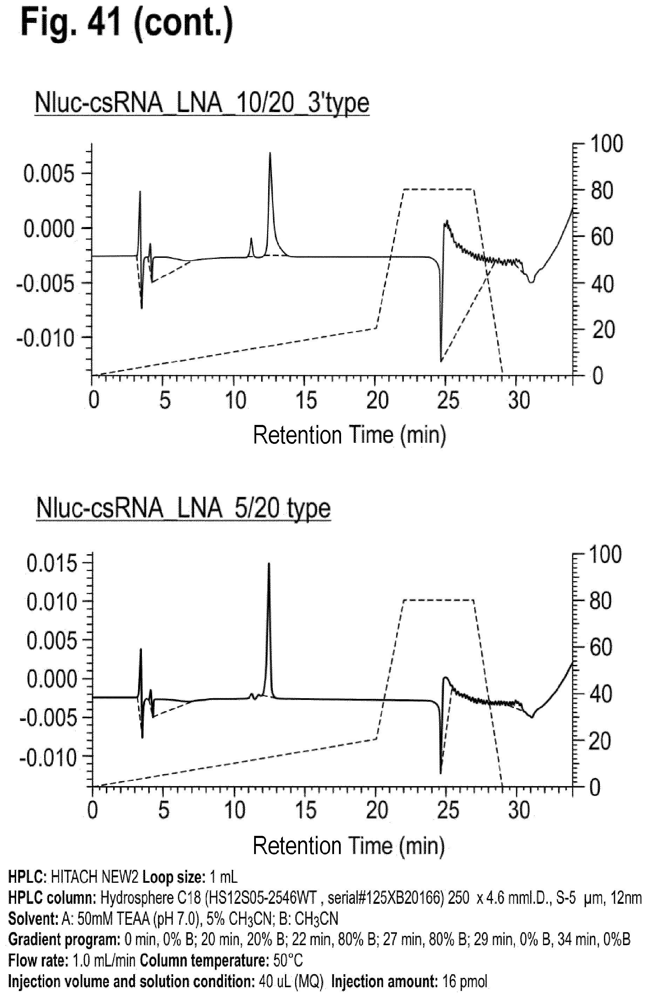

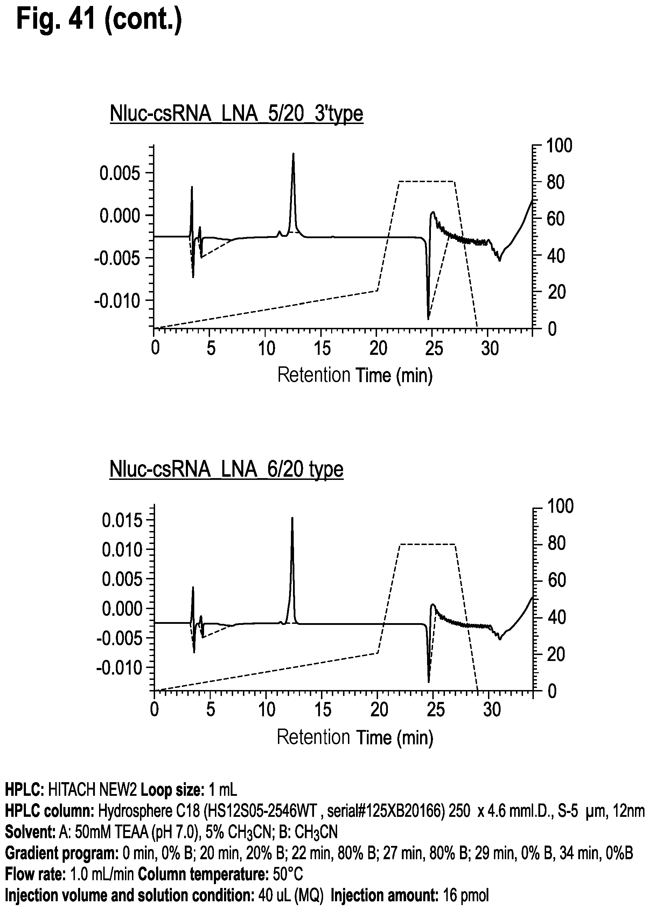

An aqueous RNA solution and an aqueous CaCl₂ solution were placed in a 1.5 mL tube, and then freeze-dried and solidified. A capping reagent dissolved in DMSO and 2-nitroimidazole were added thereto, and the mixture was scaled-up with DMSO. The composition of the final capping reaction solution is as follows (20 µM RNA, 10 mM CaCl₂, 10 mM capping reagent, 10 mM 2-nitroimidazole, scaled-up with DMSO). The reaction solution was incubated at 55°C for 3 hours, and then purification was performed by alcohol precipitation and reverse phase HPLC. Acetonitrile was removed with a centrifugal evaporator and then concentrated by freeze drying, and the concentration of the nucleic acid aqueous solution was determined from absorbance measurement at 260 nm. Thereafter, analysis by denaturing PAGE was performed. 10 pmol of RNA was mixed with 2×formamide loading solution and loaded onto the gel after heating at 80°C for 5 minutes. The gel was electrophoresed at 25 mA for 2 hours, stained by SYBR Green II, and then photographed by ChemiDoc. The capping efficiency was calculated from the band intensity. The results are shown in Figs. 41 and 42.

**[Table 7]**

| LC-MS | | |
|---|---|---|
| Nluc-csRNA natural | Nluc-csRNA_LNA_10/20_3' type | Nluc-csRNA_LNA_5/20_3' type |
| Calcd. 8490.0077 | Calcd. 8680.2527 | Calcd. 8592.1437 |
| Obs. 8491.40 | Obs. 8680.93 | Obs. 8592.84 |
| | | |

| Nluc-csRNA_LNA_10/20 type | Nluc-csRNA_LNA_5/20 type | Nluc-csRNA_LNA_6/20 type |
|---|---|---|
| Calcd. 8680.2527 | Calcd. 8564.0897 | Calcd. 8590.1277 |
| Obs. 8681.02 | Obs. 8564.98 | Obs. 8590.83 |

An annealing solution (0.2 µM circular NanoLuc mRNA, 0.4 µM csRNA (SEQ ID NOS: 56 to 58), 10 mM Tris-HCl (pH 7.5), 50 mM NaCl, 1 mM EDTA (pH 8.0)) was prepared and heated at 80°C for 5 minutes. Thereafter, annealing of mRNA and csRNA was performed by gradually cooling until the temperature reached room temperature. 4.85 µL of Mixture-2 w/o DTT, 1.648 µL of 50 mM DTT, and 8.7 µL of a HeLa cell extract were mixed and incubated at room temperature for 10 minutes, and then 1.8 µL of Mixture-3 and 1 µL of an annealing solution were added. After incubation at 32°C for 4 hours, 1 µL of the reaction solution was mixed with 20 µL of Nano-Glo (registered trademark) Luciferase Assay Substrate (Promega) diluted with 1×Glo Lysis Buffer (Promega), and luminescence measurement was performed.

Fig. 45(a) shows the sequence of csRNA into which chemical modification was introduced. The complementary strand region with mRNA is indicated by underline. Nₘ, 2'OMe; bold letters, LNA. The sequences were all described in the 5' → 3' direction. (b) is a view showing luciferase luminescence measurement in a HeLa cell extract. A relative value when the luminescence intensity of circ. Nluc mRNA was taken as 1 was shown. Error bars were standard errors of three experimental averages.

From these results, in the case of annealing (w/annealing), cap-structure-dependent translation promotion was observed in any csRNA. The effect was increased by introducing LNA. On the other hand, when annealing was not performed (w/o annealing), the translation promoting effect of Nluc-csRNA_polyA_LNA was high, and it was found that an excellent effect was exhibited even without annealing by containing LNA.

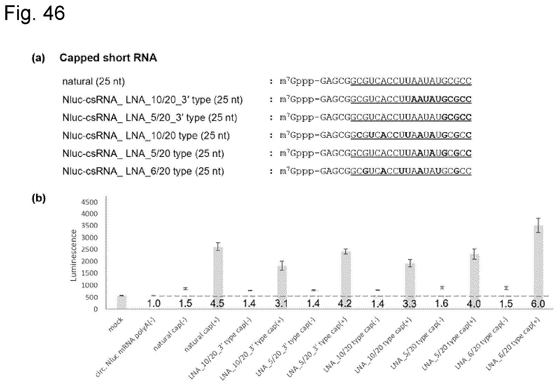

### 14. Translation reaction in HeLa cells of csRNA and circular mRNA into which LNA modification was introduced (Fig. 46)

### (1) Annealing and luminescence measurement

An annealing solution (0.1 µM circular NanoLuc mRNA, 1 µM csRNA (SEQ ID NOS: 59 to 64), 10 mM Tris-HCl (pH 7.5), 50 mM NaCl, 1 mM EDTA (pH 8.0)) was prepared and heated at 80°C for 5 minutes. Thereafter, annealing of mRNA and csRNA was performed by gradually cooling until the temperature reached room temperature. HeLa cells suspended in 100 µL of 10% FBS-containing D-MEM (Wako) at 1.5×10⁴ cells/well were seeded in a 96-well plate the day before transfection. The next day, the medium was removed and transfected with annealed mRNA by using Lipofectamine (registered trademark) MessengerMAX (registered trademark) (Thermo Fisher SCIENTIFIC) (Lipofectamine (registered trademark) MessengerMAX (registered trademark) 0.3 µL, annealing solution 1 µL, scaled-up to 100 µL with Opti-MEM (registered trademark) (Thermo Fisher SCIENTIFIC)). After incubation in an environment of 37°C and 5% CO₂ for 3 hours, the medium was removed and 100 µL of 10% FBS-containing D-MEM (Wako) was added. After incubation again in an environment of 37°C and 5% CO₂ for 3 hours, the medium was removed, and 20 µL of 1×Glo Lysis Buffer (Promega) was added thereto for lysation. 10 µL of the lysate was mixed with 20 µL of Nano-Glo (registered trademark) Luciferase Assay Substrate (Promega) diluted with 1×Glo Lysis Buffer (Promega), and luminescence measurement was performed.

Fig. 46(a) shows the sequence of csRNA used for translation. The complementary strand region with mRNA is indicated by underline. Bold letters, LNA. The sequences were all described in the 5' → 3' direction. (b) shows luciferase luminescence measurement in HeLa cells. A relative value when the luminescence intensity of mRNA (cap(-)) or mRNA (cap(+)) was taken as 1 was shown. Error bars were standard errors of three experimental averages.

From these results, full improvement in activity is observed in Nluc-csRNA_LNA_6/20 type as compared with natural csRNA.

Sequence Listing

International Application under International Patent Cooperation Treaty

## Claims

1. A capped RNA comprising:
a translated region beginning with a start codon and encoding a protein; and
an upstream region upstream of the start codon, a branch part having a cap structure being provided in the upstream region.

2. The capped RNA according to claim 1, wherein the branch part has at least one structure of (a) to (c) below:
(a) a capped probe having a sequence complementary to a sequence of a part or all of the upstream region and having the cap structure is hybridized to the upstream region;
(b) a branched chain branching from a main chain constituting the RNA is provided in a part of the upstream region and the cap structure is provided in the branched chain; or
(c) the RNA is a linear RNA and has a branch structure in which the 5' end of a main chain of the upstream region is branched into two or more branches, the cap structure being provided in each branch.

3. The capped RNA according to claim 2, wherein
in the (a), the RNA is a circular RNA, and
a capped probe having a sequence complementary to a sequence of a part or all of the upstream region of the circular RNA and having a cap structure is hybridized to the upstream region of the circular RNA.

4. The capped RNA according to claim 2, wherein
in the (a), the RNA is a linear RNA, and
a capped probe having a sequence complementary to a sequence of a part or all of the upstream region of the linear RNA and having a cap structure is hybridized to the upstream region of the linear RNA.

5. The capped RNA according to claim 2, wherein
in the (b), the RNA is a circular RNA, and
a branched chain branching from a main chain constituting the RNA is provided in a part of the upstream region of the circular RNA, and the cap structure is provided in the branched chain.

6. The capped RNA according to claim 2, wherein
in the (b), the RNA is a linear RNA, and
a branched chain branching from a main chain constituting the RNA is provided in a part of the upstream region of the linear RNA, and the cap structure is provided in the branched chain.

7. The capped RNA according to claim 2, which has a structure represented by Formula (3) below in the (b): 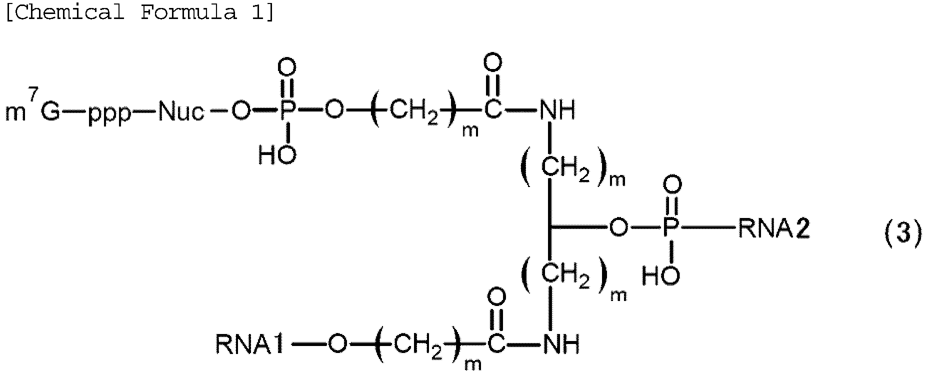 where m⁷G represents a cap, and p represents a phosphate group; Nuc represents a polynucleotide in which one or more natural or non-natural nucleotides are linked; m represents an integer of 1 to 5, and a plurality of m's may be the same as or different from each other; RNA1 and RNA2 represent RNAs constituting a main chain, and RNA1 and RNA2 are linked to a portion of a branched structure at the 3' end side and the 5' end side, respectively; and a RNA of Formula (3) may be a circular RNA in which the 5' end of RNA1 and the 3' end of RNA2 are linked to form a circular structure, or may be a linear RNA without the 5' end of RNA1 and the 3' end of RNA2 being linked.

8. The capped RNA according to claim 2, which has a structure represented by Formula (1) or Formula (2) below in the (c): 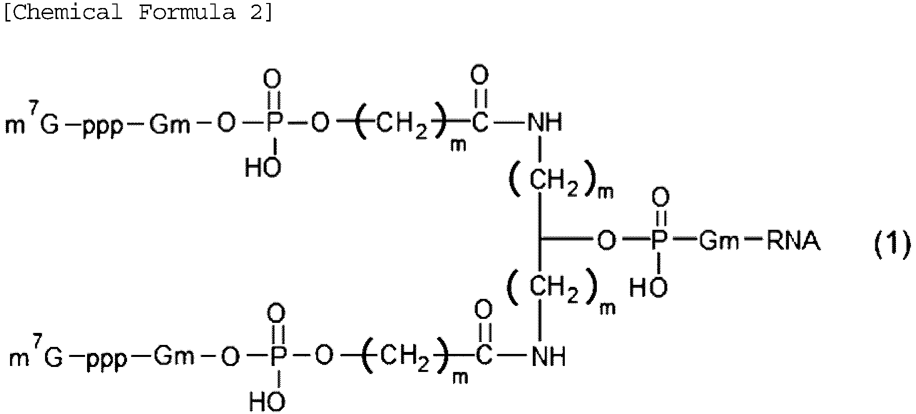 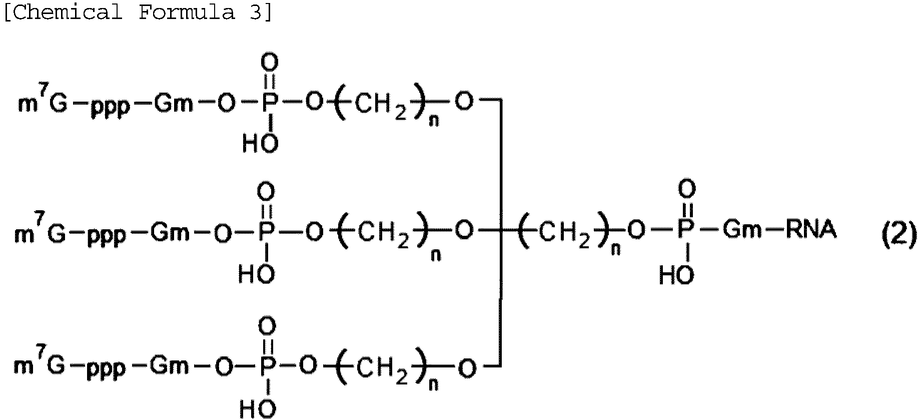 where m⁷G represents a cap, p represents a phosphate group, Gm represents a structure in which a 2'-hydroxyl group of a guanine nucleotide is methylated, m represents an integer of 1 to 5, a plurality of m's may be the same as or different from each other, n represents an integer of 1 to 5, and a plurality of n's may be the same as or different from each other.

9. The capped RNA according to claim 8, wherein the Formula (1) has a structure represented by Formula (1-1) below, and the Formula (2) has a structure represented by Formula (2-1) below.

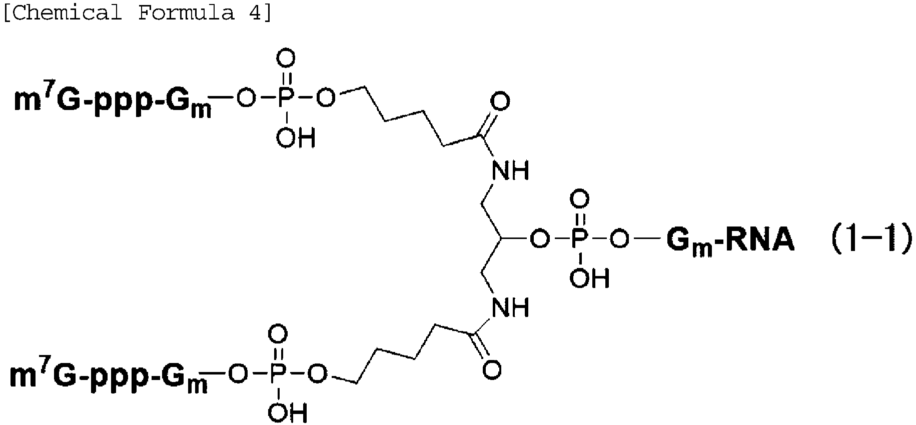

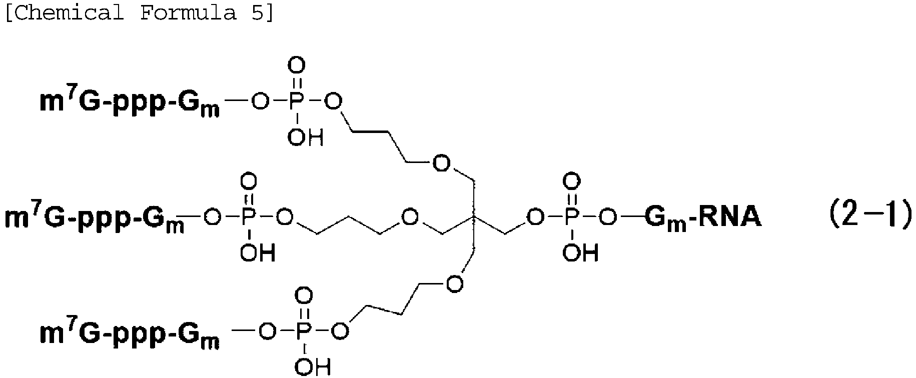

10. The capped RNA according to claim 1, wherein the RNA has a plurality of translated regions.

11. A method for producing the capped RNA according to claim 1, the method comprising at least one of steps (a) to (c) below:
(a) hybridizing a capped probe having a sequence complementary to a sequence of a part or all of the upstream region and having a cap structure to the upstream region; or
(b) synthesizing the RNA having a branched chain branching from a main chain constituting the RNA in a part of the upstream region, and then binding the cap structure to the branched chain; or
(c) with the RNA being a linear RNA, synthesizing RNA having a branch structure in which the 5' end of a main chain of the upstream region is branched into two or more branches, and then binding the cap structure to each branch.

12. An apparatus for producing a protein, comprising:
the capped RNA according to claim 1; and
an expression system of a eukaryotic cell capable of expressing the protein encoded by the translated region using the capped RNA as a template.

13. A method for producing a protein, comprising adding the capped RNA according to claim 1 to an expression system of a eukaryotic cell capable of expressing the protein encoded by the translated region using the capped RNA as a template to express the protein.

14. A detection device of a target RNA having a cap structure at the 5' end of RNA, the detection device comprising:
a detection probe containing a circular RNA having a translated region beginning with a start codon and encoding a labeled protein and an upstream region upstream of the start codon, the upstream region having a sequence complementary to a sequence of a part or all of the target RNA to be detected;
a means for hybridizing the target RNA to the upstream region of the circular RNA by adding the circular RNA to a sample containing the target RNA to generate a capped RNA;
an expression system of a eukaryotic cell capable of expressing the labeled protein by adding the capped RNA; and
a means for detecting the labeled protein expressed in the expression system.

15. A detection method of a target RNA having a cap structure at the 5' end of RNA, the detection method comprising:
a step of preparing a detection probe containing a circular RNA having a translated region beginning with a start codon and encoding a labeled protein and an upstream region upstream of the start codon, the upstream region having a sequence complementary to a sequence of a part or all of the target RNA to be detected;
a step of hybridizing the target RNA to the upstream region of the circular RNA by adding the circular RNA to a sample containing the target RNA to generate a capped RNA;
a step of adding the capped RNA to an expression system of a eukaryotic cell capable of expressing the labeled protein to express the labeled protein; and
a step of detecting the labeled protein expressed in the expression system.

16. A capped RNA having a cap structure in an untranslated region of RNA having a translated region beginning with a start codon and encoding a protein and the untranslated region upstream of the start codon, the capped RNA comprising at least one structure of (a) to (c) below:
(a) a capped probe having a sequence complementary to a sequence of a part or all of the untranslated region and having a cap structure is hybridized to the untranslated region;
(b) a branched chain branching from a main chain constituting the RNA is provided in a part of the untranslated region and the cap structure is provided in the branched chain; or
(c) the RNA is a linear RNA and has a branch structure in which the 5' end of a main chain of the untranslated region is branched into two or more branches, the cap structure being provided in each branch.
